# EUROPEAN PATENT APPLICATION

(11) **EP 3 901 156 A1**
(43) Date of publication of application: **27.10.2021**
(21) Application number: 20913089.7
(22) Date of filing: 18.11.2020
(51) Int. Cl.: C07D 495/04, A61P 27/06

(54) **NITROOXYDERIVATIVE OF ROCK KINASE INHIBITOR**

(30) Priority: 21.02.2020 CN 202010107800
(71) Applicant: Vivavision (Shanghai) Ltd, Pudong District Shanghai 200000 (CN)
(72) Inventor: SHEN, Wang, Shanghai 200000 (CN); DANG, Kuifeng, Shanghai 200000 (CN); ZHANG, Chao, Shanghai 200000 (CN); CHEN, Chunming, Shanghai 200000 (CN); SHAN, Lihong, Shanghai 200000 (CN); DING, Wen, Shanghai 200000 (CN); WANG, Xin, Shanghai 200000 (CN); MI, Jiachen, Shanghai200000 (CN); SUN, Yabo, Shanghai 200000 (CN); SHENG, Xiaxin, Shanghai 200000 (CN); SHEN, Jialei, Shanghai 200000 (CN); LI, Yong, Shanghai 200000 (CN)
(74) Representative: Tomerius, Isabel
(86) International application number: PCT/CN2020/129840
(87) International publication number: WO 2021/164351

(57) **Abstract**

The invention provides a small molecule compound of a NO donor, characterized in that the small molecule compound of the NO donor is a compound shown in the following structural formula I or a stereoisomer, a geometric isomer, a tautomer, a racemate, a deuterated isotopic derivative, a hydrate, a solvate, a metabolite, or a pharmaceutically acceptable salt or prodrug thereof; 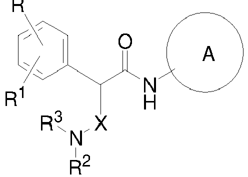 formula I; wherein a ring A is a substituted or unsubstituted heteroaromatic ring; X is selected from (CH₂)ₙ, wherein n is selected from 0, 1, 2, or 3; R is a substituent of terminal -O-NO2; R¹ is selected from hydrogen, a hydroxyl group, a halogen, an amino group, a cyano group, a substituted or unsubstituted alkyl group, a substituted or unsubstituted alkoxy group, a substituted or unsubstituted alkenyl group, a substituted or unsubstituted alkynyl group, or a substituted or unsubstituted heteroalkyl group; R² and R³ are each independently selected from hydrogen, a substituted or unsubstituted alkyl group, a substituted or unsubstituted cycloalkyl group, or an amino protecting group; alternatively, R² and R³ are connected to each other to form a substituted or unsubstituted cycloheteroalkyl group. The compound has a highly active inhibitory effect on ROCK kinase.

## Description

### BACKGROUND OF THE INVENTION

### Field of the Invention

The invention relates to a new heterocyclic ROCK kinase inhibitor, and more specifically, the invention relates to a nitroxide derivative of a ROCK kinase inhibitor. These compounds can be used to treat glaucoma and retinal diseases.

### Description of Related Art

Rho-associated coiled-coil protein kinase (ROCK) belongs to the AGC (PKA/PKG/PKC) family of serine-threonine kinases. Two human isotypes of ROCK have been described: ROCK-I (also known as p160ROCK or ROK*β*) and ROCK-II (ROK*α*), which are approximately 160 kDa proteins, containing one N-terminal Ser/Thr kinase domain, followed by one coiled-coil structure, one prück substrate protein homology domain, and one cysteine-rich region at the C-terminus (Multifunctional kinases in cell behaviour. Nat. Rev. Mol. Cell Biol. 2003(4):446-456).

Rho belongs to the GTPase superfamily of small molecule monomers and is a mammalian gene homolog of the Ras superfamily. The reorganization of the cellular actin skeleton is adjusted by the most important effector molecule Rho-kinase in the downstream of Rho (Rho-associated coiled-coil containing protein kinase, ROCK). Thus, Rho is widely involved in a series of biological processes such as cell mitosis, cytoskeleton adjustment, smooth muscle cell contraction, nerve regeneration, tumor cell infiltration, and regulation of apoptosis. After activation, Rho/ROCK can act on a variety of substrates, resulting in biological processes. The two most important substrates are myosin light chain (MLC) and myosin light chain phosphatase (MLCP). The phosphorylation level of MLC is an important factor in determining the degree of smooth muscle contraction. Myosin light chain kinase (MLCK) phosphorylates the Ser-19 site of MLC, leading to smooth muscle contraction; inhibition of MLCP can further enhance the phosphorylation of MLC and the contraction of smooth muscle. After activation, ROCK can phosphorylate MLC itself to cause myofilament contraction; at the same time, ROCK can phosphorylate MLCP and inactivate MLCP, resulting in increased phosphorylation of MLC in the cell cytoplasm, thus indirectly promoting myofilament contraction.

Inhibition of Rho-kinase activity in animal models has shown multiple benefits in the treatment of human diseases, including cardiovascular diseases such as pulmonary hypertension, high blood pressure, atherosclerosis, cardiac hypertrophy, high intraocular pressure, cerebral ischemia, cerebral vasospasm, etc., and central nervous system disorders such as neuronal degeneration, and tumors. Studies have shown that ROCK expression and activity are elevated in spontaneously hypertensive rats, indicating that ROCK is related to the occurrence of hypertension in these animals (Involvement of Rho-kinase in hypertensive vascular disease: a novel therapeutic target in hypertension. FASEB J.,2001,15(6):1062-4). ROCK inhibitor Y-27632 can significantly reduce blood pressure in three rat hypertension models (spontaneous hypertension, renal hypertension, and deoxycorticosterone acetate salt hypertension), but has less effect on blood pressure in control rats (Calcium sensitization of smooth muscle mediated by a Rho-associated protein kinase in hypertension[J]. Nature, 1997, 389(6654):990-4). Studies have also shown that ROCK inhibitors have a better effect on pulmonary hypertension (Acute vasodilator effects of a Rho-kinase inhibitor, fasudil in patients with severe pulmonary hypertension. Heart,2005:91(3):391-2).

ROCK activity is an important signal transmission mechanism in leukocyte-platelet-endothelial interaction, leukocyte extravasation, and edema. Excessive activation of Rho-kinase in endothelial cells can cause leakage caused by the destruction of cell-cell junctions that facilitate recruitment of inflammatory cells. In summary, these evidences point to the role of ROCK in pathological conditions related to acute and chronic inflammation and autoimmune diseases (Isoform-specific targeting of ROCK proteins in immune cells. Small GTPases. 2016; 7(3):173-177). The beneficial effects of ROCK inhibition in experimental models of rheumatoid arthritis and lupus are verified by animal experiments (Rho kinase inhibitors and their application to inflammatory disorders. Curr. Top. Med. Chem.2009 (9): 704-723; Antinociceptive effects of AS 1892802, a novel rho kinase inhibitor, in rat models of inflammatory and noninflammatory arthritis. J.Pharmacol.Exp.Ther.2010,334: 955-963; Administration of fasudil, a ROCK inhibitor, attenuates disease in lupus-prone NZB/W F1 female mice. Lupus. 2012; 21(6):656-61). Fasudil's inhibitory effect on T-cell migration may expand the clinical application thereof as a new therapy for multiple sclerosis (Therapeutic potential of experimental autoimmune encephalomyelitis by Fasudil, a Rho kinase inhibitor. J Neurosci Res.2010; 88(8): 1664-72). Accumulated evidence also confirms that ROCK plays a key role in the three disease factors that regulate the pathogenesis of inflammatory bowel disease (IBD): destruction of the intestinal barrier, exposure of intestinal contents to mucosal immune cells, and abnormal immune response (Role of Rho kinase signal pathway in inflammatory bowel disease. Int J ClinExp Med.2015; 8(3):3089-3097).

The ROCK inhibitor drugs currently on the market include Eril from Asahi Kasei (applicable to the treatment of cerebral vasospasm); Kowa's Glanatec (for the treatment of ocular hypertension and glaucoma, only approved in Japan), and Aerie's new glaucoma drug Rhopressa (netarsudil) was approved in the United States in 2017 for the treatment of patients suffering from open-angle glaucoma or high intraocular pressure to reduce their intraocular pressure. Rhopressa is a brand new one time-a-day eye drop.

Nitric oxide (NO) is an important intercellular information transmission factor. NO can be synthesized in organisms by NO synthase or released by drugs (such as nitroglycerin, Latanoprostene Bunod, nitro-containing prostaglandin drugs). NO binds to soluble guanylate cyclase (sGC) and then converts guanosine triphosphate into cyclic guanosine monophosphate (cGMP). cGMP is a second messenger regulating smooth muscle relaxation and vasodilation and many other important biological processes, such as platelet inhibition and cell growth and differentiation. NO plays an important physiological role in regulating the blood flow of the optic nerve head and IOP. In the optic nerve head, NO donors reduce vascular resistance by relaxing smooth muscles, leading to local vasodilation and increased blood flow to the optic nerve head. Conversely, damage to the NO pathway reduces blood flow to the optic nerve head, leading to ischemia.

NO plays an important physiological role in regulating the intraocular pressure (IOP) of the eye. For example, endothelial nitric oxide synthase (eNOS) is present in the uveal vascular system, Schlemm tube, and the endothelium of the ciliary body. NO is known to increase the anterior trabecular meshwork (TM) of humans, and NO donors reduce IOP in animal models. At the same time, mice overexpressing eNOS have lower IOP. In contrast, eNOS knockout mice (animals with no functional eNOS gene, and therefore no endogenous eNOS) have increased IOP, and sGC knockout mice have increased IOP and optic nerve degeneration. The mechanism by which NO reduces IOP seems to be by inhibiting actin-myosin interaction, thereby relaxing the cells in the TM and Schlemm tubes, resulting in increased water outflow and decreased IOP.

Therefore, the development of small-molecule drugs that simultaneously inhibit ROCK kinase and are NO donors may achieve a better reduction of intraocular pressure (IOP) than single-function small molecules, and possesses very important social and economic significance.

### SUMMARY OF THE INVENTION

An object of the invention is to provide a novel small molecule drug with high activity against ROCK kinase and at the same time is a NO donor, and a preparation method and application thereof.

A small molecule compound of a NO donor provided by the invention is a compound shown in the following structural formula I or a stereoisomer, a geometric isomer, a tautomer, a racemate, a deuterated isotopic derivative, a hydrate, a solvate, a metabolite, or a pharmaceutically acceptable salt or prodrug thereof ; 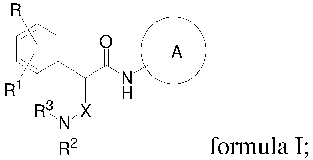
wherein a ring A is a substituted or unsubstituted heteroaromatic ring;
X is selected from (CH₂)ₙ, wherein n is selected from 0, 1, 2, or 3;
R is a substituent of terminal -O-NO2;
R¹ is selected from hydrogen, a hydroxyl group, a halogen, an amino group, a cyano group, a substituted or unsubstituted alkyl group, a substituted or unsubstituted alkoxy group, a substituted or unsubstituted alkenyl group, a substituted or unsubstituted alkynyl group, or a substituted or unsubstituted heteroalkyl group;
R² and R³ are each independently selected from hydrogen, a substituted or unsubstituted alkyl group, a substituted or unsubstituted cycloalkyl group, or an amino protecting group;
alternatively, R² and R³ are connected to each other to form a substituted or unsubstituted cycloheteroalkyl group.
More specifically, the small molecule compound of the NO donor provided by the invention is a compound shown in the following structural formula or a stereoisomer, a geometric isomer, a tautomer, a racemate, a deuterated isotopic derivative, a hydrate, a solvate, a metabolite, or a pharmaceutically acceptable salt or prodrug thereof :

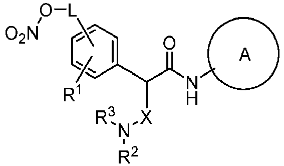

L is a linking group selected from a substituted or unsubstituted alkylene group, a substituted or unsubstituted heteroalkylene group, a substituted or unsubstituted alkyleneoxy group, a substituted or unsubstituted cycloalkyl group, a substituted or unsubstituted heterocyclic group, a substituted or unsubstituted aryl group, a substituted or unsubstituted heteroaryl group, or a substituted or unsubstituted ester group.

Further, in the small molecule compound of the NO donor provided by the invention, the ring A is selected from formula A or formula B; 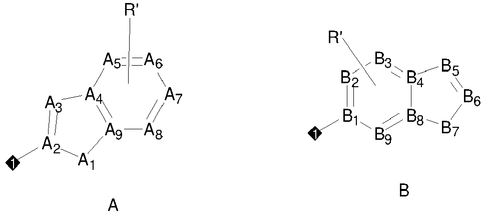
R' is one or a plurality of substituting substituent groups on the heteroaromatic ring;
the substituent group is selected from hydrogen, a hydroxyl group, a halogen, an amino group, a cyano group, a substituted or unsubstituted alkyl group, a substituted or unsubstituted alkoxy group, or a substituted or unsubstituted heteroalkyl group;
at least one of A₁, A₂, A₃, A₄, A₅, A₆, A₇, A₈, and A₉ is selected from nitrogen, sulfur, or oxygen;
at least one of B₁, B₂, B₃, B₄, B₅, B₆, B₇, B₈, and B₉ is selected from nitrogen, sulfur, or oxygen.

Further, in the small molecule compound of the NO donor provided by the invention, the ring A is selected from formula la, formula Ib, or formula Ic; 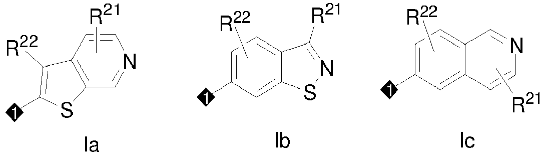
R²¹ is selected from hydrogen, a hydroxyl group, a halogen, an amino group, a cyano group, a substituted or unsubstituted alkyl group, a substituted or unsubstituted alkoxy group, or a substituted or unsubstituted heteroalkyl group;
R²² is selected from hydrogen, a halogen, a cyano group, a substituted or unsubstituted alkyl group, or a substituted or unsubstituted alkoxy group.

More specifically, the small molecule compound of the NO donor provided by the invention is a compound shown in the following structural formula or a stereoisomer, a geometric isomer, a tautomer, a racemate, a deuterated isotopic derivative, a hydrate, a solvate, a metabolite, or a pharmaceutically acceptable salt or prodrug thereof : 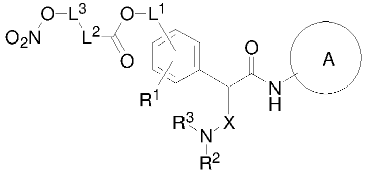
wherein L¹ is a chemical bond or a substituted or unsubstituted alkylene group;
L² is selected from a substituted or unsubstituted alkylene group, a substituted or unsubstituted alkyleneoxy group, a substituted or unsubstituted cycloalkyl group, a substituted or unsubstituted aryl group, or a substituted or unsubstituted heteroaryl group;
L³ is a chemical bond or a substituted or unsubstituted alkyleneoxy group.

More specifically, the small molecule compound of the NO donor provided by the invention is a compound shown in the following structural formula or a stereoisomer thereof, a geometric isomer, a tautomer, a racemate, a deuterated isotopic derivative, a hydrate, a solvate, a metabolite, or a pharmaceutically acceptable salt or prodrug: 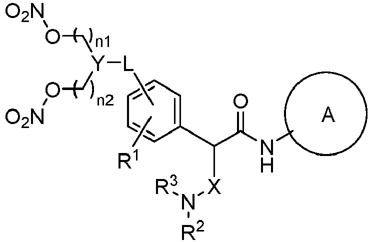
n1 is 0 or a natural number;
n2 is 0 or a natural number;
Y is selected from a substituted or unsubstituted alkyl group, a substituted or unsubstituted cycloalkyl group, a substituted or unsubstituted heterocyclic group, a substituted or unsubstituted aryl group, or a substituted or unsubstituted heteroaryl group.

More specifically, the small molecule compound of the NO donor provided by the invention is obtained by a nitration reaction of a hydroxyl group in a compound shown in the following structure; and nitration conditions can be concentrated nitric acid, or the nitration may occur by nitric acid in acetic anhydride.

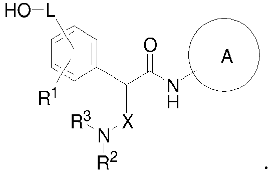

More specifically, the small molecule compound of the NO donor provided by the invention is obtained by a nitration reaction of a halogen in a compound shown in the following structure; and nitration conditions can be a silver nitrate effect.

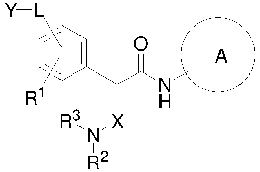

Y is a halogen.

Further, the small molecule compound of the NO donor provided by the invention is obtained by a coupling reaction of compounds represented by the following formula IIIa and formula IIIb;

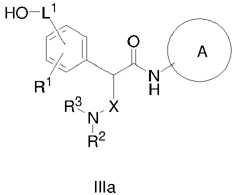

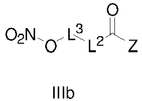

Z is a halogen.

Further, the small molecule compound of the NO donor provided by the invention is used as an inhibitor of a ROCK kinase.

The substituted alkyl group mentioned in the invention means that one or more of the hydrogen atoms on the carbon chain of the alkyl group are substituted by other groups. The other groups referred to here can be, but are not limited to, groups such as a cycloalkyl group (substituting in a form similar to 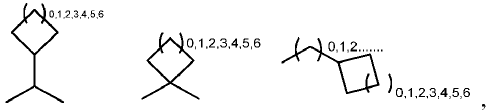 etc., any hydrogen atom on the cycloalkyl ring can also be substituted by a group such as a halogen, a cyano group, an alkyl group, a hydroxyl group, or a carboxyl group), a heterocycloalkyl group (that is, on the basis of the cycloalkyl group, at least one carbon atom on the alkyl ring is replaced by oxygen, sulfur, or nitrogen), a halogen (F, Cl, Br, or I), a carboxy group, a cyano group (-CN), a sulfonic acid group (-SO₄), a sulfonyl group (-SO₂Rₐ, Ra is hydrogen, an alkyl group, an aryl group, etc.), an alkynyl group (-C=CH, -C=CRb, Rb is an alkyl group, an aryl group, etc.), an amido group (-C(O)NRₓR_{y}, RₓR_{y} is an alkyl group, an aryl group, etc.), an ester group (-C(O)OR_{z}, R_{z} is an alkyl group, an aryl group, etc.), an aryl group, a heteroaryl group, or -O-NO₂;
in the substituted heteroalkyl group mentioned in the invention, one or a plurality of carbons in the substituted alkyl group are replaced by a heteroatom such as nitrogen, oxygen, or sulfur;
the substituted cycloalkyl group mentioned in the invention means that one or a plurality of hydrogen atoms on the alkyl ring are replaced by other groups. The other groups referred to here can be, but are not limited to, groups such as an alkyl group, a substituted alkyl group (same as above), a halogen (F, Cl, Br, or I), a carboxyl group, a cyano group (-CN), a sulfonic acid group (-SO₄), a sulfonyl group (-SO₂Rₐ, Ra is hydrogen, an alkyl group, an aryl group, etc.), an alkynyl group (-C=CH, -C=CRb, Rb is an alkyl group, an aryl group, etc.), an amido group (-C(O)NRₓR_{y}, RₓR_{y} is an alkyl group, an aryl group, etc.), an ester group (-C(O)O-R_{z}, R_{z} is an alkyl group, an aryl group, etc.), an aryl group, a heteroaryl group, or -O-NO2;
the substituted heterocycloalkyl group mentioned in the invention refers to that at least one carbon atom on the substituted cycloalkyl ring is substituted by a heteroatom such as nitrogen, oxygen, or sulfur;
the substituted alkenyl group mentioned in the invention means that the hydrogen atom in the structure -C=C- is replaced by other groups. The other groups here can be, but are not limited to, groups such as an alkyl group, a substituted alkyl group (same as above), a halogen (F, Cl, Br, or I), a carboxyl group, a cyano group (-CN), an amido group (-C(O)NRₓR_{y}, RₓR_{y} is an alkyl group, an aryl group, etc.), an ester group (-C(O)O-R_{z}, R_{z} is an alkyl group, an aryl group, etc.), an aryl group, a heteroaryl group, or -O-NO2;
the substituted alkynyl group mentioned in the invention means that the hydrogen atom in the structure -C=CH is replaced by other groups. The other groups here can be, but are not limited to, groups such as an alkyl group, a substituted alkyl group (same as above), a halogen (F, Cl, Br, or I), a carboxyl group, a cyano group (-CN), an amido group (-C(O)NRₓR_{y}, RₓR_{y} is an alkyl group, an aryl group, etc.), an ester group (-C(O)O-R_{z}, R_{z} is an alkyl group, an aryl group, etc.), an aryl group, a heteroaryl group, or -O-NO2;
the substituted alkylene group mentioned in the invention means that one or a plurality of the hydrogen atoms in the -(CH2)_{n(0, natural number)}- structure are substituted by other groups. The other groups referred to here can be, but are not limited to, groups such as a cycloalkyl group (substituting in a form similar to 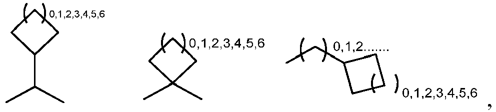 etc., any hydrogen atom on the cycloalkyl ring can also be substituted by a group such as a halogen, a cyano group, an alkyl group, a hydroxyl group, or a carboxyl group), a heterocycloalkyl group (that is, on the basis of the cycloalkyl group, at least one carbon atom on the alkyl ring is replaced by oxygen, sulfur, or nitrogen), a halogen (F, Cl, Br, or I), a carboxy group, a cyano group (-CN), a sulfonic acid group (-SO₄), a sulfonyl group (-SO₂R_{a,} Rₐ is hydrogen, an alkyl group, an aryl group, etc.), an alkynyl group (-C=CH, -C=CRb, Rb is an alkyl group, an aryl group, etc.), an amido group (-C(O)NRₓR_{y}, RₓR_{y} is an alkyl group, an aryl group, etc.), an ester group (-C(O)OR_{z}, R_{z} is an alkyl group, an aryl group, etc.), an aryl group, a heteroaryl group, or -O-NO₂. In addition, a plurality of carbon atoms can also be connected by a bridge;
the substituted alkyleneoxy group mentioned in the invention means that one or a plurality of the hydrogen atoms in the -O-(CH₂)_{n(0, natural number)}- or -(CH₂)_{n(0, natural number)}-O-(CH2)_{n(0, natural number)}- structure are substituted by other groups. The other groups referred to here can be, but are not limited to, groups such as a cycloalkyl group (substituting in a form similar to 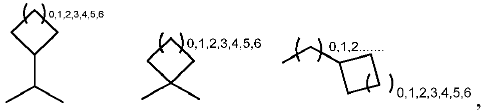 etc., any hydrogen atom on the cycloalkyl ring can also be substituted by a group such as a halogen, a cyano group, an alkyl group, a hydroxyl group, or a carboxyl group), a heterocycloalkyl group (that is, on the basis of the cycloalkyl group, at least one carbon atom on the alkyl ring is replaced by oxygen, sulfur, or nitrogen), a halogen (F, Cl, Br, or I), a carboxy group, a cyano group (-CN), a sulfonic acid group (-SO₄), a sulfonyl group (-SO₂Rₐ, Rₐ is hydrogen, an alkyl group, an aryl group, etc.), an alkynyl group (-C=CH, - C≡CR_{b}, R_{b} is an alkyl group, an aryl group, etc.), an amido group (-C(O)NRₓR_{y}, RₓR_{y} is an alkyl group, an aryl group, etc.), an ester group (-C(O)O-R_{z}, R_{z} is an alkyl group, an aryl group, etc.), an aryl group, a heteroaryl group, or -O-NO2;
the substituted heteroalkylene group mentioned in the present invention refers to that one or a plurality of carbon atoms in the substituted alkylene group are replaced by a heteroatom such as nitrogen, oxygen, or sulfur;
the substituted aryl group mentioned in the invention means that one or a plurality of hydrogen atoms on five-membered or above aromatic rings or benzo aromatic rings of, for example, benzene, naphthalene, fluorene are replaced by other groups. The other groups referred to here can be groups such as an alkyl group, a substituted alkyl group (same as above), a halogen (F, Cl, Br, or I), a carboxyl group, a cyano group (-CN), a sulfonic acid group (-SO₄), a sulfonyl group (-SO₂Rₐ, Rₐ is hydrogen, an alkyl group, an aryl group, etc.), an alkynyl group (-C=CH, - C≡CR_{b}, R_{b} is an alkyl group, an aryl group, etc.), an amido group (-C(O)NRₓR_{y}, RₓR_{y} is an alkyl group, an aryl group, etc.), an ester group (-C(O)O-R_{z}, R_{z} is an alkyl group, an aryl group, etc.), an aryl group, or a heteroaryl group.

The heteroaryl group mentioned in the invention refers to a five-membered or above aromatic heterocycle or benzo aromatic heterocycle such as thiophene, pyrrole, pyridine, furan, imidazole, benzimidazole, or quinoline.

The substituted heteroaryl group mentioned in the invention means that one or a plurality of hydrogen atoms on five-membered or above aromatic heterocycles or benzo aromatic heterocycles of, for example, thiophene, pyrrole, pyridine, furan, imidazole, benzimidazole, quinoline are replaced by other groups. The other groups referred to here can be groups such as an alkyl group, a substituted alkyl group (same as above), a halogen (F, Cl, Br, or I), a carboxyl group, a cyano group (-CN), a sulfonic acid group (-SO₄), a sulfonyl group (-SO₂Rₐ, Ra is hydrogen, an alkyl group, an aryl group, etc.), an alkynyl group (-C≡CH, -C≡CR_{b}, R_{b} is an alkyl group, an aryl group, etc.), an amido group (-C(O)NRₓR_{y}, RₓR_{y} is an alkyl group, an aryl group, etc.), an ester group (-C(O)O-R_{z}, R_{z} is an alkyl group, an aryl group, etc.), an aryl group, a heteroaryl group, or -O-NO₂.

The substituted ester group mentioned in the invention refers to the form of -O-C(O)-Rₛ-, -Lₛ-O-C(O)-R_{S}-, -O-C(O)-O-Rs-, or -Lₛ-O-C(O)-O-R_{S}-, wherein Lₛ is an alkylene group (substituted alkylene group), an aryl group, or a cycloalkyl group (substituted cycloalkyl group); and Rₛ is an alkylene group (substituted alkylene group), an aryl group, or a cycloalkyl group (substituted cycloalkyl group).

### DESCRIPTION OF THE EMBODIMENTS

### Example 1

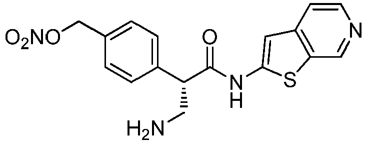

### (S)-4-(3-amino-1-oxo-1-(thieno[2,3-c]pyridin-2-ylamino)prop-2-yl)benzyl nitrate

The specific reaction equation is as follows:

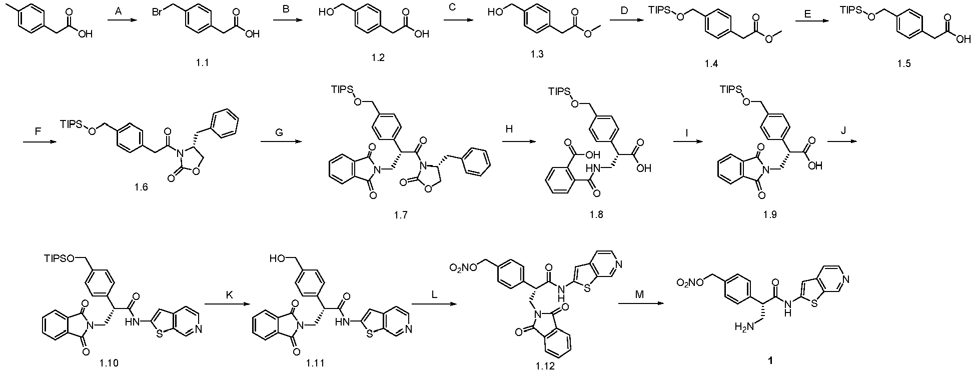

### Step A: 2-(4-(bromomethyl)phenyl)acetic acid (compound 1.1)

4-methylphenylacetic acid (100 g, 670 mmol) was dissolved in acetonitrile (1 L), N-bromosuccinimide (125.2 g, 703.5 mmol) and azobisisobutyronitrile (2.2 g, 13.4 mmol) were dissolved in the reaction solution, and the reaction solution was reacted at 80 °C for 3 hours under nitrogen protection. After the reaction was completed, the reaction solution was cooled to room temperature, a large amount of solid was precipitated, filtered, and the filter cake was washed successively with petroleum ether/ethyl acetate (1:1) (200 mL), saturated sodium bisulfite (200 mL), water (200 mL), and petroleum ether (200 mL). Then, drying was performed at 50 °C to obtain compound 1.1 (82 g, yield: 54%, white solid). LCMS ESI(+) m/z : 229.0/231.0(M+1), used directly in the next reaction.

### Step B: 2-(4-(hydroxymethyl)phenyl)acetic acid (compound 1.2)

Compound 1.1 (82 g, 358.1 mmol) was added to water (300 mL) and heating was performed at 100 °C to react for 2 hours. The reaction became clear, and then the reaction solution was cooled to room temperature. The temperature was further lowered to 0 °C in an ice bath, and a white solid was precipitated and filtered. The filter cake was washed two times with water and dried at 50 °C to obtain compound 1.2 (40 g, yield: 67%, white solid) used directly in the next reaction.

### Step C: methyl 2-(4-(hydroxymethyl)phenyl)acetate (compound 1.3)

Compound 1.2 (40 g, 241.0 mmol) was dissolved in methanol (400 mL), sulfuric acid (2.4 g, 24.1 mmol) was added, and the reaction solution was reacted in an ice bath for 2 hours. After the reaction was completed, saturated sodium bicarbonate solution (200 mL) was added. The aqueous layer was extracted with ethyl acetate (200 mL × 3), the organic layers were combined, washed with saturated saline water (300 mL), dried with anhydrous sodium sulfate, filtered, and spin-dried to obtain compound 1.3 (41 g, yield: 95%, light yellow oily substance). LCMS ESI(+) m/z:181.1 (M+1), used directly in the next reaction. ¹H NMR (400 MHz, DMSO) δ 7.23 (dd, *J* = 22.8, 8.4 Hz, 4H), 5.15 (s, 1H), 4.47 (s, 2H), 3.64 (s, 2H), 3.60 (s, 3H).

### Step d: 2-(4-(((triisopropylsilyl)oxy)methyl)phenyl)methyl acetate (compound 1.4)

Compound 1.3 (41 g, 226.5 mmol) and imidazole (23.1 g, 339.8 mmol) were dissolved in N,N-dimethylformamide (400 mL) and the reaction solution was cooled to 0 °C in an ice bath. Triisopropylchlorosilane (48.1 g, 249.2 mmol) was added in batches, and the reaction solution was naturally warmed to room temperature and stirred overnight. After the reaction was completed, water (500 mL) was added, the aqueous layer was extracted with ethyl acetate (200 mL × 3), and the organic layers were combined, washed with saturated saline water (300 mL), and dried with anhydrous sodium sulfate. Filtering and spin-drying were performed to obtain compound 1.4 (72 g, yield: 94%, colorless oily substance) used directly in the next reaction.

### Step E: 2-(4-(((triisopropylsilyl)oxy)methyl)phenyl)acetic acid (compound 1.5)

Compound 1.4 (72 g, 214.0 mmol) was dissolved in tetrahydrofuran (500 mL), lithium hydroxide (10.8 g, 256.8 mmol) was added, the reaction solution was stirred overnight at room temperature, and 1 M hydrochloric acid solution (300 mL) was added after the reaction was completed. The aqueous layer was extracted with ethyl acetate (200 mL×3), the organic layers were combined, washed with saturated saline water (300 mL), and the organic phase was dried with anhydrous sodium sulfate. Filtering and spin-drying were performed to obtain compound 1.5 (67 g, yield: 97%, colorless oily substance). ¹H NMR (400 MHz, DMSO) δ 12.28 (s, 1H), 7.25 (d, *J* = 12.8 Hz, 4H), 4.78 (s, 2H), 3.54 (s, 2H), 1.14 (s, 3H), 1.05 (d, *J* = 6.8 Hz, 18H).

### Step F: (R)-4-benzyl-3-(2-(4-(((triisopropylsilyl)oxy)methyl)phenyl)acetyl)oxazolidin-2-one (compound 1.6)

Compound 1.5 (67 g, 207.8 mmol) and anhydrous N,N-dimethylformamide (0.76 g, 10.4 mmol) were dissolved in anhydrous dichloromethane, oxalyl chloride (31.7 g, 249.4 mmol) was slowly added dropwise under ice bath conditions, and the ice bath was kept to prepare acid chloride. At the same time, (R)-4-benzyl-oxazolidinone (25.7 g, 145.2 mmol) was added to anhydrous tetrahydrofuran (300 mL) in another three-neck flask, and the flask was replaced with nitrogen. The flask was cooled down to -78 °C with dry ice acetone, n-butyllithium (2.5 M, 64 mL) was slowly added dropwise, and the temperature was kept at -78 °C. After the dropwise addition, stirring was performed at -78 °C for 2 hours, then the prepared anhydrous tetrahydrofuran solution of acid chloride was slowly added via a constant-pressure dropping funnel while keeping the dropwise addition temperature at -78 °C. After the dropwise addition was completed, the reaction solution was further reacted at -78 °C for 2 hours, and then the flask was naturally warmed to room temperature. After the reaction was completed, the reaction was extracted with saturated ammonium chloride aqueous solution (400 mL), and the aqueous layer was extracted with ethyl acetate (300 mL × 3). The organic layers were combined, washed with saturated aqueous sodium bicarbonate solution (300 mL) and saturated saline water (300 mL), dried with anhydrous sodium sulfate, filtered, and spin-dried. After purification by column chromatography, compound 1.6 (44 g, yield: 44%, colorless oily substance) was obtained.

### Step G: 2-((S)-3-((R)-4-benzyl-2-oxooxazolidin-3-yl)-3-oxo-2-(4-(((triisopropylsilyl)oxy)methyl)phenyl)propyl)isoindoline-1,3-dione (compound 1.7)

Compound 1.6 (44 g, 91.3 mmol) was dissolved in anhydrous tetrahydrofuran (500 mL), the flask was replaced with nitrogen, cooled with dry ice acetone to -78 °C, and LiHMDS (1 M, 109 mL) was slowly added dropwise. After the dropwise addition was completed, stirring was performed at -78 °C for 1 hour, and N-bromomethylphthalimide (26.3 g, 109.6 mmol) was dissolved in anhydrous tetrahydrofuran (200 mL). The reaction solution was slowly added dropwise to the above system via a constant-pressure dropping funnel, and the dropwise addition temperature was controlled to be lower than -70 °C. After the dropwise addition was completed, the reaction was kept at -78 °C and stirring was performed for 3 hours. The temperature was naturally raised to room temperature and the reaction solution was stirred overnight. After the reaction was completed, the reaction was extracted with saturated ammonium chloride aqueous solution (400 mL). The aqueous layer was extracted with ethyl acetate (300 mL × 3), and the organic layers were combined, and washed with saturated saline water (300 mL). Drying was performed with anhydrous sodium sulfate, and filtering and spin-drying were performed to obtain a yellow solid. The yellow solid was recrystallized with an ethyl acetate/petroleum ether system to precipitate a solid. The solid was filtered, and the filter cake was washed with petroleum ether (100 mL) and spin-dried to obtain compound 1.7 (32 g, yield: 55%, white solid).

### Step H: (S)-2-((2-carboxy-2-(4-(((triisopropylsilyl)oxy)methyl)phenyl)ethyl)carbamoyl)benzoic acid (compound 1.8)

Compound 1.7 (32 g, 49.9 mmol) was dissolved in tetrahydrofuran (300 mL), water (100 mL) was added, the reaction system was cooled to 0 °C in an ice bath, and then lithium hydroxide (6.3 g, 149.7 mmol) was added and stirring was performed at 0 °C for 1 hour. After the reaction was completed, the pH of the reaction solution was adjusted to 2 to 3 with 2 M hydrochloric acid solution. The organic layers were combined, washed successively with saturated saline water (300 mL), dried with anhydrous sodium sulfate, filtered, and spin-dried to obtain compound 1.8 (20 g, yield: 80%, light yellow solid). LCMS ESI(+) m/z:500.2 (M+1).

### Step I: (S)-3-(1,3-dioxoisoindolin-2-yl)-2-(4-(((triisopropylsilyl)oxy)methyl)phenyl)propionic acid (compound 1.9).

Compound 1.8 (20 g, 40 mmol), 1-hydroxybenzotriazole (5.9 g, 44 mmol), and triethylamine (12.1 g, 120 mmol) were dissolved in N,N-dimethylformamide, and the reaction system was cooled to 0 °C in an ice bath. Then, 1-ethyl-(3-dimethylaminopropyl)carbodiimide hydrochloride (8.4 g, 44 mmol) was added to the reaction solution. After stirring for a period of time, the reaction solution was clear and the temperate was slowly raised to room temperature overnight. After the reaction was completed, the reaction solution was depressurized to dryness, and saturated aqueous ammonium chloride solution (200 mL) was added. The aqueous layer was extracted with ethyl acetate (200 mL × 3), and the organic layers were combined, and washed with saturated aqueous sodium bicarbonate (300 mL) and saturated saline water (300 mL) successively. Drying was performed with anhydrous sodium sulfate, then filtering and spin-drying were performed to obtain compound 1.9 (17 g, yield: 88%, yellow solid).

### Step J: (S)-3-(1,3-dioxoisoindolin-2-yl)-N-(thieno[2,3-c]pyridin-2-yl)-2-(4-(((triisopropylsilyl)oxy)methyl)phenyl)propionamide (compound 1.10)

Compound 1.9 (65 mg, 0.123 mmol) was dissolved in 5 mL of N,N-dimethylformamide, and diisopropylethylamine (25 ul, 0.16 mmol) and 2-(7-benzotriazole oxide)-N,N,N",N'-tetramethylurea hexafluorophosphate (77 mg, 0.20 mmol) were added. Stirring was performed at room temperature for 10 minutes, and thieno[2,3-c]pyridine-2-amine (20 mg, 0.135 mmol) was added to stir for 2 hours. The reaction was quenched with saturated aqueous ammonium chloride solution, extracted three times with 30 mL ethyl acetate, and the organic phases were combined, dried with anhydrous sodium sulfate, spin-dried, and purified by column chromatography to obtain product 1.10 (48 mg, yield: 58%). LCMS ESI(+)m/z:614.2(M+1)/

### Step K: (S)-3-(1,3-dioxoisoindolin-2-yl)-2-(4-(hydroxymethyl)phenyl)-N-(thieno[2,3-c]pyridin-2-yl)propionamide (compound 1.11)

Compound 1.10 (48 mg, 0.078 mmol) was dissolved in 5 mL of tetrahydrofuran solution, 1 mL of hydrochloric acid (2 M) was added, and the reaction solution was stirred at room temperature for 0.5 hours. The solvent was directly spin-dried to obtain product 1.11 (30 mg, yield: 100%). LCMS ESI(+)m/z:458.1(M+1)

### Step L: (S)-4-(3-(1,3-dioxoisoindolin-2-yl)-1-oxo-1-(thieno[2,3-c]pyridin-2-ylamino)prop-2-yl)benzyl nitrate (compound 1.12)

2 drops of nitric acid and 1 drop of acetic anhydride were dissolved in dichloromethane, stirring was performed at room temperature for 10 minutes, and then the dichloromethane solution of compound 1.11 (20 mg, 0.04 mmol) was slowly added dropwise into the system. During the reaction, a lot of compound 1.11 was insoluble, and the same dichloromethane solution of nitric acid and acetic anhydride was placed in another reaction flask, and the insoluble reaction solution was slowly added dropwise. This process was repeated three to four times until all of compound 1.11 was dissolved. The reaction was monitored to be complete by LCMS. After the reaction was completed, water was added to quench the reaction, and then extraction was performed with dichloromethane (10 mLX3). The organic phases were combined, washed with saturated saline water (20 mL), and the organic phases were dried with anhydrous sodium sulfate. Filtering and spin-drying were performed, and the residue was purified by column chromatography to obtain product 1.12 (20 mg, yield: 93%). LCMS ESI(+) m/z:503.1(M+1).

### Step M: (S)-4-(3-amino-1-oxo-1-(thieno[2,3-c]pyridin-2-ylamino)prop-2-yl)benzyl nitrate (compound 1)

Compound 1.12 (20 mg, 0.04 mmol) was dissolved in 1 mL of methanol, then hydrazine hydrate was added, and a reaction was performed overnight at 40° C. The reaction was monitored to be complete by LCMS. After the reaction was completed, water was added to quench the reaction, and then extraction was performed with ethyl acetate (10 mL×3). The organic phases were combined and concentrated under reduced pressure. The residue was dissolved in 2 mL of methanol, filtered, and the filtrate was purified by reverse phase preparation. After lyophilization, the target product Example 1 (8 mg, yield: 44%) was obtained. LCMS ESI(+) m/z:373.1(M+1). ¹H NMR (400 MHz, DMSO-d6) δ 12.71 (s, 1H), 9.41 (s, 1H), 8.51 (d, J = 6.4 Hz, 1H), 8.06 (d, J = 6.0 Hz, 1H), 7.99 (s, 3H), 7.54 - 7.50 (m, 2H), 7.46 - 7.42(m, 2H), 7.24 (s, 1H),5.57 (s, 2H), 4.27 - 4.22 (m, 1H), 3.62 - 3.57(m, 1H), 3.25 - 3.15(m, 1H).

### Example 2

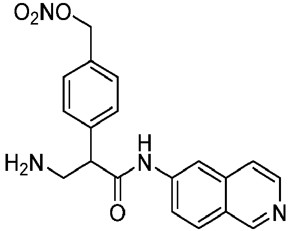

### (3-amino-1-(isoquinolin-6-ylamino)-1-oxoprop-2-yl)benzyl nitrate

The specific reaction equation is as follows:

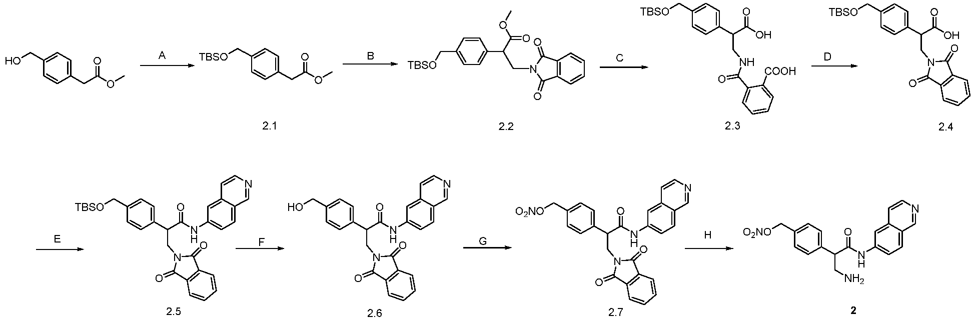

### Step A: methyl 2-(4-(((tert-butyldimethylsilyl)oxy)methyl)phenyl)acetate (compound 2.1)

Methyl 2-(4-(hydroxymethyl)phenyl)acetate (5.0 g, 27.8 mmol) and imidazole (2.8 g, 41.7 mmol) were dissolved in 50 mL of N,N-dimethylformamide, and then tert-butyldimethylchlorosilane (5.0 g, 33.4 mmol) was added to react overnight at room temperature. The reaction was monitored to be complete by TLC. Water was added to quench the reaction, extraction was performed with ethyl acetate (20 mLX3), and the organic phases were combined and washed with saturated saline water (50 mL). The organic phases were dried with anhydrous sodium sulfate, filtered, spin-dried, and purified by column chromatography to obtain product 2.1 (8.1 g, yield: 98%).

### Step B: methyl 2-(4-(((tert-butyldimethylsilyl)oxy)methyl)phenyl)-3-(1,3-dioxoisoindolin-2-yl)propionate (compound 2.2)

Compound 2.1 (3.0 g, 10.2 mmol) was dissolved in 10 mL of anhydrous tetrahydrofuran, the flask was replaced with nitrogen, the temperature was lowered to -78 °C with dry ice acetone, and lithium hexamethyldisilazide (1.0 M, 12.2 mL, 12.2 mmol) was slowly added dropwise. After the dropwise addition was completed, the system was stirred at -78 °C for 1 hour, N-bromomethyl phthalimide (2.9 g, 12.2 mmol) was dissolved in 10 mL of tetrahydrofuran, and the reaction solution was slowly added dropwise to the system. The temperature of the dropwise addition was controlled to be lower than -70 °C. After the dropwise addition was completed, the reaction was kept at -78 °C and stirred for 3 hours. The reaction was monitored to be complete by TLC. Saturated ammonium chloride solution was added to quench the reaction, extraction was performed with ethyl acetate (20 mLX3), and the organic phase was washed with saturated saline water (50 mL), dried with anhydrous sodium sulfate, filtered, spin-dried, and purified by column chromatography to obtain product 2.2 (3.6 g, yield: 78%).

### Step C: 2-((2-(4-(((tert-butyldimethylsilyl)oxy)methyl)phenyl)-2-carboxyethyl)carbamoyl)benzoic acid (compound 2.3)

Compound 2.2 (3.6 g, 8.0 mmol) was dissolved in 100 mL of tetrahydrofuran and 30 mL of water. Lithium hydroxide monohydrate (1.0 g, 24.0 mmol) was added at 0 °C to stir at 0 °C for 3 hours. The reaction was monitored to be complete by LCMS. The organic solvent was distilled off under reduced pressure, the pH was adjusted to 3 to 4 with 1 M aqueous hydrochloric acid, and then extraction was performed with ethyl acetate (20 mLX3), washed with saturated saline water (50 mL), and the organic phase was dried with anhydrous sodium sulfate, filtered, and spin-dried to obtain crude product 2.3 (3.4 g, yield: 93%). LCMS ESI(+)m/z:458.2 (M+1)

### Step D: 2-(4-(((tert-butyldimethylsilyl)oxy)methyl)phenyl)-3-(1,3-dioxoisoindolin-2-yl)propionic acid (compound 2.4)

Compound 2.3 (3.4 g, 7.4 mmol), 1-hydroxybenzotriazole (0.9 g, 7.4 mmol), and triethylamine (2.2 g, 22.2 mmol) were dissolved in 10 mL of dichloromethane, the reaction solution was put under nitrogen protection, and cooled to 0 °C. Then, 1-ethyl-(3-dimethylaminopropyl)carbodiimide hydrochloride (1.4 g, 7.4 mmol) was added to the reaction solution, stirring was performed for a period of time, the reaction solution became clear, the temperature was slowly raised to room temperature, and the reaction solution was reacted overnight. The reaction was monitored to be complete by TLC. Water was added to dilute the reaction solution, extraction was performed with dichloromethane (20 mLX3), and the organic phases were combined and washed with saturated saline water (50 mL). The organic phases were dried with anhydrous sodium sulfate, filtered, spin-dried, and purified by column chromatography to obtain product 2.4 (1.5 g, yield: 46%).

### Step E: 2-(4-(((tert-butyldimethylsilyl)oxy)methyl)phenyl)-3-(1,3-dioxoisoindolin-2-yl)-N-(isoquinolin-6-yl)propionamide (compound 2.5)

Compound 2.4 (500 mg, 1.1 mmol) was dissolved in 5 mL of N,N-dimethylformamide, and then 2-(7-benzotriazole oxide)-N,N,N',N'-tetramethylurea hexafluorophosphate (627 mg, 1.6 mmol) and N,N-diisopropylethylamine (283 mg, 2.2 mmol) were added successively. Stirring was performed at room temperature for 5 minutes, then 6-aminoisoquinoline (190 mg, 1.3 mmol) was added to react at room temperature for 2 hours. The reaction was monitored to be complete by LCMS. After the reaction was completed, water was added to quench the reaction, then extraction was performed with ethyl acetate (20 mLX3), and the organic phases were combined and washed with saturated saline water (50 mL). The organic phases were dried with anhydrous sodium sulfate, filtered, spin-dried, and purified by column chromatography to obtain product 2.5 (443mg, yield: 71%). LCMS ESI(+) m/z:566.2(M+1).

### Step F: 3-(1,3-dioxoisoindolin-2-yl)-2-(4-(hydroxymethyl)phenyl)-N-(isoquinolin-6-yl)propionamide (compound 2.6)

Compound 2.5 (443 mg, 0.80 mmol) was dissolved in 5 mL of tetrahydrofuran, and then added to 5 mL of aqueous hydrochloric acid (1 M) to react at room temperature for 30 minutes. The reaction was monitored to be complete by LCMS. After the reaction was completed, saturated sodium bicarbonate solution was added to neutralize, then extraction was performed with ethyl acetate (20 mLX3), and the organic phases were combined and washed with saturated saline water (50 mL). The organic phases were dried with anhydrous sodium sulfate, filtered, and spin-dried to obtain crude product 2.6 (242 mg, yield: 68%). LCMS ESI(+) m/z:452.2(M+1).

### Step G: 4-(3-(1,3-dioxoisoindolin-2-yl)-1-(isoquinolin-6-ylamino)-1-oxoprop-2-yl)benzyl nitrate (compound 2.7)

2 drops of nitric acid and 1 drop of acetic anhydride were dissolved in dichloromethane, stirring was performed at room temperature for 10 minutes, and then the dichloromethane solution of compound 2.6 (242 mg, 0.50 mmol) was slowly added dropwise into the system. During the reaction, a lot of compound 2.6 was insoluble, and the same dichloromethane solution of nitric acid and acetic anhydride was placed in another reaction flask, and the insoluble reaction solution was slowly added dropwise. This process was repeated three to four times until all of compound 2.6 was dissolved. The reaction was monitored to be complete by LCMS. After the reaction was completed, water was added to quench the reaction, then extraction was performed with dichloromethane (20 mLX3), and the organic phases were combined and washed with saturated saline water (50 mL). The organic phases were dried with anhydrous sodium sulfate, and filtered and purified by column chromatography to obtain product 2.7 (136 mg, yield: 54%). LCMS ESI(+) m/z:497.1(M+1).

### Step H: 3-amino-2-(4-(hydrazinomethyl)phenyl)-N-(isoquinolin-6-yl)propionamide (compound 2)

Compound 2.7 (30 mg, 0.06 mmol) was dissolved in 1 mL of methanol, then hydrazine hydrate was added to react at 40° C for 5 hours. The reaction was monitored to be complete by LCMS. After the reaction was completed, water was added to quench the reaction, and then extraction was performed three times with ethyl acetate (10 mL×3). The organic phases were combined and concentrated under reduced pressure. The residue was dissolved in 2 mL of methanol, filtered, and the filtrate was purified by reverse phase preparation. After lyophilization, target product Example 2 (1.1mg, yield: 4%) was obtained. LCMS ESI(+) m/z:367.1(M+1).

### Example 3

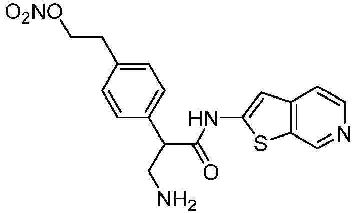

### 4-(3-amino- 1-oxo- 1-(thieno[2,3-c]pyridin-2-ylamino)prop-2-yl)phenylacetate

The specific reaction equation is as follows:

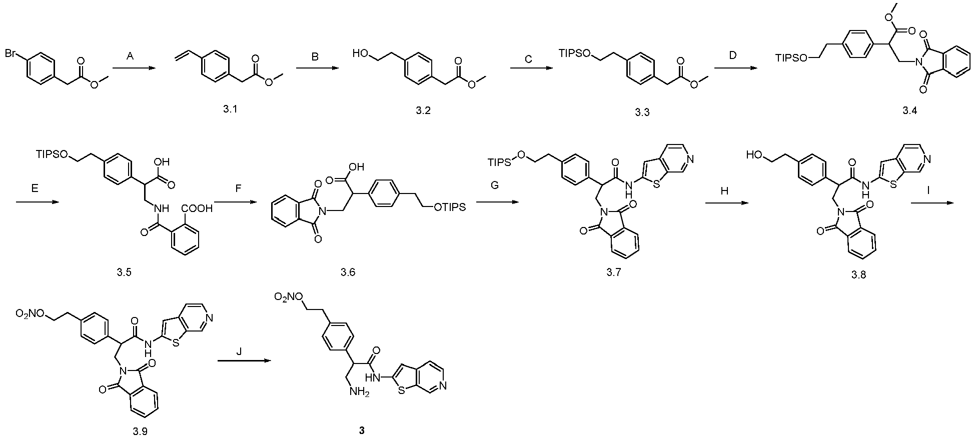

### Step A: methyl 2-(4-vinylphenyl)acetate (compound 3.1)

Methyl 2-(4-bromophenyl)acetate (11.0 g, 48.0 mmol), potassium trifluoro(vinyl)borate (7.7 g, 57.6 mmol), and cesium carbonate (31.3 g, 96.0 mmol) were dissolved in 200 mL of tetrahydrofuran and 20 mL of water. Then, bis(triphenylphosphine)palladium dichloride (674 mg, 0.96 mmol) was added to react overnight at 85 °C under nitrogen protection. The reaction was monitored to be complete by LCMS. Filtering was performed and the filtrate was extracted three times with ethyl acetate (50 mLX3), and the organic phases were combined and washed with saturated saline water (100 mL). The organic phases were dried with anhydrous sodium sulfate, filtered, spin-dried, and purified by column chromatography to obtain product 3.1 (7.0 g, yield: 83%). LCMS ESI(+) m/z:177.1(M+1).

### Step B: methyl 2-(4-(2-hydroxyethyl)phenyl)acetate (compound 3.2)

Compound 3.1 (3.5 g, 19.9 mmol) was dissolved in 20 mL of tetrahydrofuran, and borane (10.0 M, 4 mL, 40.0 mmol) was added dropwise in an ice bath to react at 0 °C for 1 hour, and then again at room temperature for 1 hour. Then, 60 mL of aqueous sodium hydroxide (1 M) was added dropwise in an ice bath, and then 5 mL of hydrogen peroxide (30%) was added dropwise to react in the ice bath for 30 minutes, and then again at room temperature for 30 minutes. The raw material reaction was monitored to be complete by TLC. Water was added to quench the reaction, extraction was performed with ethyl acetate (50 mLX3), and the organic phases were combined and washed with saturated saline water (100 mL). The organic phases were dried with anhydrous sodium sulfate, filtered, spin-dried, and purified by column chromatography to obtain product 3.2 (1.5 g, yield: 39%).

### Step C: methyl 2-(4-(2-((triisopropylsilyl)oxy)ethyl)phenyl)acetate (compound 3.3)

Compound 3.2 (1.5 g, 7.7 mmol) was dissolved in 10 mL of dichloromethane, and then 2,6-lutidine (1.4 mL, 11.6 mmol) and triisopropylsilyl triflate (2.5 mL, 9.2 mmol) were added to react overnight at room temperature. The raw material reaction was monitored to be complete by TLC. Water was added to quench the reaction, extraction was performed three times with dichloromethane (20 mLX3), and the organic phases were combined and washed with saturated saline water (50 mL). The organic phases were dried with anhydrous sodium sulfate, filtered, spin-dried, and purified by column chromatography to obtain product 3.3 (2.1 g, yield: 78%).

### Step D: methyl 3-(1,3-dioxoisoindolin-2-yl)-2-(4-(2-((triisopropylsilyl)oxy)ethyl)phenyl)propionate (compound 3.4)

Compound 3.3 (2.1 g, 6.0 mmol) was dissolved in 20 mL of anhydrous tetrahydrofuran, the flask was replaced with nitrogen, the temperature was lowered to -78 °C with dry ice acetone, and lithium hexamethyldisilazide (1.0 M, 7.2 mL, 7.2 mmol) was slowly added dropwise. After the dropwise addition was completed, the system was stirred at -78 °C for 1 hour, N-bromomethyl phthalimide (1.7 g, 7.2 mmol) was dissolved in 10 mL of tetrahydrofuran, and the mixture was slowly added dropwise to the reaction solution. The temperature of the dropwise addition was controlled to be lower than -70 °C. After the dropwise addition was completed, the reaction was kept at -78 °C and stirred for 3 hours. The raw material reaction was monitored to be complete by TLC. The reaction was quenched by adding saturated ammonium chloride solution (10 mL), extraction was performed with ethyl acetate (20 mLX3), and washing was performed with saturated saline water (50 mL). Drying was performed with anhydrous sodium sulfate, then filtering, spin-drying, and purification by column chromatography were performed to obtain product 3.4 (1.0 g, yield: 33%).

### Step E: 2-((2-carboxy-2-(4-(2-((triisopropylsilyl)oxy)ethyl)phenyl)ethyl)carbamoyl)benzoic acid (compound 3.5)

Compound 3.4 (1.0 g, 2.0 mmol) was dissolved in 10 mL of tetrahydrofuran and 3 mL of water. Lithium hydroxide monohydrate (252 mg, 6.0 mmol) was added at 0 °C to stir at 0 °C for 3 hours. The reaction was monitored to be complete by LCMS. The organic solvent was distilled off under reduced pressure, the pH was adjusted to 3 to 4 with 1 M aqueous hydrochloric acid, and then extraction was performed with ethyl acetate (20 mLX3). The organic phases were combined, washed with saturated saline water (50 mL), and the organic phases were dried with anhydrous sodium sulfate, filtered, and spin-dried to obtain product 3.5 (960 mg, yield: 95%). LCMS ESI(+)m/z:514.2(M+1).

### Step F: 3-(1,3-dioxoisoindolin-2-yl)-2-(4-(2-((triisopropylsilyl)oxy)ethyl)phenyl)propionic acid (compound 3.6)

Compound 3.5 (960 mg, 1.9 mmol), 1-hydroxybenzotriazole (257 mg, 1.9 mmol), and triethylamine (0.8 mL, 5.7 mmol) were dissolved in 10 mL of dichloromethane, and the reaction solution was put under nitrogen protection, and cooled to 0 °C. Then, 1-ethyl-(3-dimethylaminopropyl)carbodiimide hydrochloride (365 mg, 1.9 mmol) was added to the reaction solution, stirring was performed for a period of time, the reaction solution became clear, the temperature was slowly raised to room temperature, and the reaction solution was reacted overnight. The reaction was monitored to be complete by LCMS. Water was added to dilute the reaction solution, extraction was performed with dichloromethane (20 mLX3), and the organic phases were combined and washed with saturated saline water (50 mL). The organic phases were dried with anhydrous sodium sulfate, filtered, spin-dried, and purified by column chromatography to obtain product 3.6 (510 mg, yield: 54%). LCMS ESI(+)m/z:496.2(M+1).

### Step G: 3-(1,3-dioxoisoindolin-2-yl)-N-(thieno[2,3-c]pyridin-2-yl)-2-(4-(2-((triisopropylsilyl)oxy)ethyl)phenyl)propionamide (compound 3.7)

Compound 3.6 (70 mg, 0.14 mmol) was dissolved in 5 mL of N,N-dimethylformamide, and then 2-(7-benzotriazole oxide)-N,N,N',N'-tetramethylurea hexafluorophosphate (80 mg, 0.21 mmol) and N,N-diisopropylethylamine (36 mg, 0.28 mmol) were added successively. Stirring was performed at room temperature for 5 minutes, then thieno[2,3-c]pyridine-2-amine (25 mg, 0.17 mmol) was added to react at room temperature for 2 hours. The reaction was monitored to be complete by LCMS. After the reaction was completed, water was added to quench the reaction, then extraction was performed with ethyl acetate (10 mLX3), and the organic phases were combined and washed with saturated saline water (20 mL). The organic phases were dried with anhydrous sodium sulfate, filtered, spin-dried, and purified by column chromatography to obtain product 3.7 (62 mg, yield: 71%). LCMS ESI(+) m/z:628.3(M+1).

### Step H: 3-(1,3-dioxoisoindolin-2-yl)-2-(4-(2-hydroxyethyl)phenyl)-N-(thieno[2,3-c]pyridin-2-yl)propionamide (compound 3.8)

Compound 3.7 (62 mg, 0.10 mmol) was dissolved in 5 mL of tetrahydrofuran, and then the reaction solution was added to 5 mL of aqueous hydrochloric acid (1 M) to react at 40 °C for 2 hours. The reaction was monitored to be complete by LCMS. After the reaction was completed, saturated sodium bicarbonate solution was added to neutralize, then extraction was performed with ethyl acetate (10 mLX3), and the organic phases were combined and washed with saturated saline water (20 mL). The organic phases were dried with anhydrous sodium sulfate, filtered, and spin-dried to obtain crude product 3.8 (47mg, yield: 100%). LCMS ESI(+) m/z:472.1(M+1).

### Step I: 4-(3-(1,3-dioxoisoindolin-2-yl)-1-oxo-1-(thieno[2,3-c]pyridin-2-ylamino)prop-2-yl)phenylacetate (compound 3.9)

2 drops of nitric acid and 1 drop of acetic anhydride were dissolved in dichloromethane, stirring was performed at room temperature for 10 minutes, and then the dichloromethane solution of compound 3.8 (30 mg, 0.06 mmol) was slowly added dropwise into the system. During the reaction, a lot of the compound was insoluble, and the same dichloromethane solution of nitric acid and acetic anhydride was placed in another reaction flask, and the insoluble reaction solution was slowly added dropwise. This process was repeated three to four times until all of the compound was dissolved. The reaction was monitored to be complete by LCMS. After the reaction was completed, water was added to quench the reaction, then extraction was performed with dichloromethane (10 mLX3), and the organic phases were combined and washed with saturated saline water (20 mL). The organic phases were dried with anhydrous sodium sulfate, and filtered and purified by column chromatography to obtain product 3.9 (12 mg, yield: 48%). LCMS ESI(+) m/z:517.1(M+1).

### Step J: 4-(3-amino-1-oxo-1-(thieno[2,3-c]pyridin-2-ylamino)prop-2-yl)phenylacetate (compound 3)

Compound 3.9 (12 mg, 0.03 mmol) was dissolved in 1 mL of methanol, then hydrazine hydrate (14 mg, 0.30 mmol) was added to react overnight at 40° C. The reaction was monitored to be complete by LCMS. After the reaction was completed, water was added to quench the reaction, and then extraction was performed with ethyl acetate (10 mL×3). The organic phases were combined and concentrated under reduced pressure. The residue was dissolved in 2 mL of methanol, filtered, and the filtrate was purified by reverse phase preparation. After lyophilization, target product Example 3 (5 mg, yield: 36%) was obtained. LCMS ESI(+) m/z:387.1(M+1).

### Example 4

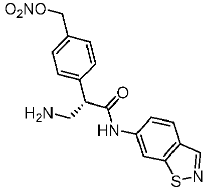

### (S)-4-(3-amino-1-(benzo[d]isothiazol-6-ylamino)-1-oxoprop-2-yl)benzyl nitrate

The specific reaction equation is as follows

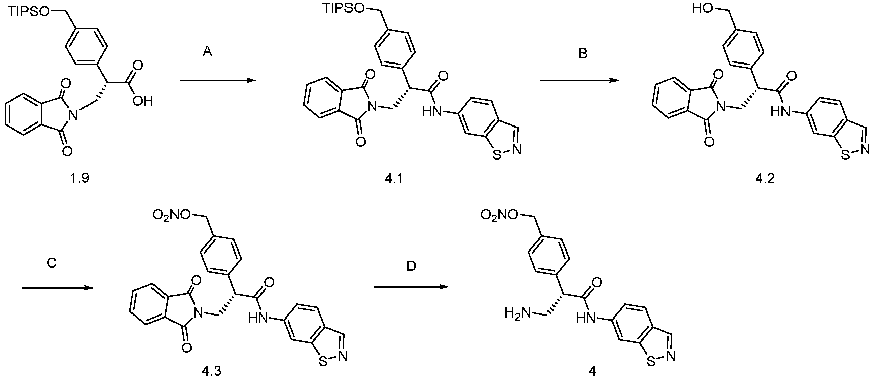

Step A: compound 4.1 was synthesized using benzo[d]isothiazole-6-amine instead of thieno[2,3-c]pyridine-2-amine in step J of Example 1. LCMS ESI(+)m/z:614.2(M+1).

Step B: compound 4.2 was synthesized using step K in Example 1. LCMS ESI(+)m/z:458.1(M+1).

Step C: (S)-4-(1-(benzo[d]isothiazol-6-ylamino)-3-(1,3-dioxoisoindolin-2-yl)-1-oxoprop-2-yl)benzyl nitrate (compound 4.3)

Compound 4.2 (150 mg, 0.33 mmol) was dissolved in 10 mL of dichloromethane solution, acetic anhydride (0.5 ml) and nitric acid (0.5 ml) were added, and stirring was performed at 40 °C for 3 hours under nitrogen protection. The solvent was spin-dried and purified by column chromatography to obtain compound 4.3 (40 mg, yield: 24%). LCMS ESI(+)m/z:503.1(M+1).

Step D: (S)-4-(3-amino-1-(benzo[d]isothiazol-6-ylamino)-1-oxoprop-2-yl)benzyl nitrate (compound 4)

Compound 4.3 (40 mg, 0.08 mmol) was dissolved in 5 mL of methylamine ethanol solution, stirring was performed at 60 °C for 3 hours, and the solvent was spin-dried. After reverse preparation and purification, compound 4 (1.4 mg, yield: 5%) was obtained. LCMS ESI(+)m/z:373.1(M+1).

### Example 5

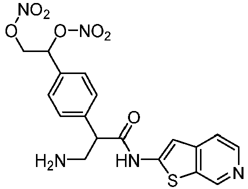

### 1-(4-(3-amino-1-oxo-1-(thieno[2,3-c]pyridin-2-ylamino)prop-2-yl)phenyl)ethane-1,2-dinitrate

The specific reaction equation is as follows:

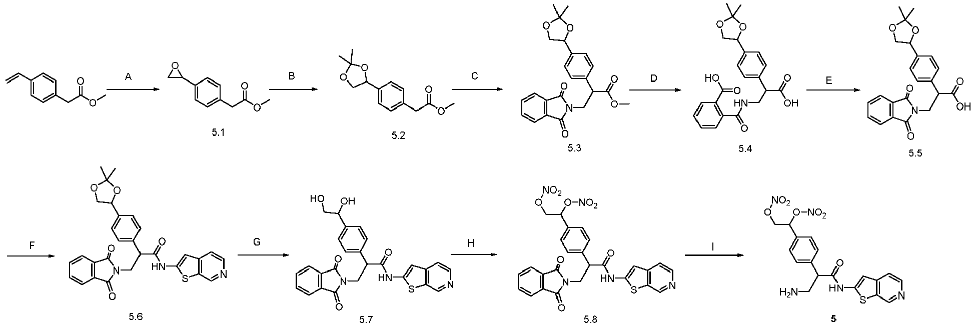

### Step A: methyl 2-(4-(oxiran-2-yl)phenyl)acetate (compound 5.1)

Weighed methyl 2-(4-vinylphenyl)acetate (2.2 g, 12.48 mmol) was dissolved in 80 mL of dichloromethane. In an ice bath, m-chloroperoxybenzoic acid (7.6 g, 37.44 mmol) was added in batches, and the reaction solution was stirred at 0 °C for 1 hour, and then at room temperature for 16 hours. 300 mL of saturated sodium sulfite solution was added to the reaction solution, stirring was performed for 1 hour, extraction was performed two times with dichloromethane (300 mL), and the organic phases were combined. The organic phases were washed two times with saturated saline water (100 mL), dried with anhydrous sodium sulfate, filtered, spin-dried, and purified by column chromatography to obtain compound 5.1 (1.84 g, yield: 77%, colorless oily substance).

### Step B: methyl 2-(4-(2,2-dimethyl-1,3-dioxolane-4-yl)phenyl)acetate (compound 5.2)

Compound 5.1 (1.84 g, 9.57 mmol) was dissolved in 30 mL of acetone, and then Amberlyst 15 (2.5 g, 12.44 mmol) was added, and the reaction solution was stirred at room temperature for 20 hours. The reaction solution was filtered, 150 mL of saturated sodium bicarbonate solution was added to the filtrate, and then extraction was performed with ethyl acetate (300 mL) two times, and the organic phases were separated and combined. The organic phases were washed one time with saturated saline water (100 mL), dried with anhydrous sodium sulfate, filtered, spin-dried, and purified by column chromatography to obtain compound 5.2 (1.5 g, yield: 63%, colorless oily substance).

### Step C: methyl 2-(4-(2,2-dimethyl-1,3-dioxolane-4-yl)phenyl)-3-(1,3-dioxoisoindolin-2-yl)propionate (compound 5.3)

Compound 5.2 (1.5 g, 5.99 mmol) was dissolved in anhydrous tetrahydrofuran (20 mL) at -78 °C, then lithium bistrimethylsilylamide (7.2 mL, 7.19 mmol) was added dropwise slowly into the reaction solution, and stirring was performed for 0.5 hours under nitrogen protection at - 78 °C. Right after, weighed N-bromomethylphthalimide (1.73 g, 7.19 mmol) was dissolved in anhydrous tetrahydrofuran (10 mL) and the reaction solution was added dropwise slowly to the reaction flask. Then, stirring was continued at this temperature for 1.5 hours. The reaction was quenched with 20 mL of saturated ammonium chloride solution, and extraction was performed two times with ethyl acetate (60 mL). The organic phases were separated and combined, and the organic phases were washed with saturated saline water (30 mL), dried with anhydrous sodium sulfate, filtered, spin-dried, and purified by column chromatography to obtain compound 5.3 (2 g, yield: 82%, white solid). LCMS ESI(+)m/z:410.1(M+1).

### Step D: 2-((2-carboxy-2-(4-(2,2-dimethyl-1,3-dioxolane-4-yl)phenyl)ethyl)carbamoyl)benzoic acid (compound 5.4)

Compound 5.3 (2 g, 4.88 mmol) was dissolved in 35 mL of tetrahydrofuran and 30 mL of water, lithium hydroxide monohydrate (615 mg, 14.64 mmol) was added, and the reaction solution was stirred at room temperature for 2 hours. Saturated citric acid solution was added to the reaction solution to pH 6, and extraction was performed with ethyl acetate (40 mL) three times. The organic phases were combined, washed with saturated saline water (40 mL) two times, dried with anhydrous sodium sulfate, filtered, and concentrated under reduced pressure to obtain product 5.4 (1.1 g, yield: 55%, white solid). LCMS ESI(+)m/z:414.1 (M+1).

### Step E: 2-(4-(2,2-dimethyl-1,3-dioxolane-4-yl)phenyl)-3-(1,3-dioxoisoindolin-2-yl)propionic acid (compound 5.5)

Compound 5.4 (1.1 g, 2.66 mmol) was dissolved in 30 mL of N,N-dimethylformamide, and 1-ethyl-(3-dimethylaminopropyl)carbodiimide hydrochloride (760 mg, 3.99 mmol), 1-hydroxybenzotriazole (540 mg, 3.99 mmol), and diisopropylethylamine (688 mg, 5.32 mmol) were added. The reaction solution was stirred at room temperature for 12 hours. The reaction solution was concentrated under reduced pressure, 20 mL of water was added to the residue, and saturated citric acid solution was used to adjust the pH to 6, and extraction was performed with ethyl acetate (40 mL) three times. The organic phases were combined, washed with saturated saline water (40 mL) two times, dried with anhydrous sodium sulfate, filtered, concentrated under reduced pressure, and the residue was purified by column chromatography to obtain product 5.5 (760 mg, yield: 72%, yellow solid). LCMS ESI(+)m/z:396.1(M+1).

### Step F: 2-(4-(2,2-dimethyl-1,3-dioxolane-4-yl)phenyl)-3-(1,3-dioxoisoindolin-2-yl)-N-(thieno[2,3-c]]pyridin-2-yl)propionamide (compound 5.6)

Compound 5.5 (260 mg, 0.66 mmol) was dissolved in 12 mL of N,N-dimethylformamide, and diisopropylethylamine (127 mg, 0.99 mmol), 6-aminoisoquinoline (104 mg, 0.69 mmol), and 2-(7-benzotriazole oxide)-N,N,N",N'-tetramethylurea hexafluorophosphate (300 mg, 0.79 mmol) were added. The reaction solution was stirred at room temperature for 1.5 hours. 30 mL of water was added to the reaction solution, and extraction was performed with ethyl acetate (50 mL) two times. The organic phases were combined, washed with saturated saline water (30 mL) two times, dried with anhydrous sodium sulfate, filtered, concentrated under reduced pressure, and the residue was purified by column chromatography to obtain product 5.6 (260 mg, yield: 75%, yellow solid). LCMS ESI(+)m/z:528.1(M+1).

### Step G: 2-(4-(1,2-dihydroxyethyl)phenyl)-3-(1,3-dioxoisoindolin-2-yl)-N-(thieno[2,3-c]pyridin-2-yl)propionamide (compound 5.7)

Compound 5.6 (260 mg, 0.49 mmol) was dissolved in 30 mL of methanol and 30 mL of acetonitrile, 3.3 mL 1.5 M aqueous hydrochloric acid was added, the reaction solution was stirred at 30 °C for 2 hours, and the reaction solution was concentrated under reduced pressure to obtain product 5.7 (240 mg, yield: 100%, yellow solid). LCMS ESI(+)m/z:488.1(M+1).

### Step H: 1-(4-(3-(1,3-dioxoisoindolin-2-yl)-1-oxo-1-(thieno[2,3-c]pyridin-2-ylamino)prop-2-yl)phenyl)ethane-1,2-dinitrodinitrate (compound 5.8)

0.4 mL of nitric acid solution was added to 6 mL of dichloromethane, the reaction solution was cooled to 0 °C, 0.2 mL of sulfuric acid was added dropwise, and the reaction solution was stirred at 0 °C for 0.5 hours. 3 mL of dichloromethane suspension of compound 5.7 (30 mg, 0.06 mmol) was added dropwise to the reaction solution. The reaction solution was stirred at 0 °C for 2 hours. Saturated sodium bicarbonate solution was added to the reaction solution to adjust the pH to 8, and extraction was performed with dichloromethane (30 mL) two times. The organic phases were combined, washed with saturated saline water (30 mL) two times, dried with anhydrous sodium sulfate, filtered, concentrated under reduced pressure, and the residue was purified by column chromatography to obtain product 5.8 (5 mg, yield: 14%, yellow solid). LCMS ESI(+)m/z:578.1(M+1).

### Step I: 1-(4-(3-amino-1-oxo-1-(thieno[2,3-c]pyridin-2-ylamino)prop-2-yl)phenyl)ethane-1,2-dinitrate (compound 5)

Compound 5.8 (5 mg, 0.01 mmol) was dissolved in 8 mL of ethanol, hydrazine hydrate (17 mg, 0.40 mmol) was added, and the reaction solution was stirred at 50 °C under nitrogen protection for 6 hours. 20 mL of water was added to the reaction solution, and extraction was performed with ethyl acetate (30 mL) two times. The organic phases were combined, washed with saturated saline water (30 mL) two times, dried with anhydrous sodium sulfate, filtered, concentrated under reduced pressure, and the residue was purified by reverse preparation to obtain compound 5 (1.2 mg, yield: 31%, white solid). LCMS ESI(+)m/z:448.1(M+1).

### Example 6

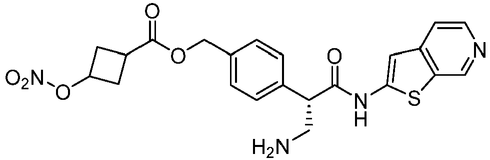

### (S)-4-(3-amino-1-oxo-1-(thieno[2,3-c]pyridin-2-ylamino)propan-2-yl)benzyl 3-(nitrooxy)cyclobutene- 1 -carboxylate

The specific reaction equation is as follows:

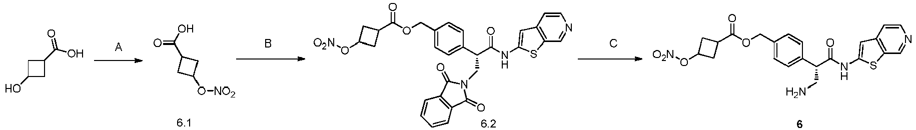

### Step A: 3-(nitrooxy)cyclobutane carboxylic acid (compound 6.1)

Under ice bath conditions, acetic anhydride (2 mL) was added into a 50 mL reaction flask, then right after, concentrated nitric acid (1 mL) was slowly added dropwise into the reaction flask. The reaction solution was stirred for 10 minutes in an ice bath. Under ice bath conditions, 3-hydroxycyclobutane carboxylic acid (100 mg, 0.862 mmol) was dissolved in acetic anhydride (3 mL), then an acetic anhydride-concentrated nitric acid mixture (3 mL) was slowly added dropwise to the reaction solution, and then the reaction solution was stirred at room temperature for 1 hour. The reaction was confirmed to be completed by TLC (bromocresol green color development). Water was added to the reaction solution, extraction was performed with ethyl acetate (15 mL), the organic phases were separated and combined, and then the organic phases were washed with saturated saline water. The organic phases were dried with anhydrous sodium sulfate, filtered, spin-dried, and purified by silica column to obtain white solid 6.1 (62 mg, yield: 44.67%).

### Step B: (S)-4-(3-(1,3-dioxyisoindol-2-yl)-1-oxy-1-(thieno[2,3-c]pyridin-2-ylamino)prop-2-yl)benzyl 3-(nitrooxy)cyclobutane-1-carboxylate (compound 6.2)

Compound 1.11 (60 mg, 0.131 mmol) was dissolved in N,N-dimethylformamide (6 mL), and then 1-(3-dimethylaminopropyl)-3-ethylcarbodiimide hydrochloride (37.7 mg, 0.197 mmol), 4-dimethylaminopyridine (24 mg, 0.197 mmol), and compound 6.1 (31.7 mg, 0.197 mmol) were added successively. The mixture was stirred overnight at room temperature. The reaction was confirmed to be complete by LCMS. Water was added to the reaction solution, extraction was performed with ethyl acetate (15 mL), the organic phases were separated and combined, and then the organic phases were washed with saturated saline water. The organic phases were dried with anhydrous sodium sulfate, filtered, spin-dried, and purified by silica column to obtain white solid 6.2 (50 mg, yield: 63.5%). LCMS ESI(+) m/z:601.1(M+1).

### Step C: (S)-4-(3-amino-1-oxo-1-(thieno[2,3-c]pyridin-2-ylamino)propan-2-yl)benzyl 3-(nitrooxy)cyclobutene-1-carboxylate (compound 6)

Compound 6.2 (83 mg, 0.138 mmol) was dissolved in 8 mL of ethanol, and hydrazine hydrate (69 mg, 1.38 mmol) was added to the reaction solution. The reaction was raised to 55 °C and stirred for 2 hours under an inert gas atmosphere. The reaction was confirmed to be complete by LCMS. Water was added to the reaction solution, extraction was performed with ethyl acetate (12 mL), and washing was performed with saturated saline water. The organic phase was dried with anhydrous sodium sulfate, filtered, concentrated and spin-dried. The residue was dissolved in methanol (3 mL). After reverse preparation and purification, the white solid product Example 6 (20 mg (hydrochloride), yield: 28.5%, purity: 97%) was obtained. LCMS ESI(+) m/z:471.1(M+1). ¹H NMR (400 MHz, DMSO) δ 13.41 (s, 1H), 9.47 (s, 1H), 8.52 (d, *J* =6.8 Hz, 1H), 8.21 - 8.12 (m, 4H), 7.49 (d, *J* = 8.4 Hz, 2H), 7.40 (d, *J* = 8.8Hz, 3H), 5.26 (p, J = 7.2 Hz, 1H), 5.10 (s, 2H), 4.45 (dd, J = 8.8, 5.6 Hz, 1H), 3.63 (s, 1H), 3.21 - 3.12 (m, 1H), 3.03 - 2.92 (m, 1H), 2.71 - 2.61(m,2H), 2.34 (ddd, J = 17.2, 9.6, 2.8 Hz, 2H).

### Example 7

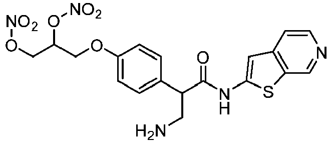

### 3-(4-(3-amino-1-oxo-1-(thieno[2,3-c]pyridin-2-ylamino)prop-2-yl)phenoxy)propane-1,2-dinitrate

The specific reaction equation is as follows:

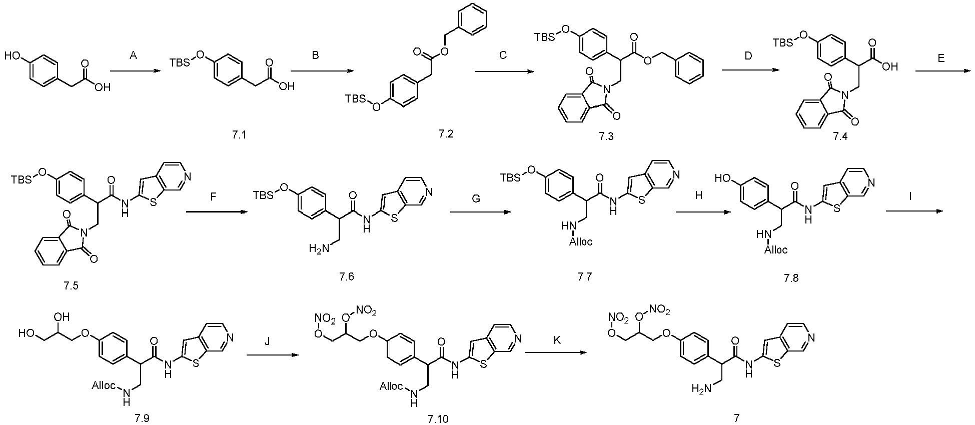

### Step A: 2-(4-((tert-butyldimethylsilyl)oxy)phenyl)acetic acid (compound 7.1)

p-hydroxyphenylacetic acid (5.0 g, 32.86 mmol) was dissolved in 400 mL of tetrahydrofuran. At 0 °C, imidazole (11 g, 164.3 mmol) and tert-butyldimethylchlorosilane (13.8 g, 92.00 mmol) were added, and the reaction solution was stirred at room temperature for 2 hours. 130 mL of saturated sodium carbonate solution was added to the reaction solution, and stirring was continued for 1 hour at room temperature. 2 M hydrochloric acid solution was added to the reaction solution to pH 4, extraction was performed with ethyl acetate (100 mLX2), the organic phases were combined, washed two times with saline water (200 mL), and dried with anhydrous sodium sulfate. The organic phases were filtered and concentrated under reduced pressure, and the residue was purified by column chromatography to obtain product 7.1 (5.78 g, yield: 66%, colorless oily substance).

### Step B: 2-(4-((tert-butyldimethylsilyl)oxy)phenyl)benzyl acetate (compound 7.2)

Compound 7.1 (5.78 g, 21.70 mmol) was dissolved in 100 mL of N,N-dimethylformamide, the solution was cooled to 0 °C, potassium carbonate (3.6 g, 26.04 mmol) and benzyl bromide (4.45 g, 26.04 mmol) were added, and the reaction solution was stirred at room temperature for 2 hours. 80 mL of water was added to the reaction solution, and extraction was performed with ethyl acetate (100 mLX3). The organic phases were combined, washed with saturated saline water (40 mL) four times, dried with anhydrous sodium sulfate, filtered, concentrated under reduced pressure, and the residue was purified by column chromatography to obtain product 7.2 (3.66 g, yield: 47%, colorless oily substance).

### Step C: 2-(4-((tert-butyldimethylsilyl)oxy)phenyl)-3-(1,3-dioxoisoindolin-2-yl)benzyl propionate (compound 7.3)

Compound 7.2 (3.66 g, 10.27 mmol) was dissolved in 30 mL of anhydrous tetrahydrofuran solution, and the reaction solution was cooled to -78 °C. Under nitrogen protection, 1 M lithium hexamethyldisilazide (12.3 mL, 12.3 mmol) was added dropwise, and the reaction solution was stirred at -78 °C for 1 hour under nitrogen protection. Then, 25 mL of a tetrahydrofuran solution of 2-(bromomethyl)isoindole-1,3-dione (2.96 g, 12.3 mmol) was added dropwise, and the reaction solution was stirred at -78 °C for 3 hours. 30 mL of water was added to the reaction solution, extraction was performed 3 times with ethyl acetate (60 mL), the organic phases were combined, washed two times with saturated saline water (40 mL), and dried with anhydrous sodium sulfate. The organic phases were filtered and the filtrate was concentrated under reduced pressure, and the residue was purified by column chromatography to obtain product 7.3 (3.9 g, yield: 74%, colorless oily substance). LCMS ESI(+)m/z:516.2(M+1).

### Step D: 2-(4-((tert-butyldimethylsilyl)oxy)phenyl)-3-(1,3-dioxoisoindolin-2-yl)propionic acid (compound 7.4)

Compound 7.3 (3.9 g, 7.56 mmol) was dissolved in 30 mL of tetrahydrofuran and 60 mL of methanol, palladium on carbon (600 mg, 10%) was added, and the reaction solution was stirred at room temperature under hydrogen protection for 3 hours. The reaction solution was filtered with diatomite, the filtrate was concentrated under reduced pressure, and the residue was purified by column chromatography to obtain product 7.4 (2.9 g, yield: 90%, yellow solid). LCMS ESI(+)m/z:426.2(M+1).

### Step E: 2-(4-((tert-butyldimethylsilyl)oxy)phenyl)-3-(1,3-dioxoisoindolin-2-yl)-N-(thieno[2,3-c]pyridin-2-yl)propionamide (compound 7.5)

Compound 7.4 (600 mg, 1.41 mmol) was dissolved in 20 mL of N,N-dimethylformamide, and diisopropylethylamine (276 mg, 2.11 mmol), 2-(7-benzotriazole oxide)-N,N,N",N'-tetramethylurea hexafluorophosphate (648 mg, 1.69 mmol), and thieno[2,3-c]pyridine-2-amine (233 mg, 1.55 mmol) were added. The reaction solution was stirred at 25 °C under nitrogen protection for 2 hours. Water (40 mL) was added to the reaction, and then extraction was performed with ethyl acetate (60 mLX2). The organic phases were combined, washed with saturated saline water (30 mLX2), and dried with anhydrous sodium sulfate. The organic phases were filtered, concentrated under reduced pressure, and purified by column chromatography to obtain product 7.5 (780 mg, yield: 99%, yellow solid). LCMS ESI(+)m/z:558.2(M+1).

### Step F: 3-amino-2-(4-((tert-butyldimethyl)oxy)phenyl)-N-(thieno[2,3-c]pyridin-2-yl)propionamide (compound 7.6)

Compound 7.5 (1.2 g, 2.15 mmol) was dissolved in 20 mL of 33% methylamine/ethanol solution, and the reaction solution was stirred at 50 °C for 1 hour. The reaction solution was concentrated under reduced pressure and purified by column chromatography to obtain compound 7.6 (655 mg, yield: 71%, yellow solid). LCMS ESI(+)m/z:428.2(M+1).

### Step G: allyl(2-(4-((tert-butyldimethylsilyl)oxy)phenyl)-3-oxo-3-(thieno[2,3-c]pyridin-2-ylamino)propyl)carbamate (compound 7.7)

Compound 7.6 (655 mg, 1.53 mmol) was dissolved in 30 mL of dichloromethane, triethylamine (620 mg, 6.12 mmol) and allyl chloroformate (369 mg, 3.06 mmol) were added, and the reaction solution was stirred at room temperature for 5 minutes. 30 mL of water was added to the reaction solution, then extraction was performed with dichloromethane (50 mLX2), the organic phases were separated and combined, and the organic phases were washed with saturated saline water (30 mL) one time, and dried with anhydrous sodium sulfate. After filtering, spin-drying, and purification by column chromatography, compound 7.7 was obtained (630 mg, yield: 80%, yellow solid). LCMS ESI(+)m/z:512.2(M+1).

### Step H: allyl(2-(4-hydroxyphenyl)-3-oxo-3-(thieno[2,3-c]pyridin-2-ylamino)propyl)carbamate (compound 7.8)

Compound 7.7 (630 mg, 1.23 mmol) was dissolved in 15 mL of methanol, potassium carbonate (255 mg, 1.84 mmol) was added, and the reaction solution was stirred at 40 °C for 1 hour. Saturated citric acid solution was added to the reaction solution to pH 7, and extraction was performed two times with ethyl acetate (60 mL), and the organic phases were separated and combined. The organic phases were washed with saturated saline water (30 mL) and dried with anhydrous sodium sulfate. After filtering, spin-drying, and purification by column chromatography, compound 7.8 was obtained (490 mg, yield: 100%, yellow solid). LCMS ESI(+)m/z:398.1(M+1).

### Step I: allyl(2-(4-(2,3-dihydroxypropoxy)phenyl)-3-oxo-3-(thieno[2,3-c]pyridin-2-ylamino)propyl)carbamate (compound 7.9)

Compound 7.8 (100 mg, 0.25 mmol) was dissolved in 10 mL of N,N-dimethylformamide, sodium hydroxide (24 mg, 0.60 mmol) and glycidol (46 mg, 0.60 mmol) were added, and the reaction solution was stirred at 50 °C for 14 hours. A 4 M hydrochloric acid/dioxane solution was added to the reaction solution to pH 6, and the reaction solution was concentrated under reduced pressure, and purified by column chromatography to obtain product 7.9 (35 mg, yield: 30%, white solid). LCMS ESI(+)m/z:472.1 (M+1).

### Step J: allyl(2-(4-(2,3-bis(nitrooxy)propoxy)phenyl)-3-oxo-3-(thieno[2,3-c]pyridin-2-ylamino)propyl)carbamate (compound 7.10)

30 drops of nitric acid solution were added to 40 mL of dichloromethane, the reaction solution was cooled to 0 °C, 7 drops of sulfuric acid were added, and the reaction solution was stirred at 0 °C for 1 hour. 20 mL of dichloromethane suspension of compound 7.9 (35 mg, 0.07mmol) was added dropwise to the reaction solution. The reaction solution was stirred at 40 °C for 16 hours. Saturated sodium bicarbonate solution was added to the reaction solution to adjust the pH to 8, extraction was performed with dichloromethane (30 mLX2), and the organic phases were combined, washed with saturated saline water (30 mLX2), and dried with anhydrous sodium sulfate. The organic phases were filtered and concentrated under reduced pressure, and the residue was purified by column chromatography to obtain product 7.10 (14 mg, yield: 34%, colorless oily substance). LCMS ESI(+)m/z:562.1(M+1).

### Step K: 3-(4-(3-amino-1-oxo-1-(thieno[2,3-c]pyridin-2-ylamino)prop-2-yl)phenoxy)propane-1,2-dinitrate (compound 7)

Compound 7.10 (14 mg, 0.02 mmol) was dissolved in 30 mL dichloromethane, 1,3-dimethylpyrimidine-2,4,6(1H,3H,5H)-trione(19 mg, 0.10 mmol) and tetrakistriphenylphosphine palladium (15 mg, 0.01 mmol) were added, and the reaction solution was stirred at 25 °C under nitrogen protection for 2 hours. The reaction solution was concentrated under reduced pressure, and the residue was purified by reverse preparation to obtain Example 7 (2.8 mg, yield: 24%, white solid). LCMS ESI(+)m/z:478.1(M+1).

### Example 8

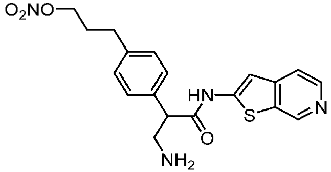

### 3-(4-(3-amino-1-oxo-1-(thieno[2,3-c]pyridin-2-ylamino)prop-2-yl)phenyl)propyl nitrate

The specific reaction equation is as follows:

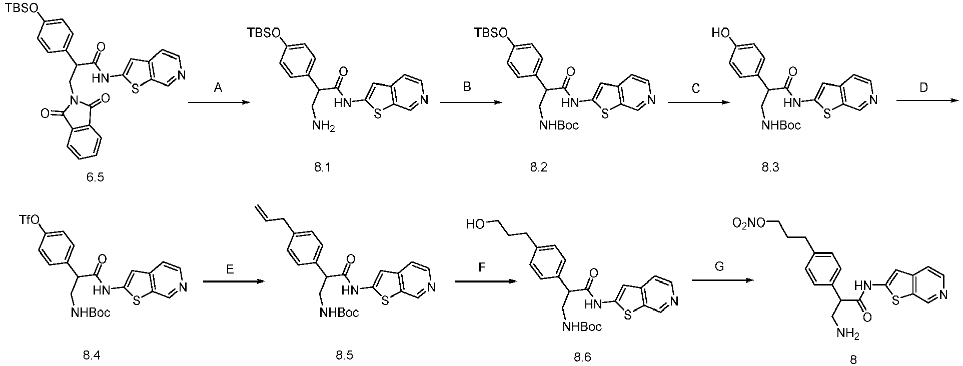

### Step A: 3-amino-2-(4-((tert-butyldimethyl)oxy)phenyl)-N-(thieno[2,3-c]pyridin-2-yl)propionamide (compound 8.1)

Compound 6.5 (1.5 g, 3.5mmol) was dissolved in 10 mL of methylamine ethanol solution to react at 50 °C for 1.5 hours. The reaction was monitored to be complete by LCMS. After the reaction was completed, water was added to quench the reaction, then extraction was performed with ethyl acetate (20 mLX3), and the organic phases were combined and washed with saturated saline water (50 mL). The organic phases were dried with anhydrous sodium sulfate, and filtered and purified by column chromatography to obtain product 8.1 (1.0 g, yield: 66%). LCMS ESI(+) m/z:428.2(M+1).

### Step B: (2-(4-((tert-butyldimethylsilyl)oxy)phenyl)-3-oxo-3-(thieno[2,3-c]pyridin-2-ylamino)propyl)tert-butyl carbamate (compound 8.2)

Compound 8.1 (1.0 g, 2.3 mmol) and N,N-diisopropylethylamine (0.7 mL, 3.5 mmol) were dissolved in 5 mL of N,N-dimethylformamide. Then, di-tert-butyl dicarbonate (0.8 mL, 3.5 mmol) was added to react at room temperature for 1 hour. The reaction was monitored to be complete by LCMS. After the reaction was completed, water was added to quench the reaction, then extraction was performed with ethyl acetate (20 mLX3), and the organic phases were combined and washed with saturated saline water (50 mL). The organic phases were dried with anhydrous sodium sulfate, and filtered and purified by column chromatography to obtain product 8.2 (1.2 g, yield: 100%). LCMS ESI(+) m/z:528.2(M+1).

### Step C: (2-(4-hydroxyphenyl)-3-oxo-3-(thieno[2,3-c]pyridin-2-ylamino)propyl)tert-butyl carbamate (compound 8.3)

Compound 8.2 (1.2 g, 2.3 mmol) was dissolved in 10 mL of methanol, then potassium carbonate (476 mg, 3.5 mmol) was added, and the temperature was raised to 50 °C to react for 2 hours. The reaction was monitored to be complete by LCMS. The reaction solution was concentrated and purified by column chromatography to obtain product 8.3 (940 mg, yield: 63%). LCMS ESI(+) m/z:414.1(M+1).

### Step D: 4-(3-((tert-butoxycarbonyl)amino)-1-oxo-1-(thieno[2,3-c]pyridin-2-ylamino)prop-2-yl)phenyl triflate (compound 8.4)

Compound 8.3 (940 mg, 1.5 mmol) and pyridine (474 mg, 6.0 mmol) were dissolved in 5 mL of dichloromethane, and then trifluoromethanesulfonic anhydride (846 mg, 3.0 mmol) was added dropwise at 0 °C to react at room temperature for 30 minutes. The reaction was monitored to be complete by LCMS. After the reaction was completed, water was added to quench the reaction, and then extraction was performed with dichloromethane (20 mLX3). The organic phases were combined, washed with saturated saline water (50 mL), and the organic phases were dried with anhydrous sodium sulfate. After filtering and purification by column chromatography, product 8.4 (220 mg, yield: 27%) was obtained. LCMS ESI(+) m/z:546.1(M+1).

### Step E: (2-(4-allylphenyl)-3-oxo-3-(thieno[2,3-c]pyridin-2-ylamino)propyl)tert-butyl carbamate (compound 8.5)

Compound 8.4 (100 mg, 0.18 mmol), 2-allyl-4,4,5,5-tetramethyl-1,3,2-dioxaborolane (37 mg, 0.22 mmol), and cesium carbonate (117 mg, 0.36 mmol) were dissolved in 9 mL of tetrahydrofuran and 1 mL of water. Then, [1,1'-bis(diphenylphosphino)ferrocene]palladium dichloride (6 mg, 5 mol%) was added, the flask was replaced with nitrogen, and the reaction solution was reacted overnight at 85 °C. The reaction was monitored to be complete by LCMS. After the reaction was completed, water was added to quench the reaction, then extraction was performed with ethyl acetate (10 mLX3), and the organic phases were combined and washed with saturated saline water (20 mL). The organic phases were dried with anhydrous sodium sulfate, and filtered and purified by column chromatography to obtain product 8.5 (78 mg, yield: 99%). LCMS ESI(+) m/z:438.2(M+1).

### Step F: (2-(4-(3-hydroxypropyl)phenyl)-3-oxo-3-(thieno[2,3-c]pyridin-2-ylamino)propyl)tert-butyl carbamate (compound 8.6)

Compound 8.5 (78 mg,0.17 mmol) was dissolved in 2 mL of tetrahydrofuran, and borane (10.0 M,0.02 mL,0.34 mmol) was added dropwise in an ice bath to react at 0 °C for 1 hour, and then again at room temperature for 1 hour. Then, 5 mL of aqueous sodium hydroxide (1 M) was added dropwise in an ice bath, and then 1 mL of hydrogen peroxide (30%) was added dropwise to react in the ice bath for 30 minutes, and then again at room temperature for 30 minutes. The reaction was monitored to be complete by LCMS. The reaction was quenched by adding water, and extraction was performed with ethyl acetate (10 mLX3). The organic phases were combined, washed with saturated saline water (20 mL), and the organic phases were dried with anhydrous sodium sulfate. After filtering, spin-drying, and purification by column chromatography, product 8.6 (20 mg, yield: 26%) was obtained. LCMS ESI(+) m/z:456.2(M+1).

### Step G: 3-(4-(3-amino-1-oxo-1-(thieno[2,3-c]pyridin-2-ylamino)prop-2-yl)phenyl)propyl nitrate (compound 8)

2 drops of nitric acid and 1 drop of acetic anhydride were dissolved in dichloromethane, and stirring was performed at room temperature for 10 minutes. Then, the dichloromethane solution of compound 8.6 (8 mg, 0.02 mmol) was slowly added dropwise into the system to react at room temperature for 1 hour. The reaction was monitored to be complete by LCMS. After the reaction was completed, the reaction solution was concentrated under reduced pressure. The residue was dissolved in 2 mL of methanol, filtered, and the filtrate was purified by reverse phase preparation. After lyophilization, target product Example 8 (1 mg, yield: 11%) was obtained. LCMS ESI(+) m/z: 401.1(M+1).

### Example 9

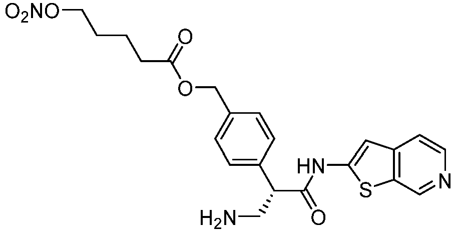

### (S)-4-(3-amino-1-oxo-l-(thieno[2,3-c]pyridin-2-ylamino)prop-2-yl)benzyl 5-(nitrooxy)valerate

The specific reaction equation is as follows

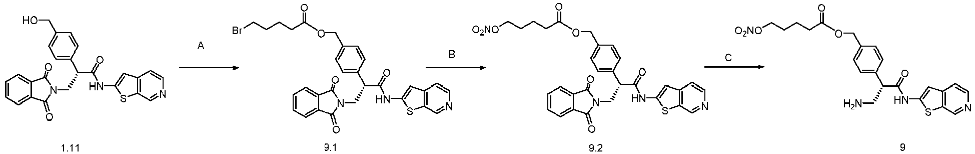

### Step A: (S)-4-(3-(1,3-dioxoisoindolin-2-yl)-1-oxo-1-(thieno[2,3-c]pyridin-2-ylamino)prop-2-yl)benzyl-5-valerate (compound 9.1)

Compound 1.11 (150 mg, 0.33 mmol) was dissolved in 10 mL of N,N-dimethylformamide, and dicyclohexylcarbodiimide (135 mg, 0.65 mmol), bromovaleric acid (60 mg, 0.33 mmol), and 4-dimethylaminopyridine (41 mg, 0.33 mmol) were added, and stirring was performed overnight at room temperature. The reaction was quenched with saturated aqueous ammonium chloride solution, extracted three times with 30 mL ethyl acetate, and the organic phases were combined, dried with anhydrous sodium sulfate, spin-dried, and purified by column chromatography to obtain product 9.1 (120 mg, yield: 59%). LCMS ESI(+)m/z:620.1,622.1(M+1).

### Step B: (S)-4-(3-(1,3-dioxoisoindolin-2-yl)-1-oxo-1-(thieno[2,3-c]pyridin-2-ylamino)prop-2-yl)benzyl 5-(nitrooxy)valeric acid (compound 9.2)

Compound 9.1 (120 mg, 0.19 mmol) was dissolved in 10 mL of acetonitrile solution, and silver nitrate (36 mg, 0.21 mmol) was added and stirring was performed overnight at 70 °C under nitrogen protection. The reaction solution was filtered by diatomite, spin-dried, and purified by column chromatography to obtain product 9.2 (40 mg, yield: 34%). LCMS ESI(+)m/z:603.1(M+1).

### Step C: (S)-4-(3-amino-1-oxo-1-(thieno[2,3-c]pyridin-2-ylamino)prop-2-yl)benzyl 5-(nitrooxy)valerate (compound 9)

Compound 9.2 (40 mg, 0.07 mmol) was dissolved in 10 mL of methylamine ethanol solution, stirring was performed at 60 °C for 3 hours, and the solvent was spin-dried. After reverse preparation and purification, Example 9 (10 mg, yield: 32%) was obtained. LCMS ESI(+)m/z:473.1(M+1). ¹H NMR (400 MHz, DMSO) δ 13.38 (s, 1H), 9.47 (s, 1H), 8.52 (d, J = 6.4 Hz, 1H), 8.20 -8.12 (m, 4H), 7.48 (d, J = 8.4 Hz, 2H), 7.39 (d, J = 9.2 Hz, 3H), 5.07 (s, 2H), 4.50 (t, J = 6.2 Hz, 2H), 4.44 (q, J = 8.2, 5.5 Hz, 1H), 3.66-3.59 (m, 1H), 3.20-3.13 (m, 1H), 2.41 (t, J = 7.2 Hz, 2H), 1.68-1.57 (m, 4H).

### Example 10

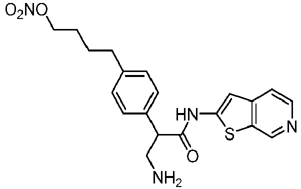

### 4-(4-(3-amino-1-oxo-1-(thieno[2,3-c]pyridin-2-ylamino)prop-2-yl)phenyl)butyl nitrate

The specific reaction equation is as follows:

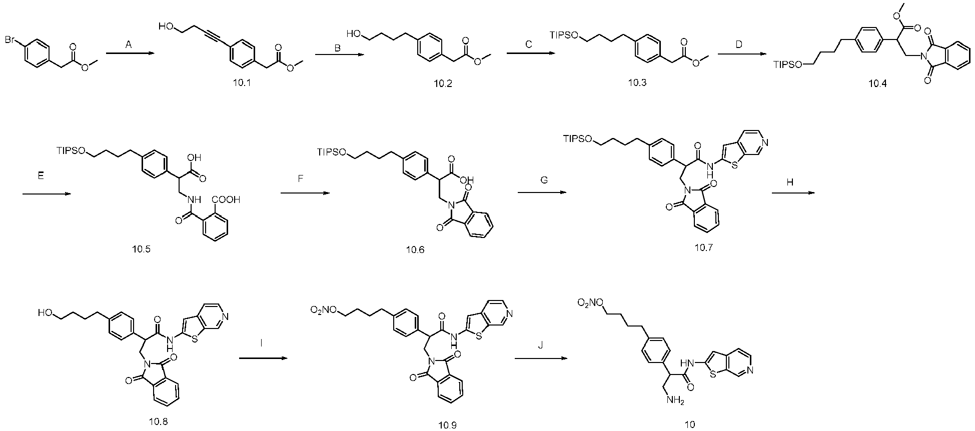

### Step A: methyl 2-(4-(4-hydroxybut-1-yn-1-yl)phenyl)acetate (compound 10.1)

Methyl 2-(4-bromophenyl)acetate (4.3 g, 18.7 mmol) and 3-butyn-1-ol (2.6 g, 37.4 mmol) were dissolved in 100 mL of triethylamine, then tetrakis(triphenylphosphine)palladium (864 mg, 4 mol%) and cuprous iodide (426 mg, 12 mol%) were added, and the flask was replaced with nitrogen to react at 100 °C for 2 hours. The reaction was monitored to be complete by LCMS. After filtering, the filtrate was concentrated under reduced pressure, spin-dried, and purified by column chromatography to obtain product 10.1 (2.8 g, yield: 68%). LCMS ESI(+) m/z:219.1(M+1).

### Step B: methyl 2-(4-(4-hydroxybutyl)phenyl)acetate (compound 10.2)

Compound 10.1 (2.8 g, 12.8 mmol) was dissolved in 20 mL of methanol, and then palladium/carbon (280 mg, 10%) was added, and the flask was replaced with hydrogen three times to react overnight at 40 °C. The reaction was monitored to be complete by LCMS. After filtering, the filtrate was concentrated under reduced pressure, spin-dried, and purified by column chromatography to obtain product 10.2 (2.0 g, yield: 70%). LCMS ESI(+) m/z:223.1(M+1).

### Step C: methyl 2-(4-(4-((triisopropylsilyl)oxy)butyl)phenyl)acetate (compound 10.3)

Compound 10.2 (2.0 g, 9.0 mmol) was dissolved in 10 mL of dichloromethane, and then 2,6-lutidine (1.6 mL, 13.5 mmol) and triisopropylsilyl triflate (3.0 mL, 10.8 mmol) were added to react overnight at room temperature. The raw material reaction was monitored to be complete by TLC. Water was added to quench the reaction, extraction was performed with dichloromethane (50 mLX3), and the organic phases were combined and washed with saturated saline water (100 mLX3). The organic phases were dried with anhydrous sodium sulfate, filtered, spin-dried, and purified by column chromatography to obtain product 10.3 (3.4 g, yield: 100%).

### Step D: methyl 3-(1,3-dioxoisoindolin-2-yl)-2-(4-(4-((triisopropylsilyl)oxy)butyl)phenyl)propionate (compound 10.4)

Compound 10.3 (3.4 g, 9.0 mmol) was dissolved in 20 mL of anhydrous tetrahydrofuran, the flask was replaced with nitrogen, the temperature was lowered to -78 °C with dry ice acetone, and lithium hexamethyldisilazide (1.0 M, 10.8 mL, 10.8 mmol) was slowly added dropwise. After the dropwise addition was completed, the system was stirred at -78 °C for 1 hour, N-bromomethyl phthalimide (2.6 g, 10.8 mmol) was dissolved in 10 mL of tetrahydrofuran, and the mixture was slowly added dropwise to the system. The temperature of the dropwise addition was controlled to be lower than -70 °C. After the dropwise addition was completed, the reaction was kept at -78 °C and stirred for 3 hours. The raw material reaction was monitored to be complete by LCMS. The reaction was quenched by adding saturated ammonium chloride solution (10 mL), extraction was performed with ethyl acetate (50 mLX3), and washing was performed with saturated saline water (100 mL). Drying was performed with anhydrous sodium sulfate, then filtering, spin-drying, and purification by column chromatography were performed to obtain product 10.4 (2.9 g, yield: 60%). LCMS ESI(+)m/z:538.3(M+1).

### Step E: 2-((2-carboxy-2-(4-(4-((triisopropylsilyl)oxy)butyl)phenyl)ethyl)carbamoyl)benzoic acid (compound 10.5)

Compound 10.4 (2.9 g, 5.4 mmol) was dissolved in 10 mL of tetrahydrofuran and 3 mL of water. Lithium hydroxide monohydrate (680 mg, 16.2 mmol) was added at 0 °C to stir at 0 °C for 3 hours. The reaction was monitored to be complete by LCMS. The organic solvent was distilled off under reduced pressure, the pH was adjusted to 3 to 4 with 1 M aqueous hydrochloric acid, and then extraction was performed with ethyl acetate (50 mLX3). The organic phases were combined, washed with saturated saline water (100 mLX3), and the organic phases were dried with anhydrous sodium sulfate, filtered, and spin-dried to obtain product 10.5 (2.8 g, yield: 94%). LCMS ESI(+)m/z:542.3(M+1).

### Step F: 3-(1,3-dioxoisoindolin-2-yl)-2-(4-(4-((triisopropylsilyl)oxy)butyl)phenyl)propionic acid (compound 10.6)

Compound 10.5 (2.8 g, 5.1 mmol), 1-hydroxybenzotriazole (688 mg, 5.1 mmol), and triethylamine (2.1 mL, 15.3 mmol) were dissolved in 10 mL of dichloromethane, and the reaction solution was put under nitrogen protection, and cooled to 0 °C. Then, 1-ethyl-(3-dimethylaminopropyl)carbodiimide hydrochloride (979 mg, 5.1 mmol) was added to the reaction solution, stirring was performed for a period of time, the reaction solution became clear, the temperature was slowly raised to room temperature, and the reaction solution was reacted overnight. The reaction was monitored to be complete by LCMS. Water was added to dilute the reaction solution, extraction was performed with dichloromethane (50 mLX3), and the organic phases were combined and washed with saturated saline water (100 mL). The organic phases were dried with anhydrous sodium sulfate, filtered, spin-dried, and purified by column chromatography to obtain product 10.6 (2.3 g, yield: 86%). LCMS ESI(+)m/z:524.3(M+1).

### Step G: 3-(1,3-dioxoisoindolin-2-yl)-N-(thieno[2,3-c]pyridin-2-yl)-2-(4-(4-((triisopropylsilyl)oxy)butyl)phenyl)propionamide (compound 10.7)

Compound 10.6 (300 mg, 0.57 mmol) was dissolved in 5 mL of N,N-dimethylformamide, and then 2-(7-benzotriazole oxide)-N,N,N',N'-tetramethylurea hexafluorophosphate (327 mg, 0.86 mmol) and N,N-diisopropylethylamine (147 mg, 1.1 mmol) were added successively. Stirring was performed at room temperature for 5 minutes, then compound 19.5 (102 mg, 0.68 mmol) was added to react at room temperature for 2 hours. The reaction was monitored to be complete by LCMS. After the reaction was completed, water was added to quench the reaction, then extraction was performed with ethyl acetate (20 mLX3), and the organic phases were combined and washed with saturated saline water (50 mL). The organic phases were dried with anhydrous sodium sulfate, filtered, spin-dried, and purified by column chromatography to obtain product 10.7 (373 mg, yield: 100%). LCMS ESI(+) m/z:656.3(M+1).

### Step H: 3-(1,3-dioxoisoindolin-2-yl)-2-(4-(4-hydroxybutyl)phenyl)-N-(thieno[2,3-c]pyridin-2-yl)propionamide (compound 10.8)

Compound 10.7 (373 mg, 0.69 mmol) was dissolved in 2 mL of tetrahydrofuran, and then the reaction solution was added to 6 mL of aqueous hydrochloric acid (1.0 M) to react at 40 °C overnight. The reaction was monitored to be complete by LCMS. After the reaction was completed, saturated sodium bicarbonate solution was added to neutralize, then extraction was performed with ethyl acetate (20 mLX3), and the organic phases were combined and washed with saturated saline water (50 mL). The organic phases were dried with anhydrous sodium sulfate, filtered, and spin-dried to obtain crude product 10.8 (270 mg, yield: 95%). LCMS ESI(+) m/z:500.2(M+1).

### Step I: 4-(4-(3-(1,3-dioxoisoindolin-2-yl)-1-oxo-1-(thieno[2,3-c]pyridin-2-ylamino)prop-2-yl)phenyl)butyl nitrate (compound 10.9)

4 drops of nitric acid and 2 drops of acetic anhydride were dissolved in dichloromethane, stirring was performed at room temperature for 10 minutes, and then the dichloromethane solution of compound 10.8 (30 mg, 0.06 mmol) was slowly added dropwise into the system. During the reaction, the compound was insoluble, and the same dichloromethane solution of nitric acid and acetic anhydride was placed in another reaction flask, and the insoluble reaction solution was slowly added dropwise. This process was repeated three to four times until all of the compound was dissolved. The reaction was monitored to be complete by LCMS. After the reaction was completed, water was added to quench the reaction, then extraction was performed with dichloromethane (10 mLX3), and the organic phases were combined and washed with saturated saline water (20 mL). The organic phases were dried with anhydrous sodium sulfate, and filtered and purified by column chromatography to obtain product 10.9 (22 mg, yield: 67%). LCMS ESI(+) m/z:545.1(M+1).

### Step J: 4-(4-(3-amino-1-oxo-1-(thieno[2,3-c]pyridin-2-ylamino)propan-2-yl)phenyl)butyl nitrate (compound 10)

Compound 10.9 (22 mg, 0.04 mmol) was dissolved in 1 mL of methanol, then hydrazine hydrate (20 mg, 0.40 mmol) was added to react overnight at 40 °C. The reaction was monitored to be complete by LCMS. After the reaction was completed, water was added to quench the reaction, and then extraction was performed with ethyl acetate (10 mL×3). The organic phases were combined and concentrated under reduced pressure. The residue was dissolved in 2 mL of methanol, filtered, and the filtrate was purified by reverse phase preparation. After lyophilization, target product Example 10 (2 mg, yield: 10%) was obtained. LCMS ESI(+) m/z:415.1(M+1).

### Example 11

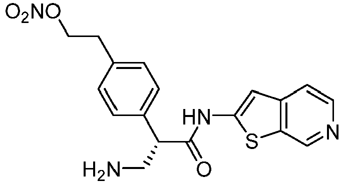

### (S)-4-(3-amino-1-oxo-1-(thieno[2,3-c]pyridin-2-ylamino)propan-2-yl)phenethyl nitrate

The specific reaction equation is as follows:

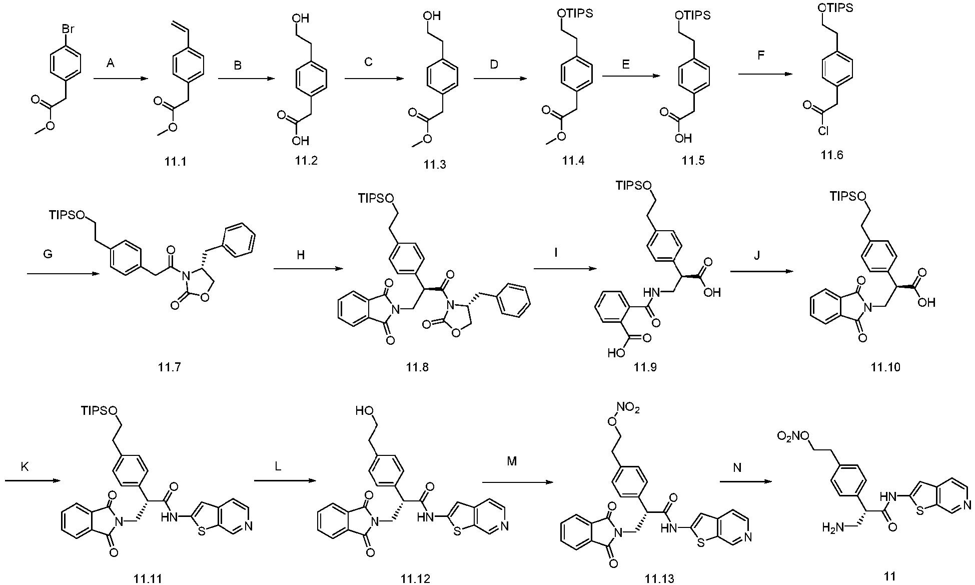

### Step A: methyl 2-(4-vinylphenyl)acetate (compound 11.1)

Methyl 2-(4-bromophenyl)acetate (10 g, 43.65 mmol) was dissolved in 200 mL of tetrahydrofuran and 20 mL of water, potassium vinyl trifluoroborate (7 g, 52.39 mmol), cesium carbonate (28.6 g, 87.31 mmol), and bis(triphenylphosphine) palladium chloride (600 mg, 0.87 mmol) were added, and the reaction solution was stirred at 78 °C under nitrogen protection for 16 hours. The reaction solution was cooled to room temperature, 100 mL of water was added to the reaction solution, and extraction was performed with ethyl acetate (400 mL) two times. The organic phases were combined, washed with saturated saline water (100 mL) two times, dried with anhydrous sodium sulfate, filtered, spin-dried, and purified by column chromatography to obtain compound 11.1 (5.6 g, yield: 73%, colorless oily substance).

### Step B: 2-(4-(2-hydroxyethyl)phenyl)acetic acid (compound 11.2)

Compound 11.1 (5.6 g, 31.78 mmol) was dissolved in 50 mL of tetrahydrofuran, the reaction was cooled to 0 °C, and 6.4 mL of 10 M borane dimethyl sulfide solution was added dropwise. The reaction solution was stirred at 0 °C under nitrogen protection for 1 hour, and then at 20 °C for 1 hour. The reaction solution was cooled to 0 °C, and 56 mL of 1 M sodium hydroxide solution and 30% hydrogen peroxide were added dropwise. The reaction solution was stirred at 0 °C for 0.5 hours, and then at 20 °C for 1 hour. 400 mL of saturated sodium sulfite solution was added to the reaction solution, then the pH was adjusted to 2 with 1 M hydrochloric acid solution, and extraction was performed with ethyl acetate (500 mL) five times. The organic phases were combined, dried with anhydrous sodium sulfate, filtered, spin-dried, and purified by column chromatography to obtain compound 11.2 (3.85 g, yield: 67%, white solid).

### Step C: methyl 2-(4-(2-hydroxyethyl)phenyl)acetate (compound 11.3)

Compound 11.2 (3.85 g, 21.37 mmol) was dissolved in 45 mL of dichloromethane and 10 mL of methanol, the reaction solution was cooled to 0 °C, 11.2 mL of 2 M trimethylsilyldiazomethane was added dropwise, and the reaction solution was stirred at room temperature for 0.5 hours. The reaction solution was concentrated under reduced pressure and purified by column chromatography to obtain compound 11.3 (2.4 g, yield: 58%, colorless oily substance).

### Step D: methyl 2-(4-(2-((triisopropylsilyl)oxy)ethyl)phenyl)acetate (compound 11.4)

Compound 11.3 (2.4 g, 12.36 mmol) was dissolved in 40 mL of dichloromethane, the reaction solution was cooled to 0 °C, 2,6-lutidine (1.99 g, 18.54 mmol) and triisopropylsilyl triflate (4.56 g, 14.83 mmol) were added, and the reaction solution was stirred at room temperature for 16 hours. 50 mL of water was added to the reaction solution, and extraction was performed with dichloromethane (80 mL) two times. The organic phases were separated and combined, washed with saturated saline water (30 mL) one time, dried with anhydrous sodium sulfate, filtered, spin-dried, and purified by column chromatography to obtain compound 11.4 (3.7 g, yield: 85%, colorless oily substance).

### Step E: 2-(4-(2-((triisopropylsilyl)oxy)ethyl)phenyl)acetic acid (compound 11.5)

Compound 11.4 (3.7 g, 10.55 mmol) was dissolved in 15 mL of water, 15 mL of methanol, and 40 mL of tetrahydrofuran, and lithium hydroxide monohydrate (1.33 g, 31.65 mmol) was added, and the reaction solution was stirred at room temperature for 2 hours. 1 M hydrochloric acid solution was added to the reaction solution to pH 3, and extraction was performed with ethyl acetate (100 mL) two times. The organic phases were combined, washed with saturated saline water (40 mL) two times, dried with anhydrous sodium sulfate, filtered, and concentrated under reduced pressure to obtain product 11.5 (3.43 g, yield: 97%, yellow solid).

### Step F: 2-(4-(2-((triisopropylsilyl)oxy)ethyl)phenyl)acetyl chloride (compound 11.6)

Compound 11.5 (3.43 g, 10.19 mmol) was dissolved in 40 mL of dichloromethane, the reaction solution was cooled to 0 °C, oxalyl chloride (1.55 g, 12.23 mmol) was added dropwise under nitrogen protection, and the reaction solution was stirred at room temperature for 12 hours. The reaction solution was concentrated under reduced pressure to obtain product 11.6 (3.62 g, yield: 100%, yellow oily substance).

### Step G: (R)-4-benzyl-3-(2-(4-(2-((triisopropylsilyl)oxy)ethyl)phenyl)acetyl)oxazolidin-2-one (compound 11.7)

(R)-4-benzyloxazolidin-2-one (1.72 g, 9.69 mmol) was dissolved in 30 mL of tetrahydrofuran, the reaction solution was cooled to -78 °C, and under nitrogen protection, 2.5 M n-butyllithium (4.28 mL, 10.71 mmol) was added dropwise, and the reaction solution was stirred at -78 °C for 1 hour. Then, 15 mL of a tetrahydrofuran solution of compound 11.6 (3.62 g, 10.20 mmol) was added dropwise to the reaction solution. The reaction solution was stirred at -78 °C for 2.5 hours. 30 mL of ammonium chloride solution was added to the reaction solution, and extraction was performed with ethyl acetate (150 mL) two times. The organic phases were combined, washed with saturated saline water (50 mL) two times, dried with anhydrous sodium sulfate, filtered, concentrated under reduced pressure, and the residue was purified by column chromatography to obtain product 11.7 (3.15 g, yield: 62%, colorless oily substance). LCMS ESI(+)m/z:496.3(M+1).

### Step H: 2-((R)-3-((R)-4-benzyl-2-oxooxazolidin-3-yl)-3-oxo-2-(4-(2-((triisopropylsilyl)oxy)ethyl)phenyl)propyl)isoindole-1,3-dione (compound 11.8)

Compound 11.7 (3.15 g, 6.35 mmol) was dissolved in 30 mL of anhydrous tetrahydrofuran at -78 °C, then lithium bistrimethylsilylamide (7.63 mL, 7.63 mmol) was added dropwise slowly into the reaction solution, and stirring was performed for 1 hour at this temperature. Right after, weighed N-bromomethylphthalimide (1.83 g, 7.63 mmol) was dissolved in anhydrous tetrahydrofuran (20 mL) and the reaction solution was added dropwise slowly to the reaction flask. Then, stirring was performed at this temperature for 2 hours. The reaction was quenched with saturated ammonium chloride solution, and extraction was performed two times with ethyl acetate (200 mL). The organic phases were separated and combined, and the organic phases were washed with saturated saline water (100 mL) two times, dried with anhydrous sodium sulfate, filtered, spin-dried, and purified by column chromatography to obtain compound 11.8 (2 g, yield: 48%, white solid). LCMS ESI(+) m/z:655.3(M+1).

### Step I: (R)-2-((2-carboxy-2-(4-(2-((triisopropylsilyl)oxy)ethyl)phenyl)ethyl)carbamoyl)benzoic acid (compound 11.9)

Compound 11.8 (2 g, 3.05 mmol) was dissolved in 25 mL of methanol and 25 mL of tetrahydrofuran. Then, lithium hydroxide monohydrate (385 mg, 9.16 mmol) was dissolved in water (20 mL) to be added dropwise into the reaction solution, and stirring was performed at room temperature for 3 hours. 60 mL of water was added to the reaction solution, the aqueous phase was washed two times with ethyl acetate (40 mL), and then the pH was adjusted to 4 with 1 M hydrochloric acid solution. Extraction was performed two times with ethyl acetate (100 mL), and the organic phases were separated and combined, dried with anhydrous sodium sulfate, filtered, and spin-dried to obtain compound 11.9 (980 mg, yield: 62%, white solid). LCMS ESI(+) m/z:514.2(M+1).

### Step J: (R)-3-(1,3-dioxoisoindolin-2-yl)-2-(4-(2-((triisopropylsilyl)oxy)ethyl)phenyl)propionic acid (compound 11.10)

Compound 11.9 (980 mg, 1.91 mmol) was dissolved in 30 mL of N,N-dimethylformamide, and 1-ethyl-(3-dimethylaminopropyl)carbodiimide hydrochloride (548 mg, 2.86 mmol), 1-hydroxybenzotriazole (387 mg, 2.86 mmol) and triethylamine (577 mg, 5.72 mmol) were added. The reaction solution was stirred at room temperature for 12 hours. The pH of the reaction solution was adjusted to 4 with 1 M hydrochloric acid solution, and extraction was performed with ethyl acetate (60 mL) two times. The organic phases were combined, washed with saturated saline water (30 mL) three times, dried with anhydrous sodium sulfate, filtered, concentrated under reduced pressure, and the residue was purified by column chromatography to obtain product 11.10 (840 mg, yield: 89%, yellow oily substance). LCMS ESI(+)m/z:496.2(M+1).

### Step K: (S)-3-(1,3-dioxoisoindolin-2-yl)-N-(thieno[2,3-c]pyridin-2-yl)-2-(4-(2-((triisopropylsilyl)oxy))ethyl)phenyl)propionamide (compound 11.11)

Compound 11.10 (840 mg, 1.69 mmol) was dissolved in 30 mL of N,N-dimethylformamide, and diisopropylethylamine (438 mg, 3.39 mmol), 2-(7-benzotriazole oxide)-N,N,N",N'-tetramethylurea hexafluorophosphate (967 mg, 2.54 mmol), and thieno[2,3-c]pyridine-2-amine (305 mg, 2.03 mmol) were added. The reaction solution was stirred at room temperature for 2 hours. 30 mL of water was added to the reaction solution, and extraction was performed with ethyl acetate (50 mL) two times. The organic phases were combined, washed with saturated saline water (30 mL) two times, dried with anhydrous sodium sulfate, filtered, concentrated under reduced pressure, and the residue was purified by column chromatography to obtain product 11.11 (580 mg, yield: 55%, yellow solid). LCMS ESI(+)m/z:628.3(M+1).

### Step L: (S)-3-(1,3-dioxoisoindolin-2-yl)-2-(4-(2-hydroxyethyl)phenyl)-N-(thieno[2,3-c]pyridin-2-yl)propionamide (compound 11.12)

Compound 11.11 (580 mg, 0.92 mmol) was dissolved in 50 mL of tetrahydrofuran, 20 mL of 1.5 M aqueous hydrochloric acid was added, and the reaction solution was stirred at 40 °C for 2 hours. The reaction solution was cooled to room temperature, and the pH was adjusted to 8 with saturated sodium bicarbonate solution, and extraction was performed with ethyl acetate (100 mL) two times. The organic phases were combined, washed with saturated saline water (30 mL) three times, dried with anhydrous sodium sulfate, filtered, concentrated under reduced pressure, and the residue was purified by column chromatography to obtain product 11.12 (350 mg, yield: 80%, yellow solid). LCMS ESI(+)m/z:472.1(M+1).

### Step M: (S)-4-(3-(1,3-dioxoisoindolin-2-yl)-1-oxo-1-(thieno[2,3-c]pyridin-2-ylamino)propan-2-yl)phenethyl nitrate (compound 11.13)

2 mL of nitric acid solution was added to 50 mL of dichloromethane, the reaction solution was cooled to 0 °C, 0.5 mL of sulfuric acid was added dropwise, and the reaction solution was stirred at 0 °C for 1 hour. 10 mL of dichloromethane suspension of compound 11.12 (250 mg, 0.53 mmol) was added dropwise to the reaction solution. The reaction solution was stirred at 40 °C for 8 hours. Saturated sodium bicarbonate solution was added to the reaction solution to adjust the pH to 8, and extraction was performed with dichloromethane (80 mL) two times. The organic phases were combined, washed with saturated saline water (30 mL) two times, dried with anhydrous sodium sulfate, filtered, concentrated under reduced pressure, and the residue was purified by column chromatography to obtain product 11.13 (250 mg, yield: 92%, yellow solid). LCMS ESI(+)m/z:517.1(M+1).

### Step N: (S)-4-(3-amino-1-oxo-1-(thieno[2,3-c]pyridin-2-ylamino)prop-2-yl) phenethyl nitrate (compound 11)

Compound 11.13 (250 mg, 0.48 mmol) was dissolved in 35 mL of ethanol, hydrazine hydrate (243 mg, 4.80 mmol) was added, and the reaction solution was stirred at 50 °C under nitrogen protection for 5 hours. 70 mL of water was added to the reaction solution, and extraction was performed with ethyl acetate (100 mL) two times. The organic phases were combined, washed with saturated saline water (40 mL) two times, dried with anhydrous sodium sulfate, filtered, concentrated under reduced pressure, and the residue was purified by reverse preparation to obtain product 11 (88 mg, yield: 47%, white solid). LCMS ESI(+)m/z:387.1 (M+1). ¹H NMR (400 MHz, DMSO-d6) δ12.75 (s, 1 H), 9.43 (s, 1 H), 8.52 (d, J = 6.4 Hz, 1 H), 8.07 (d, J = 6.4 Hz, 1 H), 8.01 (s, 2 H), 7.35 (s, 4 H), 7.26 (s, 1 H), 4.74 (t, J = 6.8 Hz, 2 H), 4.21 (dd, J = 5.4, 8.8 Hz, 1 H), 3.61 (s, 1 H), 3.18 (s, 1 H), 3.00 (t, J = 6.8 Hz, 2 H)

### Example 12

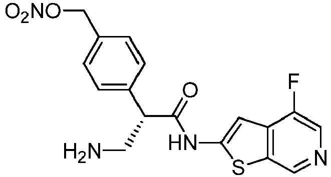

### (S)-4-(3-amino-1-((4-fluorothieno[2,3-c]pyridin-2-yl)amino)-1-oxoprop-2-yl)benzyl nitrate

The specific reaction equation is as follows:

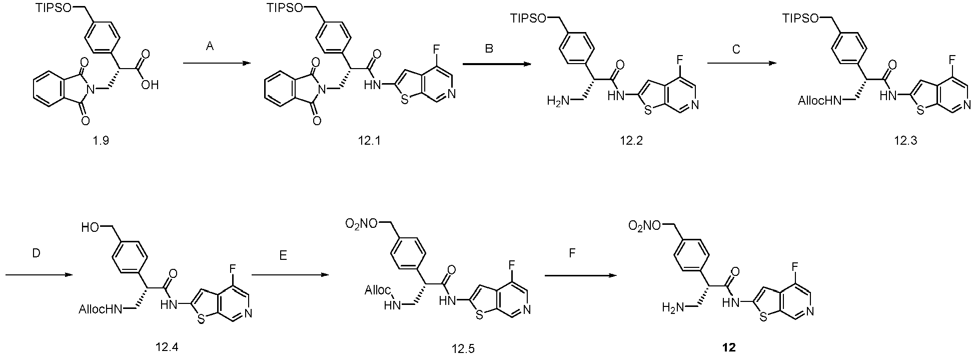

### Step A: (S)-3-(1,3-dioxoisoindolin-2-yl)-N-(4-fluorothieno[2,3-c]pyridin-2-yl)-2-(4-(((triisopropylsilyl))oxy)methyl)phenyl)propionamide (compound 12.1)

Compound 1.9 (400 mg, 0.83 mmol) was dissolved in 6 mL of N,N-dimethylformamide, and then a N,N-dimethylformamide solution of 50% 1-propylphosphoric anhydride (793 mg, 1.24 mmol), N,N-diisopropylethylamine (214 mg, 1.66 mmol), and 4-fluorothieno[2,3-c]pyridine-2-amine (154 mg, 0.92 mmol) were added to react by stirring at room temperature for 2 hours. The reaction was confirmed to be complete by LCMS. Water was added to the reaction flask, extraction was performed with ethyl acetate (10mLX2), washing was performed with saturated saline water, and drying was performed with anhydrous sodium sulfate. After filtering and purification by column chromatography, white solid 12.1 (300 mg, yield: 57%) was obtained. LCMS ESI(+) m/z:632.2(M+1).

### Step B: (S)-3-amino-N-(4-fluorothieno[2,3-c]pyridin-2-yl)-2-(4-(((triisopropylsilyl)oxy)methyl)phenyl)propionamide (compound 12.2)

Compound 12.1 (1.1 g, 1.74 mmol) was dissolved in ethanol (12 mL), then hydrazine hydrate (870 mg, 17.4 mmol) was added to the reaction solution, and the reaction was stirred at 55 °C for 2 hours under an inert gas atmosphere. The reaction was confirmed to be complete by LCMS. The reaction solution was concentrated and spin-dried with a vacuum pump, the residue was dissolved in ethyl acetate, and then the organic phase was washed with water and saturated saline water, and dried with anhydrous sodium sulfate. After filtering and concentrating in vacuo, light yellow solid product 12.2 was obtained (580 mg, yield: 66%). LCMS ESI(+) m/z:502.2(M+1).

### Step C: (S)-allyl(3-((4-fluorothieno[2,3-c]pyridin-2-yl)amino)-3-oxo-2-(4-(((triisopropylsilyl)oxy)methyl)phenyl)propyl)carbamate (compound 12.3)

Compound 12.2 (580 mg, 1.16 mmol) was dissolved in dichloromethane (10 mL), then weighed N,N-diisopropylethylamine (194 mg, 1.51 mmol) and allyl chloroformate (167 mg, 1.39 mmol) were added to the reaction flask, and the reaction was stirred at room temperature for 1 hour. The reaction was confirmed to be complete by LCMS. The reaction solution was diluted with dichloromethane, washed with saturated saline water, and dried with anhydrous sodium sulfate. After filtering and purification by column chromatography, light yellow solid 12.3 (520 mg, yield: 77%) was obtained. LCMS ESI(+) m/z:586.3(M+1).

### Step D: (S)-allyl(3-((4-fluorothieno[2,3-c]pyridin-2-yl)amino)-2-(4-(hydroxymethyl)phenyl)-3-oxopropyl)carbamate (compound 12.4)

Compound 12.3 (520 mg, 0.89 mmol) was dissolved in tetrahydrofuran (10 mL) and methanol (1 mL), then 1.5 mol/L of dilute hydrochloric acid (6 mL) was added to the reaction flask, and the reaction was placed in an oil bath at 60 °C and stirred for 2 hours. The reaction was confirmed to be complete by LCMS. Saturated sodium bicarbonate solution was added to the reaction flask, then extraction was performed with ethyl acetate (12 mL), washing was performed with saturated saline water, and drying was performed with anhydrous sodium sulfate. After filtering and concentration in vacuo, the crude product was slurried with methyl tert-butyl ether to obtain crude white solid 12.4 (400 mg, yield: 100%). LCMS ESI(+) m/z:430.1(M+1).

### Step E: (S)-allyl(3-((4-fluorothieno[2,3-c]pyridin-2-yl)amino)-2-(4-((nitrooxy)methyl)phenyl)-3-oxopropyl)carbamate (compound 12.5)

Acetic anhydride (15 drops) and concentrated nitric acid (30 drops) were dissolved in 50 mL of dichloromethane. The reaction solution was stirred at room temperature for 20 minutes, then compound 12.4 (320 mg, 0.75 mmol) was added to the reaction solution, and stirring was continued at room temperature for 50 minutes. The reaction was detected to be complete by LCMS. Saturated sodium bicarbonate solution was added to the reaction flask, then extraction was performed with dichloromethane (15 mL), washing was performed with saturated saline water, and the organic phase was dried with anhydrous sodium sulfate. After filtering and spin-drying, the residue was purified by column chromatography to obtain white solid 12.5 (123 mg, yield: 35%). LCMS ESI(+) m/z:475.1(M+1).

### Step F: (S)-4-(3-amino-1-((4-fluorothieno[2,3-c]pyridin-2-yl)amino)-1-oxoprop-2-yl)benzyl nitrate (compound 12)

Compound 12.5 (123 mg, 0.26 mmol) was dissolved in 8 mL of dichloromethane, then tetrakis(triphenylphosphine)palladium (30 mg, 0.026 mmol) and 1,3-dimethylbarbituric acid (44.6 mg, 0.286 mmol) were added to the reaction solution, and stirring was performed at room temperature for 1 hour. The reaction was detected to be complete by LCMS. Saturated sodium bicarbonate solution was added to the reaction flask, then extraction was performed with dichloromethane (12 mL), and the organic phase was washed with saturated saline water, and dried with anhydrous sodium sulfate. After filtering and spin-drying, the residue was subjected to reverse preparation and lyophilized to obtain white solid 12 (65 mg, yield: 50%, purity: 98%). LCMS ESI(+) m/z:391.1(M+1).

### Example 13

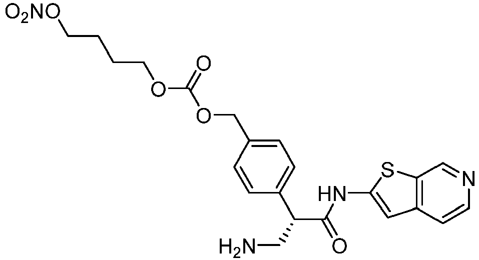

### (S)-4-(3-amino-1-oxo-1-(thieno[2,3-c]pyridin-2-ylamino)prop-2-yl)benzyl(4-(nitrooxy)butyl)carbonate

The specific reaction equation is as follows:

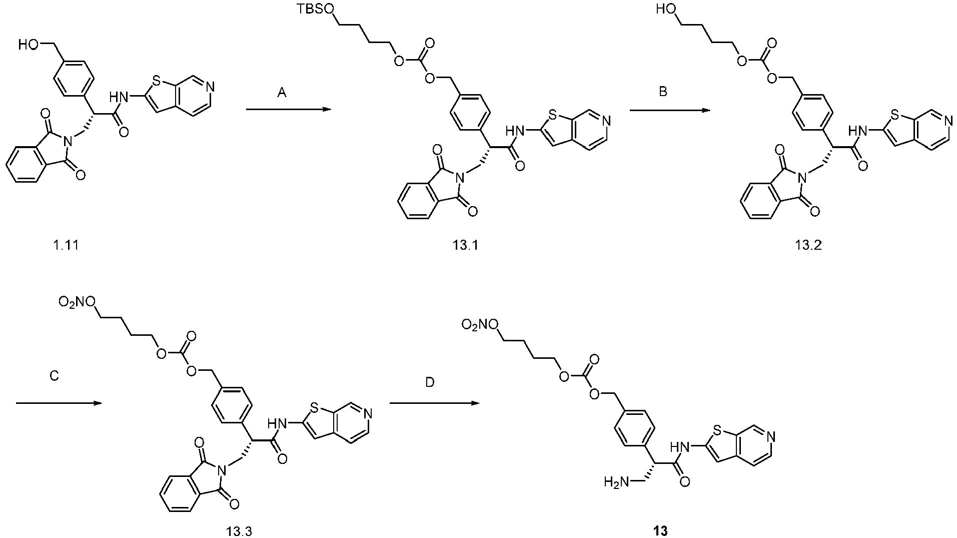

### Step A: (S)-4-((tert-butyldimethylsilyl)oxy)butyl 4-(3-(1,3-dioxoisoindolin-2-yl)-1-oxo-1-(thieno[2,3-c]pyridine-2)-amino)prop-2-yl)benzyl carbonate (compound 13.1)

Under an ice bath, 4-((tert-butyldimethylsilyl)oxy)butan-1-ol (100 mg, 0.49 mmol) was dissolved in dichloromethane (8 mL), then triphosgene (87 mg, 0.29 mmol) and pyridine (46 mg, 0.59 mmol) were added to the reaction solution, and then stirring was performed at room temperature for 3 hours. Then, compound 1.11 (40 mg, 0.088 mmol) and pyridine (7 mg, 0.096 mmol) were added to the reaction flask and stirring was performed overnight at room temperature. The reaction was confirmed to be complete by LCMS. The reaction solution was diluted with dichloromethane, washed successively with saturated sodium bicarbonate, 0.5 mol/L of dilute hydrochloric acid, and saturated saline water, dried with anhydrous sodium sulfate, filtered, and purified by column chromatography to obtain light yellow oily product 13.1 (25 mg, yield: 42%). LCMS ESI(+) m/z:688.2(M+1).

### Step B: (S)-4-(3-(1,3-dioxoisoindolin-2-yl)-1-oxo-1-(thieno[2,3-c]pyridin-2-ylamino)prop-2-yl)benzyl(4-hydroxybutyl)carbonate (13.2)

Compound 13.1 (25 mg, 0.036 mmol) was dissolved in tetrahydrofuran (5 mL) and methanol (1 mL), then 4 mol/L of hydrochloric acid-dioxane solution (0.1 mL) was added to the reaction flask to react by stirring at room temperature for 10 minutes. The reaction was confirmed to be complete by LCMS, and the reaction solution was concentrated in vacuo to obtain crude white solid 13.2 (15 mg, yield: 72%). LCMS ESI(+) m/z:574.2(M+1).

### Step C: S)-4-(3-(1,3-dioxoisoindolin-2-yl)-1-oxo-1-(thieno[2,3-c]pyridin-2-ylamino)prop-2-yl)benzyl(4-(nitrooxy)butyl)carbonate (compound 13.3)

Acetic anhydride (1 drop) and concentrated nitric acid (2 drops) were dissolved in dichloromethane (10 mL). The reaction solution was stirred at room temperature for 20 minutes, then compound 13.2 (15 mg, 0.026 mmol) was added to the reaction flask, and the reaction was continued by stirring at room temperature for 20 minutes. The reaction was confirmed to be complete by LCMS. Saturated sodium bicarbonate solution was added to the reaction flask, then extraction was performed with dichloromethane (10 mL), washing was performed with saturated saline water, and drying was performed with anhydrous sodium sulfate. After filtering and column chromatography, light yellow oily product 13.3 (5 mg, yield: 31%) was obtained. LCMS ESI(+) m/z:619.1(M+1).

### Step D: (S)-4-(3-amino-1-oxo-1-(thieno[2,3-c]pyridin-2-ylamino)prop-2-yl)benzyl(4-(nitrooxy)butyl)carbonate (compound 13)

Compound 13.3 (5 mg, 0.008 mmol) was dissolved in ethanol (5 mL), then hydrazine hydrate (1 drop) was added to the reaction solution, and the reaction was stirred at 55 °C for 1.5 hours under an inert gas atmosphere. The reaction was confirmed to be complete by LCMS. Water was added to the reaction solution, extraction was performed with ethyl acetate (10 mL), washing was performed with saturated saline water, drying was performed with anhydrous sodium sulfate, then filtering, concentration, and spin-drying were performed. The residue was dissolved in methanol (1 mL), and after reverse preparation and purification, white solid Example 13 (1.5 mg (trifluoroacetate), yield: 31%, purity: 95%) was obtained. LCMS ESI(+) m/z:489.1(M+1).

### Example 14

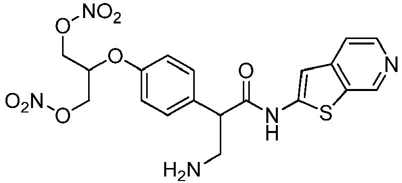

### 2-(4-(3-amino-1-oxo-1-(thieno[2,3-c]pyridin-2-ylamino)propan-2-yl)phenoxy)propane-1,3-dinitrate

The specific reaction equation is as follows:

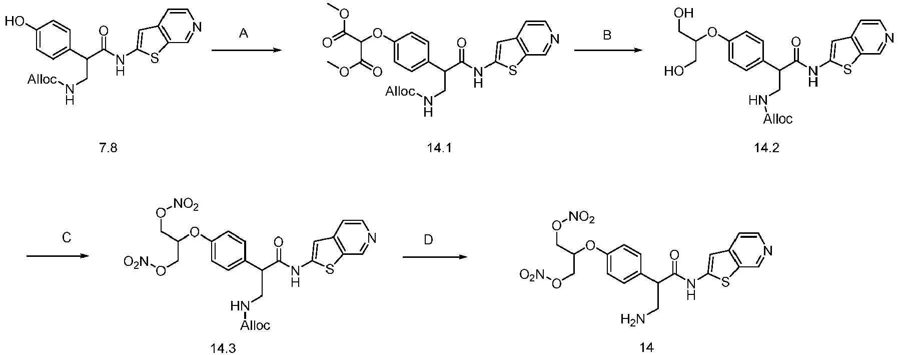

### Step A: 2-(4-(3-(((allyloxy)carbonyl)amino)-1-oxo-1-(thieno[2,3-c]pyridin-2-ylamino)prop-2-yl)phenoxy)dimethyl malonate (compound 14.1)

Compound 7.8 (500 mg, 1.26 mmol) was dissolved in 20 mL of N,N-dimethylformamide, and dimethyl 2-bromomalonate (319 mg, 1.51 mmol) and potassium carbonate (260 mg, 1.89 mmol) were added. The reaction solution was stirred at 50 °C for 1.5 hours. 25 mL of water was added to the reaction solution, and extraction was performed with ethyl acetate (80 mL) two times. The organic phases were combined, washed with saturated saline water (40 mL) two times, dried with anhydrous sodium sulfate, filtered, concentrated under reduced pressure, and the residue was purified by column chromatography to obtain product 14.1 (250 mg, yield: 38%, yellow oily substance). LCMS ESI(+)m/z:528.1(M+1).

### Step B: allyl(2-(4-((1,3-dihydroxypropan-2-yl)oxy)phenyl)-3-oxo-3-(thieno[2,3-c]pyridin-2-ylamino)propyl)carbamate (compound 14.2)

Compound 14.1 (250 mg, 0.47 mmol) was dissolved in 15 mL of tetrahydrofuran, the reaction solution was cooled to 0 °C, and sodium borohydride (107 mg, 2.84 mol) was added in batches. The reaction solution was stirred at 0 °C for 20 minutes, 15 mL of methanol was added dropwise, and the reaction solution was stirred at room temperature for 1 hour. Lithium borohydride (100 mg, 4.70 mmol) was added to the reaction solution, and the reaction solution was stirred at 50 °C for 3 hours. 1 M hydrochloric acid solution was added to the reaction solution to pH 6, the reaction solution was concentrated under reduced pressure, and the residue was purified by reverse preparation to obtain product 14.2 (130 mg, yield: 58%, white solid). LCMS ESI(+)m/z:472.1(M+1).

### Step C: allyl (2-(4-((1,3-bis(nitrooxy)prop-2-yl)oxy)phenyl)-3-oxo-3-(thieno[2,3-c]pyridin-2-ylamino)propyl)carbamate (compound 14.3)

3 mL of nitric acid solution was added to 100 mL of dichloromethane, the reaction solution was cooled to 0 °C, 0.7 mL of sulfuric acid was added dropwise, and the reaction solution was stirred at 0 °C for 1 hour. Compound 14.2 (130 mg, 0.27 mmol) was added to the reaction solution, and the reaction solution was stirred at 40 °C for 4 hours. Saturated sodium bicarbonate solution was added to the reaction solution to adjust the pH to 8, and extraction was performed with dichloromethane (80 mL) two times. The organic phases were combined, washed with saturated saline water (30 mL) two times, dried with anhydrous sodium sulfate, filtered, concentrated under reduced pressure, and the residue was purified by column chromatography to obtain product 14.3 (8 mg, yield: 5%, yellow solid). LCMS ESI(+)m/z:562.1(M+1).

### Step D: 2-(4-(3-amino-1-oxo-1-(thieno[2,3-c]pyridin-2-ylamino)prop-2-yl)phenoxy)propane-1,3-dinitrate (compound 14)

Compound 14.3 (8 mg, 0.01 mmol) was dissolved in 8 mL dichloromethane, 1,3-dimethylpyrimidine-2,4,6(1H,3H,5H)-trione(2.7 mg, 0.02 mmol) and tetrakistriphenylphosphine palladium (2 mg, 0.002 mmol) were added, and the reaction solution was stirred at room temperature under nitrogen protection for 0.5 hours. The reaction solution was concentrated under reduced pressure, and the residue was purified by reverse preparation to obtain Example 14 (6.5 mg, yield: 96%, white solid). LCMS ESI(+)m/z:478.1(M+1).

### Example 15

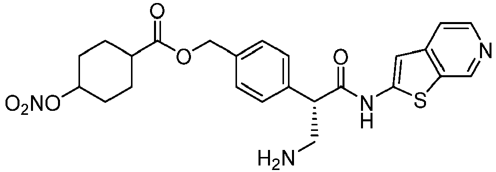

### (S)-4-(3-amino-1-oxy-1-(thieno[2,3-c]pyridin-2-ylamino)prop-2-yl)benzyl 4-(nitrooxy)cyclohexane- 1-carboxylic acid

The specific reaction equation is as follows:

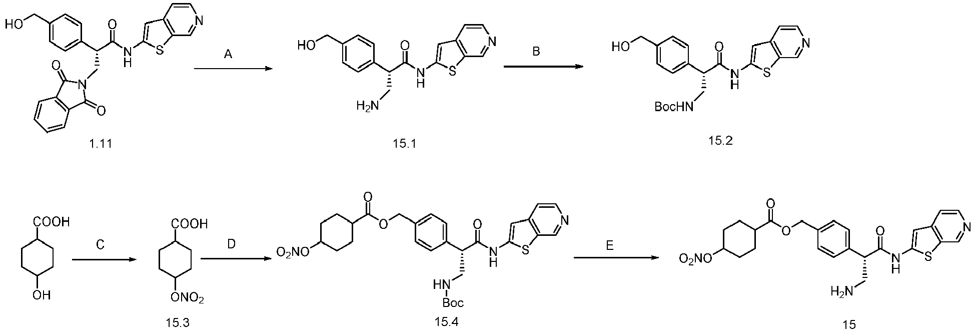

### Step A: (S)-3-amino-2-(4-(hydroxymethyl)phenyl)-N-(thieno[2,3-c]pyridin-2-yl)propionamide (compound 15.1)

Compound 1.11 (6 g, 13.13 mmol) was dissolved in ethanol (130 mL) and N,N-dimethylformamide (25 mL), then hydrazine hydrate (6.56 g, 131.3 mmol) was added to the reaction solution to react at 60 °C by stirring for 3 hours. The reaction was confirmed to be complete by LCMS, and the reaction solution was cooled to room temperature and then filtered by diatomite, and the filtrate was concentrated in vacuo and spin-dried to obtain crude compound 15.1 (5 g). LCMS ESI(+) m/z:328.1(M+1).

### Step B: (S)-tert-butyl(2-(4-(hydroxymethyl)phenyl)-3-oxo-3-(thieno[2,3-c]pyridin-2-ylamino)propyl)carbamate (compound 15.2)

Crude compound 15.1 (5 g, 15.3 mmol) was dissolved in 60 mL of N,N-dimethylformamide, and triethylamine (6.18 g, 61.2 mmol) and di-tert-butyl dicarbonate (6.67 g, 30.6 mmol) were added to react at room temperature by stirring for 0.5 hours. The reaction was confirmed to be complete by LCMS, water was added to the reaction solution, extraction was performed with ethyl acetate (35 mL), and the organic phase was washed with saturated saline water, and dried with anhydrous sodium sulfate. After filtering, concentration, and spin-drying, the residue was purified by silica column to obtain light yellow solid product 15.2 (3.5 g, purity: 97%). LCMS ESI(+) m/z:428.1(M+1).

### Step C: 4-(nitrooxy)cyclohexane-1-carboxylic acid (compound 15.3)

4-hydroxycyclohexane-1-carboxylic acid (200 mg, 1.39 mmol) was dissolved in 1 mL of acetic anhydride, a mixed acid solution of concentrated nitric acid and acetic anhydride (0.2 mL of nitric acid, 0.4 mL of acetic anhydride) was added under an ice-water bath, and stirring was performed at 0 °C for 1 hour. The reaction was monitored to be complete by TLC. After the reaction was completed, 20 mL of water was added to quench the reaction, then extraction was performed with ethyl acetate (10 mLX3), and the organic phases were combined and washed with saturated saline water (10 mLX2). The organic phases were dried with anhydrous sodium sulfate, filtered, spin-dried, and purified by column chromatography to obtain product 15.3 (210 mg, yield: 80%).

### Step D: (S)-4-(3-((tert-butoxycarbonyl)amino)-1-oxo-1-(thieno[2,3-c]pyridin-2-ylamino)prop-2-yl)benzyl 4-(nitrooxy))-1-carboxylic acid cyclohexane (compound 15.4)

Compound 15.2 (100 mg, 0.23 mmol) and compound 15.3 (49 mg, 0.26 mmol) were dissolved in 3 mL of N,N-dimethylformamide. Then, 1-ethyl-(3-dimethylaminopropyl)carbodiimide hydrochloride (68 mg, 0.35 mmol) and 4-dimethylaminopyridine (28 mg, 0.23 mmol) were added to react overnight at room temperature. The reaction was monitored to be complete by TLC, water was added to quench the reaction, then extraction was performed with ethyl acetate (20 mLX2), and the organic phases were combined and washed with saturated saline water (10 mLX2). The organic phases were dried with anhydrous sodium sulfate, filtered, spin-dried, and purified by column chromatography to obtain product 15.4 (127 mg, yield: 91%).

### Step E: (S)-4-(3-amino-1-oxy-1-(thieno[2,3-c]pyridin-2-ylamino)prop-2-yl)benzyl 4-(nitrooxy)cyclohexane-1-carboxylic acid (compound 15)

Compound 15.4 (127 mg, 0.215 mmol) was dissolved in 5 mL of methanol, 2 mL of hydrochloric acid methanol solution (4 M) was added, and the reaction solution was stirred at room temperature for 1 hour, then the system was heated to 50 °C to react for 1 hour. The reaction was monitored to be complete by TLC. After the reaction was completed, the reaction solution was concentrated under reduced pressure. The residue was dissolved in 5 mL of methanol and aqueous solution, filtered, and the filtrate was purified by reverse phase preparation. After lyophilization, target product Example 15 (19 mg, yield: 18%, purity: 97%) was obtained. LCMS ESI(+) m/z: 499.2(M+1).¹H NMR (400 MHz, DMSO-*d*₆) δ 13.36 (d, J = 10.0 Hz, 1H), 9.46 (s, 1H), 8.52 (d, J = 6.5 Hz, 1H), 8.12 - 8.06 (m, 1H), 7.48 (d, J = 8.2 Hz, 2H), 7.39 (d, J = 8.6 Hz, 3H), 5.17 (s, 1H), 5.08 (d, J = 3.9 Hz, 2H), 5.05 - 4.88 (m, 1H), 4.54 - 4.30 (m, 1H), 3.63 (s, 1H), 3.18 (s, 2H), 2.02 (dd, J = 21.9, 11.0 Hz, 3H), 1.93 - 1.68 (m, 2H), 1.68 - 1.33 (m, 4H).

### Example 16

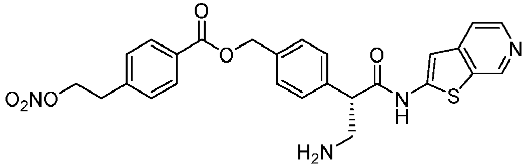

### (S)-4-(3-amino-1-oxy-1-(thieno[2,3-c]pyridin-2-ylamino)prop-2-yl)benzyl 4-(2-(nitrooxy)ethyl)benzoate

The specific reaction equation is as follows:

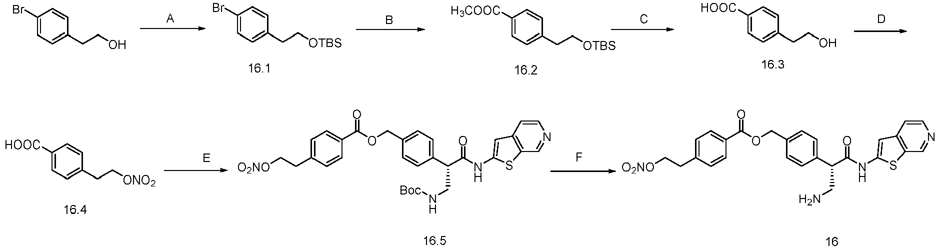

### Step A: (4-bromophenethoxy)(tert-butyl)dimethylsilane (compound 16.1)

4-bromophenethyl alcohol (2 g, 9.95 mmol) and imidazole (1.7 g, 25 mmol) were dissolved in 20 mL of N,N-dimethylformamide, and tert-butyldimethylchlorosilane (3 g, 19.9 mmol) was added in batches under an ice-water bath, and then the system was stirred at room temperature for 2 hours. The reaction was monitored to be complete by TLC. After the reaction was completed, water was added to quench the reaction, then extraction was performed with ethyl acetate (30 mLX3), and the organic phases were combined and washed with saturated saline water (40 mLX2). The organic phases were dried with anhydrous sodium sulfate, filtered, spin-dried, and purified by column chromatography to obtain product 16.1 (2.82 g, yield: 90%).

### Step B: methyl 4-(2-(((tert-butyldimethylsilyl)oxy)ethyl)benzoate (compound 16.2)

Compound 16.1 (2.82 g, 8.95 mmol) and [1,1'-bis(diphenylphosphino)ferrocene]palladium dichloride (659 mg, 0.1 mmol) were dissolved in 30 mL of N,N-dimethylformamide and 90 mL of methanol mixed solvent, and then 10 mL of triethylamine was added. The system was reacted overnight at 80 °C under a CO atmosphere. The reaction was monitored to be complete by LCMS. After the reaction was completed, water was added to quench the reaction, then extraction was performed with ethyl acetate (40 mLX3), and the organic phases were combined and washed with saturated saline water (40 mLX2). The organic phases were dried with anhydrous sodium sulfate, filtered, spin-dried, and purified by column chromatography to obtain product 16.2 (1.84 g, yield: 70%).

### Step C: 4-(2-(((tert-butyldimethylsilyl)oxy)ethyl)benzoic acid (compound 16.3)

Compound 16.2 (736 mg, 2.5 mmol) was dissolved in 5 mL of methanol, then lithium hydroxide monohydrate (315 mg, 7.5 mmol) was added to react at room temperature overnight. The reaction was monitored to be complete by LCMS. After the reaction was completed, water was added to quench the reaction, the pH was adjusted to 3 with 6N aqueous hydrochloric acid and stirring was performed for half an hour, then extraction was performed with ethyl acetate (30 mLX2). The organic phases were combined, washed with saturated saline water (20 mLX2), and the organic phases were dried with anhydrous sodium sulfate, filtered, spin-dried, and purified by column chromatography to obtain product 16.3 (254 mg, yield: 61%).

### Step D: 4-(2-(nitrooxy)ethyl)benzoic acid (compound 16.4)

Compound 16.3 (100 mg, 0.6 mmol) was dissolved in 3 mL of dichloromethane, and a mixed acid solution of concentrated nitric acid and concentrated sulfuric acid (0.4 mL of concentrated nitric acid, 0.1 mL of concentrated sulfuric acid) was added in an ice-water bath to react at room temperature for 2 hours. The reaction was monitored to be complete by TLC. After the reaction was completed, water was added to quench the reaction, then extraction was performed with dichloromethane (15 mLX2), and the organic phases were combined and washed with saturated saline water (10 mLX2). The organic phases were dried with anhydrous sodium sulfate, and filtered, spin-dried, and purified by column chromatography to obtain product 16.4 (92 mg, yield: 73%).

### Step E: (S)-4-(3-((tert-butoxycarbonyl)amino)-1-oxo-1-(thieno[2,3-c]pyridin-2-ylamino)propan-2-yl)benzyl 4-(2-(nitrooxy)ethyl)benzoate (compound 16.5)

Compound 15.2 (100 mg, 0.234 mmol) and compound 16.4 (55 mg, 0.258 mmol) were dissolved in 3 mL of N,N-dimethylformamide. Then, 1-ethyl-(3-dimethylaminopropyl)carbodiimide hydrochloride (67 mg, 0.351 mmol) and 4-dimethylaminopyridine (28 mg, 0.234 mmol) were added to react overnight at room temperature. The reaction was monitored to be complete by TLC. Water was added to quench the reaction, extraction was performed with ethyl acetate (20 mLX2), and the organic phases were combined and washed with saturated saline water (20 mLX2). The organic phases were dried with anhydrous sodium sulfate, filtered, spin-dried, and purified by column chromatography to obtain product 16.5 (117 mg, yield: 81%).

### Step F: (S)-4-(3-amino-1-oxy-1-(thieno[2,3-c]pyridin-2-ylamino)prop-2-yl)benzyl 4-(2-(nitrooxy)ethyl)benzoate (compound 16)

Compound 16.5 (117 mg, 0.19 mmol) was dissolved in 5 mL of methanol, 1.5 mL of hydrochloric acid methanol solution (4 M) was added, and the reaction solution was first stirred at room temperature for 1 hour, then the system was heated to 50 °C to react for 1 hour. The reaction was monitored to be complete by TLC. After the reaction was completed, the reaction solution was concentrated under reduced pressure. The residue was dissolved in 5 mL of methanol and aqueous solution, filtered, and the filtrate was purified by reverse phase preparation. After lyophilization, target product Example 16 (83 mg, yield: 84%, purity: 94%) was obtained. LCMS ESI(+) m/z: 521.2(M+1).¹H NMR (400 MHz, DMSO-*d*₆) δ 9.47 (s, 1H), 8.52 (d, J = 6.4 Hz, 1H), 8.22 (s, 3H), 8.14 (d, J = 6.4 Hz, 1H), 7.93 (d, J = 8.4 Hz, 2H), 7.51 (d, J = 3.2 Hz, 3H), 7.44 (d, J = 8.4 Hz, 2H), 7.39 (s, 1H), 5.33 (s, 2H), 4.78 (t, J = 6.4 Hz, 2H), 4.48 (dd, J = 8.4, 5.6 Hz, 1H), 3.64 (s, 1H), 3.18 (d, J = 6.0 Hz, 1H), 3.09 (t, J = 6.4 Hz, 2H).

### Example 17

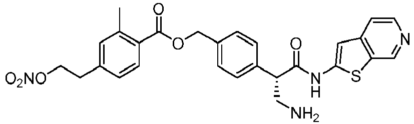

### (S)-4-(3-amino-1-oxy-1-(thieno[2,3-c]pyridin-2-ylamino)prop-2-yl)benzyl 2-methyl-4-(2-(nitrooxy))ethyl)benzoate

The specific reaction equation is as follows:

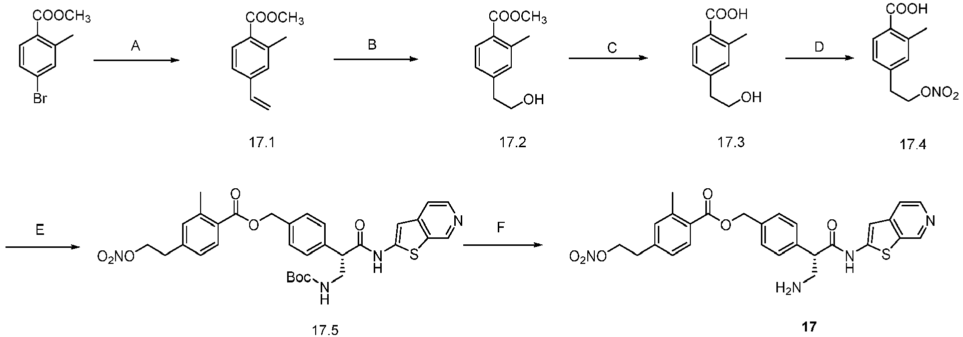

### Step A: methyl 2-methyl-4-vinylbenzoate (compound 17.1)

Methyl 4-bromo-2-methylbenzoate (2 g, 8.73 mmol) was dissolved in 40 mL of tetrahydrofuran and 4 mL of water, potassium vinyl trifluoroborate (1.4 g, 10.48 mmol), cesium carbonate (5.7 g, 17.46 mmol), and bis(triphenylphosphine) palladium chloride (122 mg, 0.17 mmol) were added, and the reaction solution was stirred at 78 °C under nitrogen protection for 16 hours. The reaction solution was cooled to room temperature, 50 mL of water was added to the reaction solution, and extraction was performed with ethyl acetate (60 mLX2). The organic phases were combined, washed with saturated saline water (30 mLX2), dried with anhydrous sodium sulfate, filtered, spin-dried, and purified by column chromatography to obtain compound 17.1 (1.3 g, yield: 85%, yellow oily substance).

### Step B: methyl 4-(2-hydroxyethyl)-2-methylbenzoate (compound 17.2)

Compound 17.1 (1.3 g, 7.39 mmol) was dissolved in 10 mL of tetrahydrofuran, the reaction was cooled to 0 °C, and 2.2 mL of 10 M borane dimethyl sulfide solution was added dropwise. The reaction solution was stirred at 0 °C under nitrogen protection for 1 hour, and then at 20 °C for 1 hour. The reaction solution was cooled to 0 °C, and 10 mL of 2 M sodium hydroxide solution and 2.5 mL of 30% hydrogen peroxide were added dropwise successively. The reaction solution was stirred at 0 °C for 0.5 hours, and then at 20 °C for 1 hour. 100 mL of saturated sodium sulfite solution was added to the reaction solution, then the pH was adjusted to 2 with 2 M hydrochloric acid solution, and extraction was performed with ethyl acetate (100 mLX3). The organic phases were combined, dried with anhydrous sodium sulfate, filtered, spin-dried, and purified by column chromatography to obtain compound 17.2 (0.61 g, yield: 42%, colorless liquid).

### Step C: 4-(2-hydroxyethyl)-2-methylbenzoic acid (compound 17.3)

Compound 17.2 (610 mg, 3.14 mmol) was dissolved in 6 mL of methanol, and then 5 mL of 2M sodium hydroxide solution was added, and the reaction solution was stirred and refluxed for 4 hours. The reaction was monitored to be complete by LCMS. After the reaction was completed, water was added to quench the reaction, the pH was adjusted to 3 with 6N aqueous hydrochloric acid and stirring was performed for half an hour, then extraction was performed with ethyl acetate (30 mLX2). The organic phases were combined, washed with saturated saline water (20 mL), and the organic phases were dried with anhydrous sodium sulfate, filtered, spin-dried, and purified by column chromatography to obtain product 17.3 (509 mg, yield: 90%, white solid).

### Step D: 2-methyl-4-(2-(nitrooxy)ethyl)benzoic acid (compound 17.4)

Compound 17.3 (200 mg, 1.11 mmol) was dissolved in 5 mL of dichloromethane, and a mixed acid solution of concentrated nitric acid and concentrated sulfuric acid (1.4 mL of concentrated nitric acid, 0.35 mL of concentrated sulfuric acid) was added in an ice-water bath to react at room temperature for 5 hours. The reaction was monitored to be complete by TLC. After the reaction was completed, water was added to quench the reaction, then extraction was performed with ethyl acetate (15 mLX2), and the organic phases were combined and washed with saturated saline water (10 mLX2). The organic phases were dried with anhydrous sodium sulfate, filtered, spin-dried, and purified by column chromatography to obtain product 17.4 (192 mg, yield: 77%, white solid).

### Step E: (S)-4-(3-((tert-butoxycarbonyl)amino)-1-oxo-1-(thieno[2,3-c]pyridin-2-ylamino)prop-2-yl)benzyl 2-methyl-4-(2-(nitrooxy)ethyl)benzoate (compound 17.5)

Compound 15.2 (100 mg, 0.234 mmol) and compound 17.4 (58 mg, 0.258 mmol) were dissolved in 3 mL of N,N-dimethylformamide. Then, 1-ethyl-(3-dimethylaminopropyl)carbodiimide hydrochloride (68 mg, 0.351 mmol) and 4-dimethylaminopyridine (28 mg, 0.234 mmol) were added to react overnight at room temperature. The reaction was monitored to be complete by TLC. Water was added to quench the reaction, extraction was performed with ethyl acetate (20 mLX2), and the organic phases were combined and washed with saturated saline water (20 mLX2). The organic phases were dried with anhydrous sodium sulfate, filtered, spin-dried, and purified by column chromatography to obtain product 17.5 (115 mg, yield: 78%, light yellow solid).

### Step F: (S)-4-(3-amino-1-oxy-1-(thieno[2,3-c]pyridin-2-ylamino)prop-2-yl)benzyl 2-methyl-4-(2-(nitrooxy))ethyl)benzoate (compound 17)

Compound 17.5 (98 mg, 0.15 mmol) was dissolved in 1 mL of methanol, 3 mL of hydrochloric acid methanol solution (4 M) was added, and the reaction solution was stirred at room temperature for 2 hours. The reaction was monitored to be complete by TLC. After the reaction was completed, the reaction solution was concentrated under reduced pressure. The residue was dissolved in 5 mL of methanol and aqueous solution, filtered, and the filtrate was purified by reverse phase preparation. After lyophilization, target product compound 17 (72 mg, yield: 90%, purity: 98.8%) was obtained. LCMS ESI(+) m/z: 535.2(M+1). ¹H NMR (400 MHz, DMSO-*d*₆) δ 13.52 (s, 1H), 9.48 (s, 1H), 8.52 (d, J = 6.4 Hz, 1H), 8.24 (s, 3H), 8.15 (d, J = 6.4 Hz, 1H), 7.82 (d, J = 8.0 Hz, 1H), 7.51 (q, J = 8.4 Hz, 4H), 7.41 (s, 1H), 7.26 (s, 1H), 7.23 (d, J = 8.0 Hz, 1H), 5.30 (s, 2H), 4.76 (t, J = 6.4 Hz, 2H), 4.50 (dd, J = 8.4, 5.5 Hz, 1H), 3.65 (s, 1H), 3.18 (d, J = 6.4 Hz, 1H), 3.02 (t, J = 6.4 Hz, 2H), 2.50 (s, 3H).

### Example 18

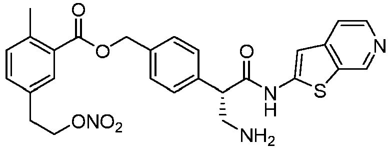

### (S)-4-(3-amino-1-oxy-1-(thieno[2,3-c]pyridin-2-ylamino)prop-2-yl)benzyl 2-methyl-5-(2-(nitrooxy))ethyl)benzoate

The specific reaction equation is as follows:

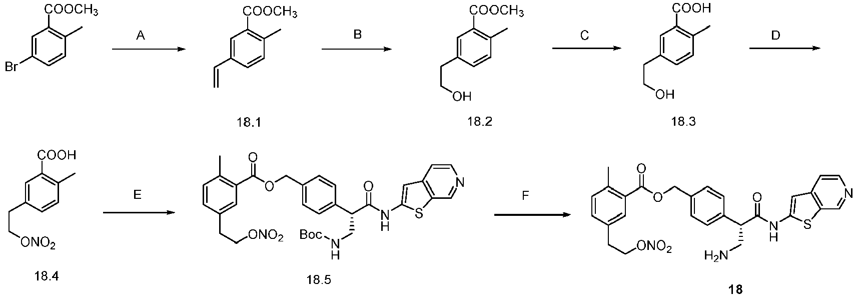

### Step A: methyl 2-methyl-5-vinylbenzoate (compound 18.1)

Methyl 5-bromo-2-methylbenzoate (2 g, 8.73 mmol) was dissolved in 40 mL of tetrahydrofuran and 4 mL of water, potassium vinyl trifluoroborate (1.4 g, 10.48 mmol), cesium carbonate (5.7 g, 17.46 mmol), and bis(triphenylphosphine) palladium chloride (122 mg, 0.17 mmol) were added, and the reaction solution was stirred at 78 °C under nitrogen protection for 16 hours. The reaction solution was cooled to room temperature, 50 mL of water was added to the reaction solution, and extraction was performed with ethyl acetate (60 mLX2). The organic phases were combined, washed with saturated saline water (30 mLX2), dried with anhydrous sodium sulfate, filtered, spin-dried, and purified by column chromatography to obtain compound 18.1 (1.13 g, yield: 74%, yellow oily substance).

### Step B: methyl 5-(2-hydroxyethyl)-2-methylbenzoate (compound 18.2)

Compound 18.1 (1.13 g, 6.42 mmol) was dissolved in 10 mL of tetrahydrofuran, the reaction was cooled to 0 °C, and 2.2 mL of 10 M borane dimethyl sulfide solution was added dropwise. The reaction solution was stirred at 0 °C under nitrogen protection for 1 hour, and then at 20 °C for 1 hour. The reaction solution was cooled to 0 °C, and 10 mL of 2 M sodium hydroxide solution and 2.5 mL of 30% hydrogen peroxide were added dropwise successively. The reaction solution was stirred at 0 °C for 0.5 hours, and then at 20 °C for 1 hour. 100 mL of saturated sodium sulfite solution was added to the reaction solution, then the pH was adjusted to 2 with 2 M hydrochloric acid solution, and extraction was performed with ethyl acetate (100 mLX3). The organic phases were combined, dried with anhydrous sodium sulfate, filtered, spin-dried, and purified by column chromatography to obtain compound 18.2 (0.62 g, yield: 50%, colorless liquid).

### Step C: 5-(2-hydroxyethyl)-2-methylbenzoic acid (compound 18.3)

Compound 18.2 (620 mg, 3.20 mmol) was dissolved in 6 mL of methanol, and then 5 mL of 2M sodium hydroxide solution was added, and the reaction solution was stirred and refluxed for 4 hours. The reaction was monitored to be complete by LCMS. After the reaction was completed, water was added to quench the reaction, the pH was adjusted to 3 with 6N aqueous hydrochloric acid and stirring was performed for half an hour, then extraction was performed with ethyl acetate (30 mLX2). The organic phases were combined, washed with saturated saline water (20 mL), and the organic phases were dried with anhydrous sodium sulfate, filtered, and spin-dried to obtain product 18.3 (570 mg, yield: 99%, white solid).

### Step D: 2-methyl-5-(2-(nitrooxy)ethyl)benzoic acid (compound 18.4)

Compound 18.3 (200 mg, 1.11 mmol) was dissolved in 5 mL of dichloromethane, and a mixed acid solution of concentrated nitric acid and concentrated sulfuric acid (1.4 mL of concentrated nitric acid, 0.35 mL of concentrated sulfuric acid) was added in an ice-water bath to react at room temperature for 5 hours. The reaction was monitored to be complete by TLC. After the reaction was completed, water was added to quench the reaction, then extraction was performed with ethyl acetate (15 mLX2), and the organic phases were combined and washed with saturated saline water (10 mLX2). The organic phases were dried with anhydrous sodium sulfate, filtered, spin-dried, and purified by column chromatography to obtain product 18.4 (191 mg, yield: 76%, light yellow solid).

### Step E: (S)-4-(3-((tert-butoxycarbonyl)amino)-1-oxo-1-(thieno[2,3-c]pyridin-2-ylamino)prop-2-yl)benzyl 2-methyl-5-(2-(nitrooxy)ethyl)benzoate (compound 18.5)

Compound 15.2 (100 mg, 0.234 mmol) and compound 18.4 (58 mg, 0.258 mmol) were dissolved in 3 mL of N,N-dimethylformamide. Then, 1-ethyl-(3-dimethylaminopropyl)carbodiimide hydrochloride (68 mg, 0.351 mmol) and 4-dimethylaminopyridine (28 mg, 0.234 mmol) were added to react overnight at room temperature. The reaction was monitored to be complete by TLC. Water was added to quench the reaction, extraction was performed with ethyl acetate (20 mLX2), and the organic phases were combined and washed with saturated saline water (20 mLX2). The organic phases were dried with anhydrous sodium sulfate, filtered, spin-dried, and purified by column chromatography to obtain product 18.5 (88 mg, yield: 59%, light yellow solid).

### Step F: (S)-4-(3-amino-1-oxy-1-(thieno[2,3-c]pyridin-2-ylamino)prop-2-yl)benzyl 2-methyl-5-(2-(nitrooxy))ethyl)benzoate (compound 18)

Compound 18.5 (88 mg, 0.14 mmol) was dissolved in 1 mL of methanol, 3 mL of hydrochloric acid methanol solution (4 M) was added, and the reaction solution was stirred at room temperature for 2 hours. The reaction was monitored to be complete by TLC. After the reaction was completed, the reaction solution was concentrated under reduced pressure. The residue was dissolved in 5 mL of methanol and aqueous solution, filtered, and the filtrate was purified by reverse phase preparation. After lyophilization, target product compound 18 (64 mg, yield: 86%, purity: 97%) was obtained. LCMS ESI(+) m/z: 535.2(M+1). ¹H NMR (400 MHz, DMSO-*d*₆) δ 13.54 (s, 1H), 9.48 (s, 1H), 8.52 (d, *J* = 6.4 Hz, 1H), 8.26 (s, 3H), 8.15 (d, *J* = 6.4 Hz, 1H), 7.76 (d, *J* = 1.6 Hz, 1H), 7.52 (q, *J* = 8.4 Hz, 4H), 7.46 - 7.35 (m, 2H), 7.28 (d, *J* = 7.6 Hz, 1H), 5.32 (s, 2H), 4.72 (t, *J* = 6.4 Hz, 2H), 4.51 (dd, *J* = 8.4, 5.4 Hz, 1H), 3.65 (s, 1H), 3.17 (d, *J =* 6.0 Hz, 1H), 3.01 (t, *J* = 6.4 Hz, 2H), 2.48 (s, 3H).

### Example 19

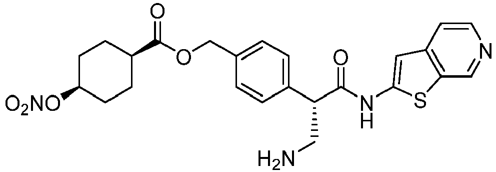

### 4-((S)-3-amino-1-oxy-1-(thieno[2,3-c]pyridin-2-ylamino)prop-2-yl)benzyl(1s,4R)-4-(nitrooxy)cyclohexane- 1 -carboxylate

The specific reaction equation is as follows:

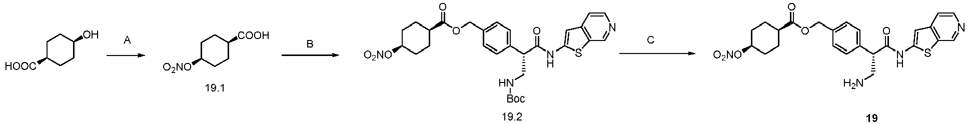

### Step A: (1s,4s)-4-(nitrooxy)cyclohexane-1-carboxylic acid (compound 19.1)

Compound (1s,4s) (200 mg, 1.39 mmol) was dissolved in 1.5 mL of acetic anhydride, a mixed acid solution of concentrated nitric acid and acetic anhydride (0.8 mL of nitric acid, 1.6 mL of acetic anhydride) was added under an ice-water bath, and stirring was performed at 0 °C for 4 hour. The reaction was monitored to be complete by TLC. After the reaction was completed, 30 mL of ethyl acetate was added to dilute, and the reaction solution was washed with water (10 mLX3) and saturated saline water (10 mLX2) successively. The organic phase was dried with anhydrous sodium sulfate, filtered, spin-dried, and purified by column chromatography to obtain product 19.1 (220 mg, yield: 90%, white solid).

### Step B: 4-((S)-3-((tert-butoxycarbonyl)amino)-1-oxo-1-(thieno[2,3-c]pyridin-2-ylamino)prop-2-yl)benzyl(1s,4R)-4-(nitrooxy)cyclohexane-1-carboxylic acid (compound 19.2)

Compound 15.2 (100 mg, 0.234 mmol) and compound 19.1 (50 mg, 0.257 mmol) were dissolved in 3 mL of N,N-dimethylformamide. Then, 1-ethyl-(3-dimethylaminopropyl)carbodiimide hydrochloride (68 mg, 0.351 mmol) and 4-dimethylaminopyridine (28 mg, 0.234 mmol) were added to react overnight at room temperature. The reaction was monitored to be complete by TLC. Water was added to quench the reaction, extraction was performed with ethyl acetate (20 mLX2), and the organic phases were combined and washed with saturated saline water (10 mLX2). The organic phases were dried with anhydrous sodium sulfate, filtered, spin-dried, and purified by column chromatography to obtain product 19.2 (72 mg, yield: 51%).

### Step C: 4-((S)-3-amino-1-oxy-1-(thieno[2,3-c]pyridin-2-ylamino)prop-2-yl)benzyl( 1s,4R)-4-(nitrooxy)cyclohexane-1 -carboxylate (compound 19)

Compound 19.2 (72 mg, 0.12 mmol) was dissolved in 3 mL of dichloromethane, 2 mL of trifluoroacetic acid was added, and the mixture was stirred at room temperature for 2 hours. The reaction was monitored to be complete by TLC. After the reaction was completed, saturated sodium bicarbonate (40 mL) was added to quench the reaction, and extraction was performed with dichloromethane (20 mLX3). The organic phases were combined, washed with saturated saline water (30 mL), and the organic phases were dried with anhydrous sodium sulfate, filtered, and spin-dried. The residue was dissolved in 10 mL of ethyl acetate, 0.2 mL of hydrogen chloride ethyl acetate solution (2 M) was added to stir for 1 hour, the solvent was spin-dried under vacuum, and the solid was slurried with a mixed solution of ethyl acetate and methanol and filtered. The filter cake was dissolved in ultrapure water, filtered, and purified by reverse phase preparation. After lyophilization, target product Example 19 (54 mg, yield: 90%, purity: 98%) was obtained. LCMS ESI(+) m/z: 499.2(M+1). ¹H NMR (400 MHz, DMSO-*d*₆) δ 13.50 (s, 1H), 9.47 (s, 1H), 8.52 (d, J = 6.4 Hz, 1H), 8.23 (s, 3H), 8.14 (d, J = 6.4 Hz, 1H), 7.50 (d, J = 8.4 Hz, 2H), 7.45 - 7.23 (m, 3H), 5.17 (s, 1H), 5.09 (s, 2H), 4.48 (dd, J = 8.4, 5.6 Hz, 1H), 3.64 (s, 1H), 3.64 (s, 1H), 3.24 - 3.04 (m, 1H), 3.21 - 3.02 (m, 1H), 2.58 - 2.52 (m, 1H), 1.90 - 1.81 (m, 2H), 1.81 - 1.71 (m, 4H), 1.69 - 1.57 (m, 2H).

### Example 20

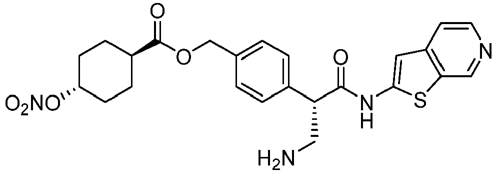

### 4-((S)-3-amino-1-oxy- 1-(thieno[2,3-c]pyridin-2-ylamino)prop-2-yl)benzyl(1r,4S)-4-(nitrooxy)cyclohexane-1-carboxylate

The specific reaction equation is as follows:

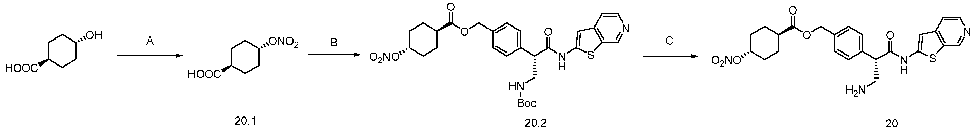

### Step A: (1r,4r)-4-(mtrooxy)cyclohexane-1-carboxylic acid (compound 20.1)

Compound (1r,4r) (200 mg, 1.39 mmol) was dissolved in 1.5 mL of acetic anhydride, a mixed acid solution of concentrated nitric acid and acetic anhydride (0.8 mL of nitric acid, 1.6 mL of acetic anhydride) was added under an ice-water bath, and stirring was performed at 0 °C for 4 hours. The reaction was monitored to be complete by TLC. After the reaction was completed, 30 mL of ethyl acetate was added to dilute, and the reaction solution was washed with water (10 mLX3) and saturated saline water (10 mLX2) successively. The organic phase was dried with anhydrous sodium sulfate, filtered, spin-dried, and purified by column chromatography to obtain product 20.1 (210 mg, yield: 80%, white solid).

### Step B: 4-((S)-3-((tert-butoxycarbonyl)amino)-1-oxo-1-(thieno[2,3-c]pyridin-2-ylamino)prop-2-yl)benzyl(1r,4S)-4-(nitrooxy)cyclohexane-1-carboxylic acid (compound 20.2)

Compound 15.2 (100 mg, 0.234 mmol) and compound 20.1 (50 mg, 0.257 mmol) were dissolved in 3 mL of N,N-dimethylformamide. Then, 1-ethyl-(3-dimethylaminopropyl)carbodiimide hydrochloride (68 mg, 0.351 mmol) and 4-dimethylaminopyridine (28 mg, 0.234 mmol) were added to react overnight at room temperature. The reaction was monitored to be complete by TLC. Water was added to quench the reaction, extraction was performed with ethyl acetate (30 mLX2), and the organic phases were combined and washed with saturated saline water (10 mLX2). The organic phases were dried with anhydrous sodium sulfate, filtered, spin-dried, and purified by column chromatography to obtain product 20.2 (126 mg, yield: 90%, light yellow solid).

### Step C: 4-((S)-3-amino-1-oxy-1-(thieno[2,3-c]pyridin-2-ylamino)prop-2-yl)benzyl(1r,4S)-4-(nitrooxy)cyclohexane-1-carboxylate (compound 20)

Compound 20.2 (126 mg, 0.21 mmol) was dissolved in 3 mL of dichloromethane, 2 mL of trifluoroacetic acid was added, and the mixture was stirred at room temperature for 2 hours. The reaction was monitored to be complete by TLC. After the reaction was completed, saturated sodium bicarbonate (40 mL) was added to quench the reaction, and extraction was performed with dichloromethane (20 mLX3). The organic phases were combined, washed with saturated saline water (30 mL), and the organic phases were dried with anhydrous sodium sulfate, filtered, and spin-dried. The residue was dissolved in 10 mL of ethyl acetate, 0.2 mL of hydrogen chloride ethyl acetate solution (2 M) was added to stir for 1 hour, the solvent was spin-dried under vacuum, and the solid was slurried with a mixed solution of ethyl acetate and methanol and filtered. The filter cake was dissolved in ultrapure water and filtered. After lyophilization, target product Example 20 (58 mg, yield: 55%, purity: 96.5%) was obtained. LCMS ESI(+) m/z: 499.2(M+1). ¹H NMR (400 MHz, DMSO-*d*₆) δ 13.06 (s, 1H), 9.28 (s, 1H), 8.44 (d, J = 6.0 Hz, 1H), 8.19 (s, 3H), 7.92 (d, J = 6.0 Hz, 1H), 7.48 (d, J = 8.4 Hz, 2H), 7.38 (d, J = 8.4 Hz, 2H), 7.26 (s, 1H), 5.08 (s, 2H), 5.05 - 4.92 (m, 1H), 4.41 (dd, J = 8.8, 5.6 Hz, 1H), 3.68 - 3.54 (m, 1H), 3.14 (d, J = 8.0 Hz, 1H), 2.43 (tt, J = 11.2, 3.6 Hz, 1H), 2.12 - 1.89 (m, 4H), 1.64 - 1.36 (m, 4H).

### Example 21

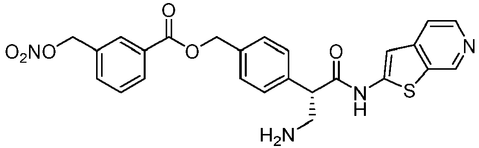

### (S)-4-(3-amino-1-oxo-1-(thieno[2,3-c]pyridin-2-ylamino)prop-2-yl)benzyl 3-((nitrooxy)methyl)benzoate

The specific reaction equation is as follows:

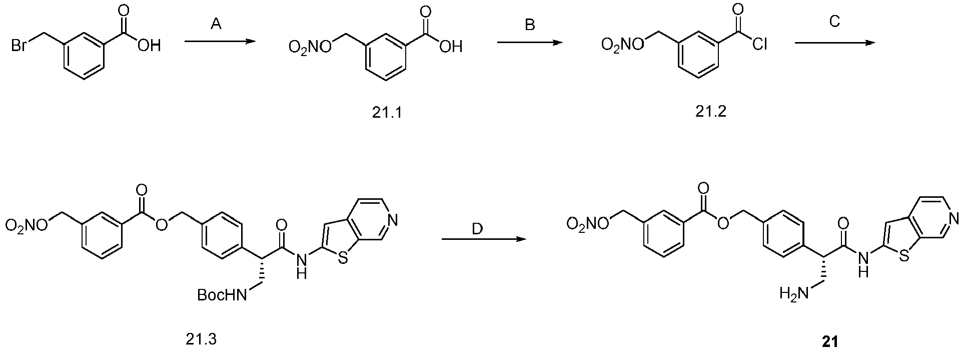

### Step A: 3-((nitrooxy)methyl)benzoic acid (compound 21.1)

3-bromomethylbenzoic acid (537 mg, 2.5 mmol) was dissolved in 10 mL of acetonitrile, silver nitrate (510 mg, 3.0 mmol) was added, and the reaction solution was stirred overnight in the dark at room temperature. After the reaction was monitored to be complete by TLC, the reaction solution was concentrated, dissolved in ethyl acetate (20 mL), filtered, and the filtrate was spin-dried to obtain compound 21.1 (495 mg, yield: 99%, white solid).

### Step B: 3-((nitrooxy)methyl)benzoyl chloride (compound 21.2)

Compound 21.1 (71 mg, 0.36 mmol) was dissolved in 4 mL of dichloromethane, oxalyl chloride (0.06 mL, 0.72 mmol) and 2 drops of DMF were added in an ice-water bath, and the reaction solution was stirred at room temperature for 1 hour. After the reaction was monitored to be complete by TLC, the reaction solution was concentrated to obtain the crude product of compound 21.2 used directly in the next reaction.

### Step C: (S)-4-(3-((tert-butoxycarbonyl)amino)-1-oxy-1-(thieno[2,3-c]pyridin-2-ylamino)prop-2-yl)benzyl 3-((nitrooxy)methyl)benzoate (compound 21.3)

Compound 21.2 (0.36 mmol), compound 15.2 (51 mg, 0.12 mmol), and 4-dimethylaminopyridine (1.5 mg, 0.01 mmol) obtained in the previous steps were dissolved in a mixed solvent of 5 mL of tetrahydrofuran and 3mL of dichloromethane, and triethylamine (0.05 mL, 0.36 mmol) was added under an ice-water bath. After the reaction was completed, the reaction solution was diluted with dichloromethane (10 mL), and washed with saturated sodium bicarbonate solution (10 mL), water (10 mL), and saturated saline water (10 mL) successively. The organic layer was dried with anhydrous sodium sulfate, filtered, spin-dried, purified by column chromatography and then separated and purified by thin-layer chromatography to obtain compound 21.3 (28.6 mg, yield: 39%, light yellow solid). LCMS ESI(+)m/z:607.2(M+1).

### Step D: (S)-4-(3-amino-1-oxy-1-(thieno[2,3-c]pyridin-2-ylamino)prop-2-yl)benzyl 3-((nitrooxy)methyl)benzoate (compound 21)

Compound 21.3 (28.6 mg, 0.047 mmol) was dissolved in 2 mL of methanol, and hydrogen chloride methanol solution (4 M, 1 mL) was added to react at room temperature for 2 hours. After the reaction was monitored to be complete by LCMS, the reaction solution was concentrated and purified by reverse preparation to obtain Example 21 (13.7 mg, yield: 64%, white solid). LCMS ESI(+)m/z:507.1(M+1).

### Example 22

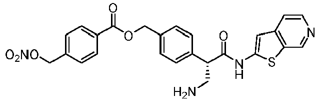

### (S)-4-(3-amino-1-oxo-1-(thieno[2,3-c]pyridin-2-ylamino)prop-2-yl)benzyl 4-((nitrooxy)methyl)benzoate

The specific reaction equation is as follows:

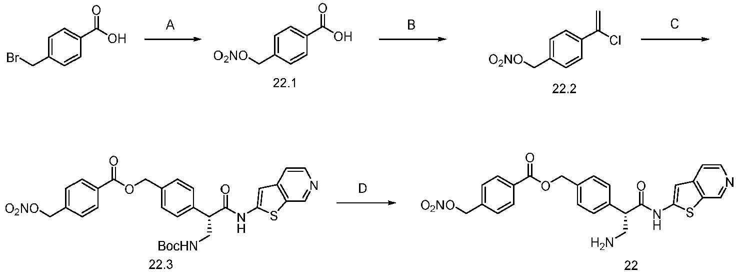

### Step A: 4-((nitrooxy)methyl)benzoic acid (compound 22.1)

4-bromomethylbenzoic acid (537 mg, 2.5 mmol) was dissolved in 10 mL of acetonitrile, silver nitrate (510 mg, 3.0 mmol) was added, and the reaction solution was stirred overnight in the dark at room temperature. The reaction solution was concentrated, dissolved in ethyl acetate (20 mL), filtered, and the filtrate was spin-dried to obtain compound 22.1 (476 mg, yield: 97%, white solid).

### Step B: 3-((nitrooxy)methyl)benzoyl chloride (compound 22.2)

Compound 22.1 (94.6 mg, 0.48 mmol) was dissolved in 5 mL of dichloromethane, oxalyl chloride (0.08 mL, 0.96 mmol) and 2 drops of DMF were added in an ice-water bath, and the reaction solution was stirred at room temperature for 1 hour. After the reaction was monitored to be complete by TLC, the reaction solution was concentrated to obtain the crude product of compound 22.2 used directly in the next reaction.

### Step C: (S)-4-(3-((tert-butoxycarbonyl)amino)-1-oxy-1-(thieno[2,3-c]pyridin-2-ylamino)prop-2-yl)benzyl 4-((nitrooxy)methyl)benzoate (compound 22.3)

Compound 22.2 (0.48 mmol), compound 15.2 (102.5 mg, 0.24 mmol), and 4-dimethylaminopyridine (3 mg, 0.024 mmol) obtained in the previous steps were dissolved in a mixed solvent of 8 mL of tetrahydrofuran and 3 mL of dichloromethane, and triethylamine (0.07 mL, 0.48 mmol) was added under an ice-water bath. After the reaction was completed, the reaction solution was diluted with dichloromethane (15 mL), and washed with saturated sodium bicarbonate solution (15 mL), water (15 mL), and saturated saline water (15 mL) respectively. The organic layer was dried with anhydrous sodium sulfate, filtered, spin-dried, purified by column chromatography and then separated and purified by thin-layer chromatography to obtain compound 22.3 (70 mg, yield: 48%, light yellow solid). LCMS ESI(+)m/z:607.2(M+1).

### Step D: (S)-4-(3-amino-1-oxy-1-(thieno[2,3-c]pyridin-2-ylamino)prop-2-yl)benzyl 4-((nitrooxy)methyl)benzoate (compound 22)

Compound 22.3 (70 mg, 0.115 mmol) was dissolved in hydrogen chloride methanol solution (4 M, 3 mL) to react at room temperature. After the reaction was monitored to be complete by LCMS, the reaction solution was concentrated and purified by reverse preparation to obtain product Example 22 (36.7 mg, yield: 59%, white solid). LCMS ESI(+)m/z:507.1(M+1).

### Example 23

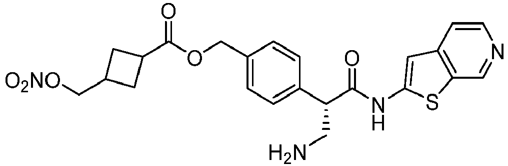

### (S)-4-(3-amino-1-oxy-1-(thieno[2,3-c]pyridin-2-ylamino)propan-2-yl)benzyl 3-((nitrooxy)methyl)cyclobutane-1-carboxylate

The specific reaction equation is as follows:

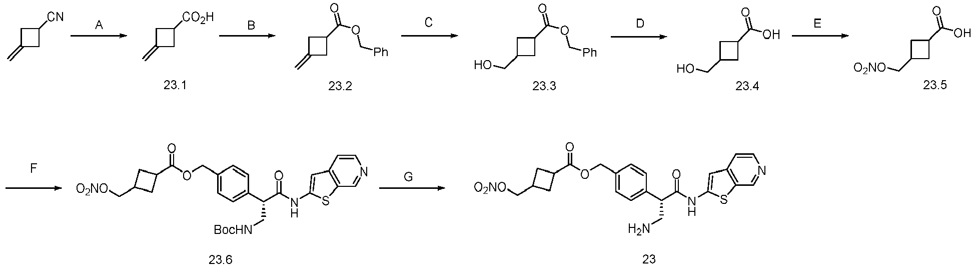

### Step A: 3-methylenecyclobutane-1-carboxylic acid (compound 23.1)

3-methylenecyclobutane-1-carbonitrile (3.1 g, 33.7 mmol) was dissolved in 20 mL of ethanol, and an aqueous solution (20 mL) of potassium hydroxide (7.6 g, 135 mmol) was added to react at 80 °C for 3 hours. After the reaction was monitored to be complete by LCMS, the organic solvent was span off, the aqueous phase was adjusted to pH<2 with concentrated hydrochloric acid, and extraction was performed with ethyl acetate (50 mL×2). The combined organic phases were washed with saturated saline water (50 mL), dried with anhydrous sodium sulfate, filtered, spin-dried, and purified by column chromatography to obtain product 23.1 (3.88 g, yield: 100%, colorless oily substance). LCMS ESI(+)m/z:113.1(M+1).

### Step B: benzyl 3-methylenecyclobutane-1-carboxylate (compound 23.2)

Compound 23.1 (1.8 g, 16 mmol) was dissolved in 20 mL of N,N-dimethylformamide, and potassium carbonate (3.32 g, 24 mmol) and benzyl bromide (2.09 mL, 17.6 mmol) were added to react at room temperature overnight. After the reaction was monitored to be complete by LCMS, the reaction solution was filtered, the filtrate was concentrated, diluted with water (50 mL), and extracted with ethyl acetate (50 mL×2). The combined organic phases were washed with saturated saline water (50 mL×5), dried with anhydrous sodium sulfate, filtered, spin-dried, and purified by column chromatography to obtain product 23.2 (2.85 g, yield: 88%, colorless oily substance). LCMS ESI(+)m/z:203.2(M+1).

### Step C: benzyl 3-(hydroxymethyl)cyclobutane-1-carboxylate (compound 23.3)

Under nitrogen protection, compound 23.2 (1.41 g, 7 mmol) was dissolved in 10 mL of tetrahydrofuran, and 2.1 mL of borane dimethyl sulfide solution (10 M) was added dropwise under an ice-salt bath. After the dropwise addition was completed, the mixture was reacted at room temperature for 1 hour. After the reaction was monitored to be complete by TLC, 7 mL of sodium hydroxide aqueous solution (3 M) and 2.4 mL of hydrogen peroxide solution (30%) were successively added in the ice-salt bath, and the reaction was continued for 2 hours at room temperature. After the reaction was monitored to be complete by TLC, the reaction was quenched with saturated sodium sulfite solution (5 mL), diluted with water (20 mL), then extracted with ethyl acetate (50 mL×2). The organic phases were combined, washed with saturated saline water (50 mL), dried with anhydrous sodium sulfate, filtered, spin-dried, and purified by column chromatography to obtain product 23.3 (749 mg, yield: 49%, colorless oily substance). LCMS ESI(+)m/z:221.2(M+1).

### Step D: 3-(hydroxymethyl)cyclobutane-1-carboxylic acid (compound 23.4)

Compound 23.3 (716 mg, 3.25 mmol) was dissolved in 10 mL of methanol, and an aqueous solution (5 mL) of sodium hydroxide (390 mg, 9.7 mmol) was added to react by stirring at room temperature. After the reaction was monitored to be complete by LCMS, the reaction solution was diluted with water (20 mL) and extracted with ethyl acetate (25 mL×3). The organic phases were combined, washed with saturated saline water (50 mL), dried with anhydrous sodium sulfate, filtered, and spin-dried to obtain compound 23.4 (236 mg, yield: 56%, colorless liquid).

### Step E: 3-((nitrooxy)methyl)cyclobutane-1-carboxylic acid (compound 23.5)

In an ice-water bath, 1 mL of fuming nitric acid was added dropwise to 2 mL of acetic anhydride and stirring was performed for 5 minutes. Compound 23.4 (200 mg, 1.54 mmol) was dissolved in 5 mL of acetic anhydride, and the nitric acid acetic anhydride solution was added dropwise under an ice-water bath to react at constant temperature for 1 hour. After the reaction was monitored to be complete by TLC, the reaction solution was diluted with water (20 mL) and extracted with ethyl acetate (20 mL×2). The organic phases were combined, washed with saturated saline water (20 mL), dried with anhydrous sodium sulfate, filtered, spin-dried, and purified by column chromatography to obtain product 23.5 (155.6 mg, yield: 58%, light brown liquid).

### Step F: (S)-4-(3-((tert-butoxycarbonyl)amino)-1-oxy-1-(thieno[2,3-c]pyridin-2-ylamino)prop-2-yl)benzyl 3-((nitrooxy)methyl)cyclobutane-1-carboxylate (compound 23.6)

Compound 23.5 (75 mg, 0.43 mmol), compound 15.2 (100 mg, 0.23 mmol), and 4-dimethylaminopyridine (41.5 mg, 0.34 mmol) were dissolved in N,N-dimethylformamide (5 mL), and 1-(3-dimethylaminopropyl)-3-ethylcarbodiimide hydrochloride (88 mg, 0.46 mmol) was added to react at room temperature overnight. After the reaction was monitored to be complete by TLC, the reaction solution was diluted with water (20 mL) and extracted with ethyl acetate (20 mL×2). The organic phases were combined, washed with saturated saline water (20 mL), dried with anhydrous sodium sulfate, filtered, spin-dried, and purified by column chromatography to obtain product 23.6 (158.1 mg, yield: 81%, light yellow solid). LCMS ESI(+)m/z:585.2(M+1).

### Step G: (S)-4-(3-amino-1-oxy-1-(thieno[2,3-c]pyridin-2-ylamino)prop-2-yl)benzyl 3-((nitrooxy)methyl)cyclobutane-1-carboxylate (compound 23)

After compound 23.6 (75 mg, 0.128 mmol) was dissolved in 3 mL of dichloromethane, 0.3 mL of trifluoroacetic acid was added to react at room temperature for 1 hour. After the reaction was monitored to be complete by LCMS, the reaction solution was concentrated, 20 mL of ethyl acetate was added, and washing was performed with saturated sodium bicarbonate solution (20 mL) and saturated saline water (20 mL) successively. The organic phase was dried with anhydrous sodium sulfate, filtered, spin-dried, and purified by reverse preparation to obtain product Example 23 (47.8 mg, yield: 72%, white solid). LCMS ESI(+)m/z:485.2 (M+1). 1H NMR (400 MHz, DMSO) δ13.56 (s, 1 H), 9.48 (s, 1 H), 8.52 (d, J = 6.4 Hz, 1 H), 8.26 ( s, 3 H), 8.15 (d, J = 6.4 Hz, 1 H), 7.51 (d, J = 8.0 Hz, 2 H), 7.43 - 7.35 (m, 3 H), 5.08 (d, J = 7.2 Hz, 2 H), 4.58 (d, J = 7.2 Hz, 1 H), 4.52 - 4.44 (m, 2 H), 3.72 - 3.57 (m, 1 H), 3.31 - 3.21 (m, 1 H), 3.20 - 3.08 (m, 1 H), 2.69 - 2.56 (m, 1 H), 2.36 - 2.21 (m, 2 H), 2.14 - 1.94 (m, 2 H).

### Example 24

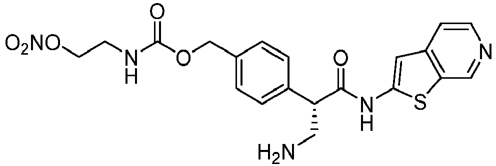

### (S)-4-(3-amino-1-oxy-1-(thieno[2,3-c]pyridin-2-ylamino)prop-2-yl)benzyl(2-(nitrooxy)ethyl)carbamate

The specific reaction equation is as follows:

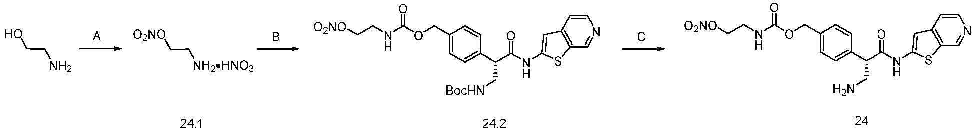

### Step A: 2-aminoethyl nitrate nitrate (24.1)

In an ice-water bath, 3 mL of fuming nitric acid was added dropwise to 12 mL of acetic anhydride and stirring was performed for 30 minutes. Then, ethanolamine (977.3 mg, 16 mmol) was added in the ice-water bath, and the temperature was kept below 5 °C and stirring was performed for 30 minutes. The reaction solution was concentrated, 50 mL of ether was added to slurry for 1 hour, and product 24.1 (1.95 g, yield: 72%, white solid) was obtained by filtering.

### Step B: (S)-tert-butyl(2-(4-((((((2-(nitrooxy)ethyl)carbamoyl)oxy)methyl)phenyl)-3-oxo-3-(thio[2,3-c]pyridin-2-ylamino)propyl)carbamate (24.2)

Under argon protection, compound 15.2 (64 mg, 0.15 mmol) and triphosgene (22.2 mg, 0.075 mmol) were dissolved in 3 mL of tetrahydrofuran, and a tetrahydrofuran (1 mL) solution of diisopropylethylamine (19.4 mg, 0.15 mmol) and 4-dimethylaminopyridine (3.7 mg, 0.03 mmol) was added under an ice-water bath to react at constant temperature for 1 hour. After the reaction was monitored to be complete by TLC, a tetrahydrofuran (1 mL) solution of compound 24.1 (50.7 mg, 0.3 mmol) and diisopropylethylamine (38.8 mg, 0.3 mmol) was added to react at room temperature for 1 hour. After the reaction was monitored to be complete by TLC, the reaction solution was diluted with ethyl acetate (20 mL) and washed with saturated ammonium chloride solution (20 mL) and saturated saline water (20 mL) successively. Filtering, spin-drying, and purification by column chromatography were performed to obtain product 24.2 (24.8 mg, yield: 30%, light yellow solid). LCMS ESI(+)m/z:560.2(M+1).

### Step C: (S)-4-(3-amino-1-oxy-1-(thieno[2,3-c]pyridin-2-ylamino)prop-2-yl)benzyl (2-(nitrooxy)ethyl)carbamate (compound 24)

Compound 24.2 (24.8 mg, 0.044 mmol) was dissolved in 1.5 mL of methanol, and hydrogen chloride methanol solution (4 M, 1.5 mL) was added to react at room temperature. After the reaction was completed, the reaction solution was concentrated and purified by reverse preparation to obtain product Example 24 (11.6 mg, yield: 53%, white solid). LCMS ESI(+)m/z:460.1 (M+1). 1H NMR (400 MHz, DMSO) δ13.50 ( s, 1 H), 9.48 (s, 1 H), 8.52 (d, J = 6.4 Hz, 1 H), 8.24 (s, 3 H), 8.15 (d, J = 6.4 Hz, 1 H), 7.55 - 7.45 (m, 3 H), 7.43 - 7.32 (m, 3 H), 5.02 (s, 2 H), 4.59 - 4.40 (m, 3 H), 3.72 - 3.57 (m, 1 H), 3.36 - 3.31 (m, 2 H), 3.20 - 3.10 (m, 1 H).

### Example 25

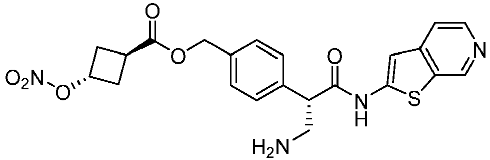

### 4-((S)-3-amino-1-oxo-1-(thieno[2,3-c]pyridin-2-ylamino)propan-2-yl)benzyl (1r,3S)-3-(nitrooxy)cyclobutane-1- carboxylate

The specific reaction equation is as follows:

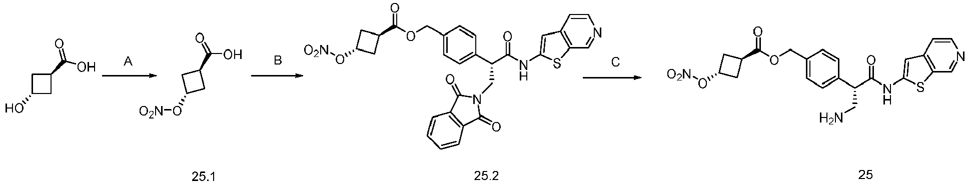

### Step A: (1R,3R)-3-(nitrooxy)cyclobutanecarboxylic acid (compound 25.1)

Under ice bath conditions, weighed acetic anhydride (3 mL) was added to a single-neck reaction flask (50 mL), and then concentrated nitric acid (1.5 mL) was slowly added dropwise to the reaction flask, and the reaction solution was stirred under the ice bath for 10 minutes. Under ice bath conditions, (1R,3R)-3-hydroxycyclobutane carboxylic acid (150 mg, 1.29 mmol) was dissolved in acetic anhydride (4 mL), then an acetic anhydride-concentrated nitric acid mixture (4.5 mL) was slowly added dropwise to the reaction solution, and then the reaction solution was stirred at room temperature for 1 hour. The reaction was confirmed to be complete by TLC (bromocresol green color development). Water was added to the reaction solution, extraction was performed with ethyl acetate (15 mL), the organic phases were separated and combined, and then the organic phases were washed with saturated saline water. The organic phases were dried with anhydrous sodium sulfate, filtered, spin-dried, and purified by silica column to obtain white solid 25.1 (120 mg, yield: 58%).

### Step B: 4-((S)-3-(1,3-dioxoisoindolin-2-yl)-1-oxo-1-(thieno[2,3-c]pyridin-2-ylamino)propan-2-yl)benzyl (1r,3S)-3-(nitrooxy)cyclobutane-1- carboxylate (compound 25.2)

Compound 1.11 (100 mg, 0.219 mmol) was dissolved in N,N-dimethylformamide (6 mL), and then 1-(3-dimethylaminopropyl)-3-ethylcarbodiimide hydrochloride (63.3 mg, 0.33 mmol), 4-dimethylaminopyridine (40.26 mg, 0.33 mmol), and compound 25.1 (42.34 mg, 0.26 mmol) were added successively. The mixture was stirred overnight at room temperature. The reaction was detected to be complete by LCMS. Water was added to the reaction solution, extraction was performed with ethyl acetate (15 mLX2), the organic phases were combined, and the organic phases were washed with saturated saline water and dried with anhydrous sodium sulfate. Filtering and spin-drying were performed, and the residue was purified by silica column to obtain light yellow solid 25.2 (105 mg, yield: 80%). LCMS ESI(+) m/z:601.1(M+1).

### Step C: 4-((S)-3-amino-1-oxo-1-(thieno[2,3-c]pyridin-2-ylamino)propan-2-yl)benzyl (1r,3S)-3-(nitrooxy)cyclobutane-1-carboxylate (compound 25)

Compound 25.2 (105 mg, 0.175 mmol) was dissolved in ethanol (8 mL), then hydrazine hydrate (87.5 mg, 1.75 mmol) was added to the reaction solution, and the reaction was stirred at 60 °C for 2 hours under an inert gas atmosphere. The reaction was detected to be complete by LCMS. Water was added to the reaction solution, and extraction was performed with ethyl acetate (12 mLX2). The organic phase was washed with saturated saline water, and dried with anhydrous sodium sulfate. Filtering, concentrate, and spin-drying were performed. The residue was dissolved in 3 mL of methanol and purified by reverse preparation. After lyophilization, light yellow solid Example 25 was obtained (25 mg, yield: 30%, purity: 96%). LCMS ESI(+) m/z:471.1(M+1). ¹H NMR (400 MHz, DMSO) δ 13.40 (s, 1H), 9.47 (s, 1H), 8.52 (d, J = 6.5 Hz, 1H), 8.22 - 8.12 (m, 4H), 7.49 (d, J = 8.2 Hz, 2H), 7.43 - 7.37 (m, 3H), 5.45 - 5.35 (m, 1H), 5.12 (s, 2H), 4.48 - 4.42 (m, 1H), 3.64 (s, 1H), 3.25 (ddd, J = 14.4, 9.7, 4.6 Hz, 2H), 3.21 - 3.11 (m, 1H), 2.62 (ddd, J = 12.1, 7.3, 4.9 Hz, 2H), 2.48 - 2.44 (m, 1H).

### Example 26

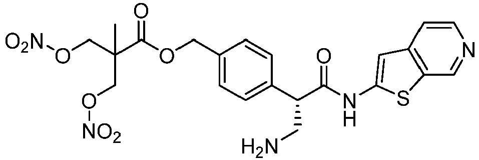

### (S)-4-(3-amino-1-oxo-1-(thieno[2,3-c]pyridin-2-ylamino)prop-2-yl)benzyl 2-methyl-3-(nitrooxy)-2-((nitrooxy)methyl)propionate

The specific reaction equation is as follows:

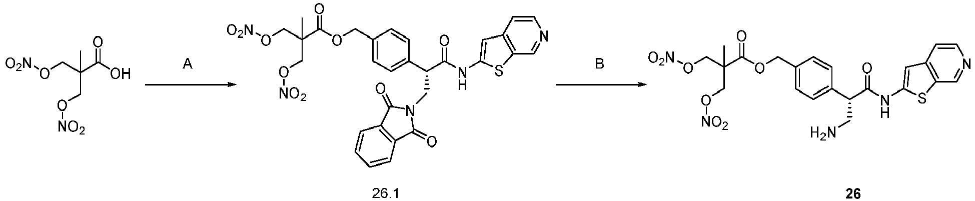

### Step A: (S)-4-(3-(1,3-dioxyisoindol-2-yl)-1-oxy-1-(thieno[2,3-c]pyridin-2-ylamino)prop-2-yl)benzyl 2-3-(nitrooxy)methyl-2-((nitrooxy)methyl)propionate (compound 26.1)

Compound 1.11 (50 mg, 0.11 mmol) was dissolved in N,N-dimethylformamide (6 mL), and then O-(7-azabenzotriazol-1-yl)-N,N,N',N'-tetramethylurea (62.7 mg, 0.165 mmol) and N,N-diisopropylethylamine (35.5 mg, 0.275 mmol) were added. The reaction solution was stirred at room temperature for 30 minutes, and then 2-methyl-3-(nitrooxy)-2-((nitrooxy)methyl)propionic acid (29.57 mg, 0.132 mmol) was added. The mixture was stirred overnight at room temperature. The reaction was confirmed to be complete by LCMS. Water was added to the reaction solution, extraction was performed with ethyl acetate (15 mL), the organic phases were separated and combined, and the organic phases were washed with saturated saline water and dried with anhydrous sodium sulfate. Filtering, spin-drying, and purification by silica column were performed to obtain light yellow solid 26.1 (40 mg, yield: 55%). LCMS ESI(+) m/z:664.1(M+1).

### Step B: (S)-4-(3-amino-1-oxo-1-(thieno[2,3-c]pyridin-2-ylamino)prop-2-yl)benzyl 2-methyl-3-(nitrooxy)-2-((nitrooxy)methyl)propionate (compound 26)

Compound 26.1 (40 mg, 0.06 mmol) was dissolved in 7 mL of ethanol, and then hydrazine hydrate (30 mg, 0.6 mmol) was added to the reaction solution. The reaction was stirred at 60 °C for 2 hours under an inert gas atmosphere. The reaction was confirmed to be complete by LCMS. Water was added to the reaction solution, extraction was performed with ethyl acetate (12 mL), and washing was performed with saturated saline water. The organic phase was dried with anhydrous sodium sulfate, filtered, concentrated, and spin-dried. The residue was dissolved in methanol (3 mL). After reverse preparation and purification, white solid product Example 26 (10.5 mg (hydrochloride), yield: 30.5%, purity: 98%) was obtained. LCMS ESI(+) m/z:534.1(M+1). ¹H NMR (400 MHz, DMSO) δ 13.35 (s, 1H), 9.46 (s, 1H), 8.52 (d, J =6.4 Hz, 1H), 8.22 - 8.11 (m, 4H), 7.48 (d, J = 8.4 Hz, 2H), 7.42 - 7.36 (m, 3H), 5.17 (s, 2H), 4.78 - 4.69 (m, 4H), 4.44 (dd, J = 8.8, 5.0 Hz, 1H), 3.64 (s, 1H), 3.20 - 3.11 (m, 1H), 1.29 (s, 3H).

### Example 27

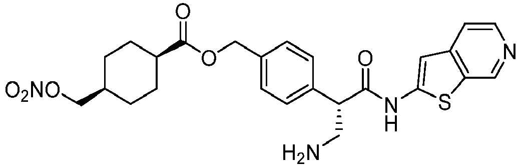

### 4-((S)-3-amino-1-oxy-1-(thieno[2,3-c]pyridin-2-ylamino)prop-2-yl)benzyl(1s,4R)-4-((nitrooxy)methyl)cyclohexane-1-carboxylic acid

The specific reaction equation is as follows:

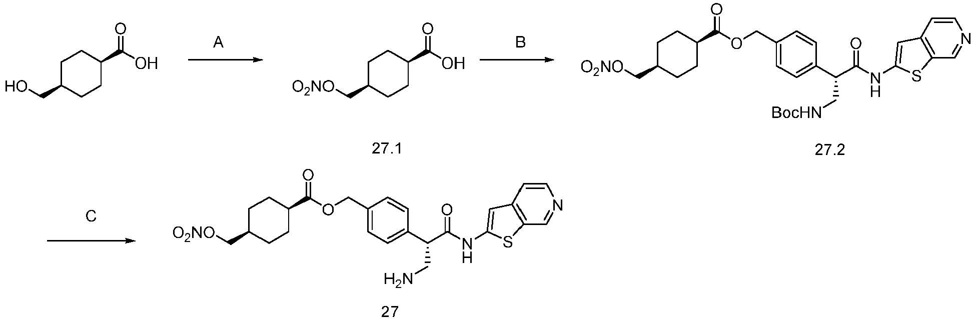

### Step A: trans-4-((nitrooxy)methyl)cyclohexane-1-carboxylic acid (27.1)

In an ice-water bath, 2 mL of concentrated nitric acid was added dropwise to 2 mL of concentrated sulfuric acid, and stirring was performed for 100 minutes. Next, a dichloromethane (4 mL) solution of trans-4-(hydroxymethyl)cyclohexane-1-carboxylic acid (60 mg, 0.38 mmol) was added under the ice-water bath, and the temperature was kept below 5 °C and the mixture was stirred for 50 minutes. After the reaction was monitored to be complete by TLC, the reaction solution was diluted with water (20 mL) in the ice-water bath and extracted with dichloromethane (20 mL×2). The organic phases were combined, dried with anhydrous sodium sulfate, filtered, spin-dried, and purified by column chromatography to obtain product 27.1 (63 mg, yield: 81%, white solid).

### Step B: trans-(S)-4-(3-((tert-butoxycarbonyl)amino)-1-oxo-1-(thieno[2,3-c]pyridin-2-ylamino)propane-2-yl)benzyl 4-((nitrooxy)methyl)cyclohexane-1-carboxylic acid (27.2)

Compound 27.1 (43.25 mg, 0.21 mmol), 1-(3-dimethylaminopropyl)-3-ethylcarbodiimide hydrochloride (54.8 mg, 0.28 mmol), and 4-dimethylaminopyridine (33.5 mg, 0.28 mmol) were dissolved in N,N-dimethylformamide (3 mL), and compound 15.2 (60.8 mg, 0.14 mmol) was added to react at room temperature overnight. After the reaction was monitored to be complete by LCMS, the reaction solution was diluted with water (15 mL) and extracted with ethyl acetate (15 mL×2). The organic phases were combined, washed with saturated saline water (20 mL), dried with anhydrous sodium sulfate, filtered, spin-dried, and purified by column chromatography to obtain product 27.2 (62.7 mg, yield: 72%, light yellow solid). LCMS ESI(+)m/z:613.1(M+1).

### Step C: trans-(S)-4-(3-amino-1-oxy-1-(thieno[2,3-c]pyridin-2-ylamino)prop-2-yl)benzy14-((nitrooxy)methyl)cyclohexane-1-carboxylate (compound 27)

Compound 27.2 (57 mg, 0.093 mmol) was dissolved in 1,4-dioxane solution (4 M) of 5 mL of hydrogen chloride to react at room temperature for 1 hour. After the reaction was monitored to be complete by LCMS, the reaction solution was concentrated to dryness and purified by reverse preparation to obtain product Example 27 (30.9 mg, yield: 60%, light yellow solid). LCMS ESI(+)m/z:513.1(M+1).

### Example 28

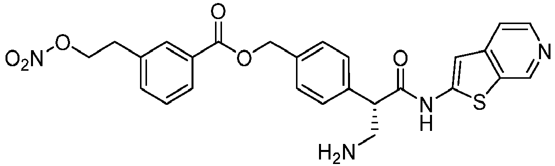

### (S)-4-(3-amino-1-oxy-1-(thieno[2,3-c]pyridin-2-ylamino)prop-2-yl)benzyl 3-(2-(nitrooxy)ethyl)benzoate

The specific reaction equation is as follows:

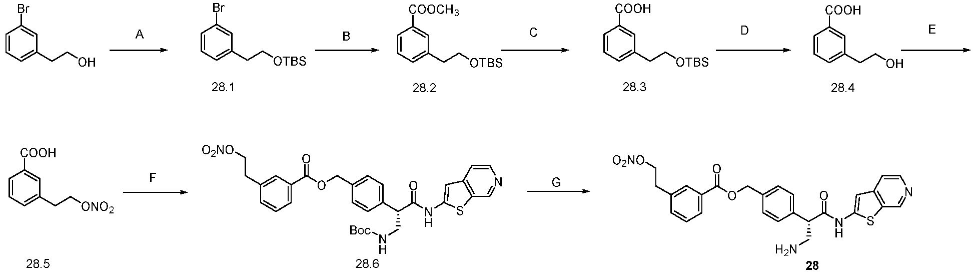

### Step A: (3-bromophenethoxy)(tert-butyl)dimethylsilane (compound 28.1)

3-bromophenethyl alcohol (1.89 g, 9.4 mmol) and imidazole (2.56 g, 36.16 mmol) were dissolved in 50 mL of dichloromethane, tert-butyldimethylchlorosilane (2.83 g, 18.78 mmol) was added at room temperature, and then the system was stirred at room temperature for 2.5 hours. The reaction was monitored to be complete by TLC. After the reaction was completed, washing was performed with water (30 mLX2), saturated sodium bicarbonate solution (30 mLX2), and saturated saline water (30 mLX2) successively. The organic phase was dried with anhydrous sodium sulfate, filtered, spin-dried, and purified by column chromatography to obtain product 28.1 (2.64 g, yield: 89%).

### Step B: methyl 3-(2-(((tert-butyldimethylsilyl)oxy)ethyl)benzoate (compound 28.2)

Compound 28.1 (2.64 g, 8.37 mmol) and [1,1'-bis(diphenylphosphino)ferrocene]palladium dichloride (612 mg, 0.0837 mmol) were dissolved in 15 mL of N,N-dimethylformamide and 45 mL of methanol mixed solvent, and then 15 mL of triethylamine was added. The system was reacted overnight at 80 °C under a carbon monoxide atmosphere. The reaction was monitored to be complete by LCMS. After the reaction was completed, water was added to quench the reaction, then after concentration, extraction was performed with ethyl acetate (30 mLX3), and the organic phases were combined and washed with saturated saline water (40 mLX2). The organic phases were dried with anhydrous sodium sulfate, filtered, spin-dried, and purified by column chromatography to obtain product 28.2 (2 g, yield: 81%).

### Step C: 3-(2-(((tert-butyldimethylsilyl)oxy)ethyl)benzoic acid (compound 28.3)

Compound 28.2 (2 g, 6.79 mmol) was dissolved in 20 mL of methanol, then 20 mL of tetrahydrofuran and 15 mL of water were added, then lithium hydroxide monohydrate (859 mg, 20.37 mmol) was added, and the reaction was performed overnight at room temperature. The reaction was monitored to be complete by LCMS. After the reaction was completed, the pH was adjusted to 3 with 1 M aqueous hydrochloric acid and stirring was performed for half an hour, then extraction was performed with ethyl acetate (60 mLX2). The organic phases were combined, washed with saturated saline water (30 mLX1), and the organic phases were dried with anhydrous sodium sulfate, filtered, spin-dried, and purified by column chromatography to obtain product 28.3 (1.2 g, yield: 63%).

### Step D: 3-(2-hydroxyethyl)benzoic acid (compound 28.4)

Compound 28.3 (700 mg, 2.5 mmol) was dissolved in 10 mL of ethyl acetate, then 3 mL of hydrogen chloride/methanol (4 M) solution was added. The reaction was stirred at room temperature for 1.5 hours. After the reaction was completed, the reaction was concentrated and the residue was subjected to column chromatography to obtain product 28.4 (258 mg, yield: 62%)

### Step E: 3-(2-(nitrooxy)ethyl)benzoic acid (compound 28.5)

A mixed acid solution of concentrated nitric acid and concentrated sulfuric acid (20 drops of concentrated nitric acid, 4 drops of concentrated sulfuric acid) was added to 30 mL of dichloromethane under an ice-water bath. After stirring for 1 hour in the ice bath, compound 28.4 (250 mg, 1.5 mmol) was added to react at room temperature for 4 hours. The reaction was monitored to be complete by TLC. After the reaction was completed, water was added to quench the reaction, then extraction was performed with dichloromethane (40 mLX2), and the organic phases were combined and washed with saturated saline water (30 mLX3). The organic phases were dried with anhydrous sodium sulfate, filtered, spin-dried, and purified by column chromatography to obtain product 28.5 (235 mg, yield: 74%).

### Step F: (S)-4-(3-((tert-butoxycarbonyl)amino)-1-oxy-1-(thieno[2,3-c]pyridin-2-ylamino)prop-2-yl)benzyl 3-(2-(nitrooxy)ethyl)benzoate (compound 28.6)

Compound 15.2 (161 mg, 0.378 mmol) and compound 28.5 (114 mg, 0.539 mmol) were dissolved in 3 mL of N,N-dimethylformamide. Then, 1-ethyl-(3-dimethylaminopropyl)carbodiimide hydrochloride (155 mg, 0.808 mmol) and 4-dimethylaminopyridine (98.6 mg, 0.808 mmol) were added to react overnight at room temperature. The reaction was monitored to be complete by TLC. Water was added to quench the reaction, extraction was performed with ethyl acetate (20 mLX2), and the organic phases were combined and washed with saturated saline water (20 mLX2). The organic phases were dried with anhydrous sodium sulfate, filtered, spin-dried, and purified by column chromatography to obtain product 28.6 (185 mg, yield: 79%).

### Step G: (S)-4-(3-amino-1-oxy-1-(thieno[2,3-c]pyridin-2-ylamino)prop-2-yl)benzyl 3-(2-(nitrooxy)ethyl)benzoate (compound 28)

Compound 28.6 (100 mg,0.16 mmol) was dissolved in 6 mL of methanol, 4 mL of hydrochloric acid methanol solution (4 M) was added, and the reaction solution was stirred at room temperature for 3 hours. The reaction was monitored to be complete by TLC. After the reaction was completed, the reaction solution was concentrated under reduced pressure. The residue was dissolved in 5 mL of methanol and aqueous solution, filtered, and the filtrate was purified by reverse phase preparation. After lyophilization, target product compound Example 28 (76 mg, yield: 91%, purity: 99%) was obtained. LCMS ESI(+) m/z: 521.2(M+1). ¹H NMR (400 MHz, DMSO) δ13.55 (s, 1 H), 9.48 (s, 1 H), 8.52 (d, J = 6.4 Hz, 1 H), 8.25 (s, 3 H), 8.15 (d, J = 6.4 Hz, 1 H), 7.94 - 7.81 (m, 2 H), 7.59 (d, J = 7.6 Hz, 1 H), 7.56 - 7.45 (m, 5 H), 7.41 (s, 1 H), 5.35 (s, 2 H), 4.76 (t, J = 6.4 Hz, 2 H), 4.51 (dd, J = 5.6, 8.4 Hz, 1 H), 3.63-3.66 (m, 1 H), 3.22 - 3.12 (m, 1 H), 3.08 (t, J = 6.4 Hz, 2 H).

### Example 29

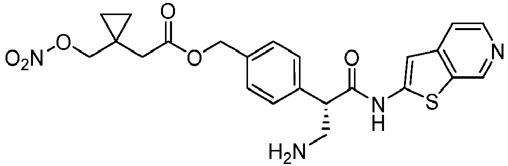

### (S)-4-(3-amino-1-oxy-1-(thieno[2,3-c]pyridin-2-ylamino)prop-2-yl)benzyl 2-(1-((nitrooxy)methyl)cyclopropyl acetate

The specific reaction equation is as follows:

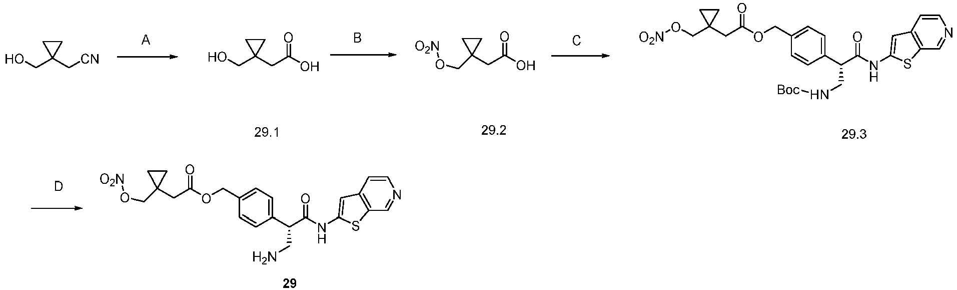

### Step A: 2-(1-(hydroxymethyl)cyclopropyl)acetic acid (compound 29.1)

Compound 2-(1-(hydroxymethyl)cyclopropyl)acetonitrile (1 g, 9 mmol) was dissolved in 15 mL of ethanol, and then an aqueous (20 mL) solution of potassium hydroxide (5 g, 89.12 mmol) was added. Stirring was performed at 80 °C to react for 16 hours. After the reaction was monitored to be complete by TLC, ethanol was removed by concentration, then the temperature was cooled to 0 °C, and the pH was adjusted to 1 to 2 with concentrated hydrochloric acid. Then, extraction was performed with ethyl acetate (80 mLX2), and the combined organic phases were washed with saturated saline water, dried with anhydrous sodium sulfate, and concentrated to obtain compound 29.1 (870 mg, yield: 74%)

### Step B: 2-(1-(((nitrooxy)methyl)cyclopropyl)acetic acid (compound 29.2)

At 0 °C, 1 mL of concentrated nitric acid was added to 2 mL of acetic anhydride, and then the mixture was stirred at 0 °C for 10 minutes. Then, the mixture was added dropwise to an acetic anhydride (15 mL) solution of compound 29.1 (200 mg, 1.54 mmol) at 0 °C, and the temperature was kept at 0 °C and stirring was performed for 1.5 hours. After the reaction was monitored to be complete by TLC, water (20 mL) was added and extraction was performed with dichloromethane (40mLX2). The combined organic phases were respectively washed with water and saturated saline water, dried with anhydrous sodium sulfate, concentrated, and subjected to column chromatography to obtain compound 29.2 (56 mg, yield: 21%).

### Step C: (S)-4-(3-((tert-butoxycarbonyl)amino)-1-oxy-1-(thieno[2,3-c]pyridin-2-ylamino)prop-2-yl)benzyl 2-(1-((nitrooxy)methyl)cyclopropyl)acetate (compound 29.3)

Compound 29.2 (56 mg, 0.319 mmol) was dissolved in N,N-dimethylformamide (10 mL). 1-ethyl-(3-dimethylaminopropyl)carbodiimide hydrochloride (92 mg, 0.479 mmol), 4-dimethylaminopyridine (59 mg, 0.483 mmol), and compound 15.2 (68 mg, 0.159 mmol) were added. Stirring was performed at room temperature overnight under N2 protection. After the reaction was monitored to be complete by LC-MS, water (30 mL) was added, and then extraction was performed with ethyl acetate (40mLX2). The organic phases were combined, washed with saturated saline water, dried with anhydrous sodium sulfate, filtered, concentrated, and subjected to column chromatography to obtain compound 29.3 (72 mg, yield: 77%). LCMS ESI(+) m/z: 585.2(M+1).

### Step D: (S)-4-(3-amino-1-oxo-1-(thieno[2,3-c]pyridin-2-ylamino)prop-2-yl)benzyl 2-(1-((nitrooxy)methyl)cyclopropyl acetate (compound 29)

Compound 29.3 (72 mg, 0.123 mmol) was dissolved in 15 mL of tetrahydrofuran, and 30 mL of hydrogen chloride/methanol solution (4 M) was added to stir at room temperature for 5 hours. The reaction was monitored to be complete by TLC. After the reaction was completed, the reaction solution was concentrated under reduced pressure, the pH was adjusted to 8 to 10 with sodium bicarbonate solution, and extraction was performed with ethyl acetate (50 mLX2). Then, washing was performed with saturated saline water (30 mLX1), and the organic phase was concentrated and then sent to p-HPLC for reverse phase preparation. After lyophilization, target product Example 29 (17 mg, yield: 28%, purity: 81%) was obtained. LCMS ESI(+) m/z: 485.1(M+1).

### Example 30

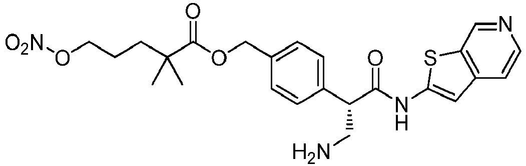

### (S)-4-(3-amino-1-oxy-1-(thieno[2,3-c]pyridin-2-ylamino)prop-2-yl)benzyl 2,2-dimethyl-5-(nitrooxy)valerate

The specific reaction equation is as follows:

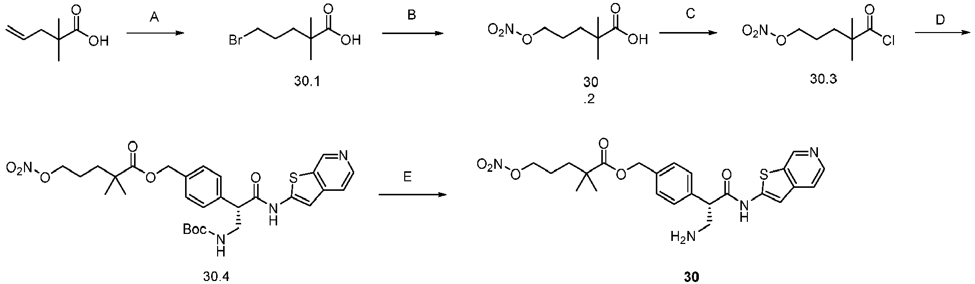

### Step A: 5-bromo-2,2-dimethylpentanoic acid (compound 30.1)

Compound 2,2-dimethylpent-4-enoic acid (900 mg, 7.02 mmol) was dissolved in n-hexane (35 mL), and then the mixture was cooled to 0 °C in an ice bath. An acetic acid solution of hydrobromic acid (3.44 g, 33% content) was added at this temperature, and then stirring was performed at room temperature for 4 hours. After the reaction was monitored to be complete by TLC, water (40 mL) was added to the mixture. Then, extraction was performed with ethyl acetate (50mL×2), and the combined organic phases were washed with water (30mL×2) and saturated saline water (30 mL×2), dried with anhydrous sodium sulfate, filtered, and concentrated to obtain compound 30.1 (1.36 g, yield: 93%).

### Step B: 2,2-dimethyl-5-(nitrooxy)pentanoic acid (compound 30.2)

Compound 30.1 (1.1 g, 5.26 mmol) was dissolved in acetonitrile (16 mL), then silver nitrate (1.79, 10.59 mmol) was added, and under nitrogen protection, the temperature was raised to 80 °C to react for 6 hours. After the reaction was completed, the reaction was cooled to room temperature, filtered, and the filtrate was concentrated. The residue was subjected to column chromatography to obtain compound 30.2 (340 mg, yield: 31%).

### Step C: 5-chloro-4,4-dimethyl-5-oxopentyl nitrate (compound 30.3)

Compound 30.2 (150 mg, 0.78 mmol) was dissolved in dichloromethane (16 mL), and the mixture was cooled to 0 °C in an ice bath under nitrogen protection. Oxalyl chloride (0.133 mL) and a catalytic amount of N,N-dimethylformamide were added, and stirring was performed at room temperature for 3 hours. After the reaction was completed, the reaction solution was concentrated to obtain the crude product of compound 30.3 used directly in the next step.

### Step D: (S)-4-(3-((tert-butoxycarbonyl)amino)-1-oxy-1-(thieno[2,3-c]pyridin-2-ylamino)prop-2-yl)benzyl 2,2-5-(nitrooxy)dimethyl valerate (compound 30.4)

Compound 15.2 (168 mg, 351.27 mmol) was dissolved in tetrahydrofuran (20 mL), and after diisopropylethylamine (303 mg, 2.35 mmol) was added, the mixture was cooled to 0 °C. Then, a dichloromethane (10 mL) solution of the crude product of compound 30.3 and 4-dimethylaminopyridine (96 mg, 0.786 mmol) were added dropwise. The mixture was stirred at room temperature for 1 hour. After the reaction was monitored to be complete by LCMS, water (30 mL) was added, and extraction was performed with ethyl acetate (50 mL×2). The combined organic phases were washed with saturated saline water (30 mL×1), dried with anhydrous sodium sulfate, filtered, and concentrated, and the residue was subjected to column chromatography to obtain compound 30.4 (150 mg, yield: 64%). LCMS ESI(+) m/z: 601.2(M+1).

### Step E: (S)-4-(3-amino-1-oxy-1-(thieno[2,3-c]pyridin-2-ylamino)prop-2-yl)benzyl 2,2-dimethyl-5-(nitrooxy)valerate (compound 30)

Compound 30.4 (150 mg, 0.25 mmol) was dissolved in 8 mL of dichloromethane, trifluoroacetic acid (2 mL) was added, and stirring was performed at room temperature for 1 hour. After the reaction was monitored to be complete by LC-MS, the pH was adjusted to 8 with sodium bicarbonate solution, and then extraction was performed with ethyl acetate (50 mL×2). The combined organic phases were washed with saturated saline water (30 mL×1), dried with anhydrous sodium sulfate, filtered, and concentrated. The residue was prepared by p-HPLC to obtain product Example 30 (82 mg, yield: 66%). LCMS ESI(+) m/z: 501.2(M+1). ¹HNMR (400 MHz, DMSO) δ13.63 (s, 1 H), 9.50 (s, 1 H), 8.52 (d, J = 6.4 Hz, 1 H), 8.30 ( s, 3 H), 8.16 (d, J = 6.4 Hz, 1 H), 7.51 (d, J = 8.4 Hz, 2 H), 7.43 (s, 1 H), 7.38 (d, J = 8.4 Hz, 2 H), 5.07 (s, 2 H), 4.52 (dd, J = 5.2, 8.8 Hz, 1 H), 4.48 - 4.40 (m, 2 H), 3.65 (d, J = 4.4 Hz, 1 H), 3.20 - 3.07 (m, 1 H), 1.64 - 1.47 (m, 4 H), 1.14 (s, 6 H).

### Example 31

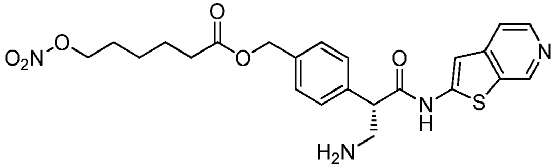

### (S)-4-(3-amino-1-oxy-1-(thieno[2,3-c]pyridin-2-ylamino)propan-2-yl)benzyl 6-(nitrooxy)hexanoate

The specific reaction equation is as follows:

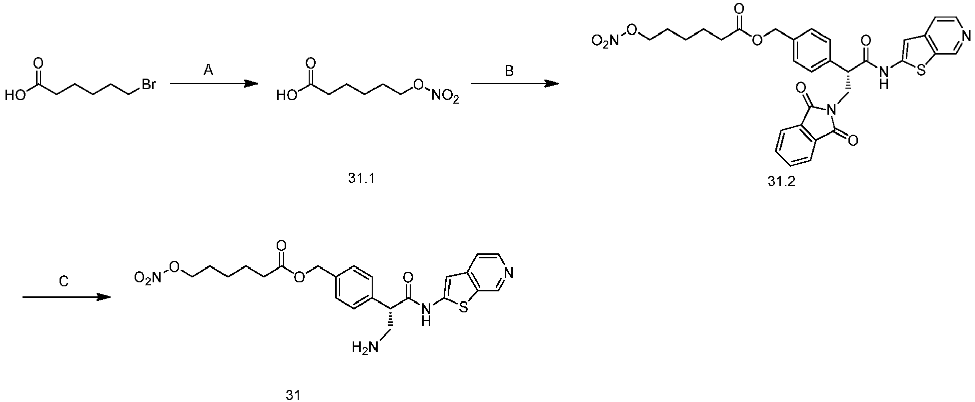

### Step A: 6-(nitrooxy)hexanoic acid (compound 31.1)

Weighed 6-bromohexanoic acid (500 mg, 2.56 mmol) was dissolved in 8 mL of acetonitrile, and silver nitrate (871 mg, 5.13 mmol) was added to the reaction solution. Protected from light, and under an inert gas atmosphere, the reaction solution was stirred at 85 °C for 5 hours. The reaction was confirmed to be complete by TLC (bromocresol green color development). The precipitated solid in the reaction solution was filtered off with diatom, the filter cake was washed with acetonitrile, the filtrate was spin-dried, and the residue was dissolved in ethyl acetate. The organic phase was washed with water (15 mL) and saturated saline water (15 mL) successively, dried with anhydrous sodium sulfate, filtered, and spin-dried to obtain colorless liquid 31.1 (400 mg, yield: 88%).

### Step B: ((S)-4-(3-(1,3-dioxyisoindol-2-yl)-1-oxy-1-(thieno[2,3-c]pyridin-2-ylamino)prop-2-yl)benzyl 6-(nitrooxy)hexanoate (compound 31.2)

Compound 1.11 (100 mg, 0.219 mmol) was dissolved in 7 mL of N,N-dimethylformamide, and then 1-(3-dimethylaminopropyl)-3-ethylcarbodiimide hydrochloride (63.3 mg, 0.33 mmol), 4-dimethylaminopyridine (40.26 mg, 0.33 mmol), and compound 31.1 (46.55 mg, 0.263 mmol) were added. The mixture was stirred overnight at room temperature. The reaction was confirmed to be complete by LCMS. Water was added to the reaction solution, extraction was performed with ethyl acetate (15 mL), the organic phases were separated and combined, and then the organic phases were washed with saturated saline water. The organic phases were dried with anhydrous sodium sulfate, filtered, and spin-dried. The residue was purified by silica column to obtain light yellow solid 31.2 (80 mg, yield: 59%). LCMS ESI(+) m/z:617.2(M+1).

### Step C: (S)-4-(3-amino-1-oxy-1-(thieno[2,3-c]pyridin-2-ylamino)propan-2-yl)benzyl 6-(nitrooxy)hexanoate (compound 31)

Compound 31.2 (80 mg, 0.13 mmol) was dissolved in 8 mL of ethanol, hydrazine hydrate (65 mg, 1.3 mmol) was added, the reaction was heated to 60 °C, and the mixture was stirred for 3 hours under an inert gas atmosphere. The reaction was confirmed to be complete by LCMS. The reaction was quenched by adding water, and extraction was performed with ethyl acetate (12 mL). The organic phase was washed with saturated saline water, dried with anhydrous sodium sulfate, filtered, concentrated, and spin-dried. The residue was dissolved in methanol/tetrahydrofuran (1.5 mL/1.5 mL). After reverse preparation and purification, light yellow solid product Example 31 (35 mg, yield: 52%, purity: 96%) was obtained. LCMS ESI(+) m/z:487.2(M+1). ¹H NMR (400 MHz, DMSO) δ 13.54 (s, 1H), 9.48 (s, 1H), 8.52 (d, *J* = 6.4 Hz, 1H), 8.25 (s, 3H), 8.15 (d, J = 6.4 Hz, 1H), 7.50 (d, J = 8.0 Hz, 2H), 7.42 - 7.36 (m, 3H), 5.07 (s, 2H), 4.52 - 4.45 (m, 3H), 3.70 - 3.56 (m, 1H), 3.20 - 3.10 (m, 1H), 2.36 (t, *J* = 7.6 Hz, 2H), 1.68 - 1.60 (m, 2H), 1.56 (dt, *J* = 15.2, 7.6 Hz, 2H), 1.39 - 1.28 (m, 2H).

### Example 32

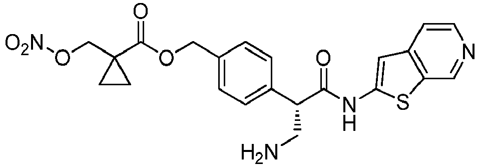

### (S)-4-(3-amino-1-oxy-1-(thieno[2,3-c]pyridin-2-ylamino)propan-2-yl)benzyl 1-((nitrooxy)methyl)cyclopropane carboxylate

The specific reaction equation is as follows:

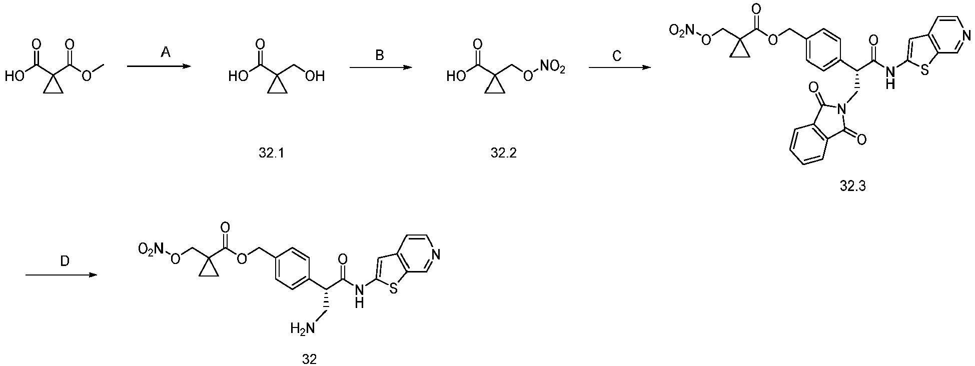

### Step A: 1-(hydroxymethyl)cyclopropanecarboxylic acid (compound 32.1)

Under an ice bath, 1-(methoxycarbonyl)cyclopropane carboxylic acid (650 mg, 4.51 mmol) was dissolved in 10 mL of anhydrous tetrahydrofuran. Under nitrogen protection, the dimethyl sulfide solution of borane (0.5 mL, 4.961 mmol) was slowly added dropwise to the reaction solution, and then the reaction was stirred at room temperature for 1.5 hours. The reaction was confirmed to be complete by TLC (bromocresol green color development). Then, the reaction solution was cooled to 0 °C, and the reaction was quenched with methanol. The reaction solution was concentrated and spin-dried to obtain light yellow oily crude product 32.1 (410 mg, yield: 69.87%).

### Step B: 1-((nitrooxy)methyl)cyclopropanecarboxylic acid (compound 32.2)

Under ice bath conditions, weighed acetic anhydride (2 mL) was added to a single-neck reaction flask (50 mL), and then concentrated nitric acid (1 mL) was slowly added dropwise to the reaction flask, and the reaction solution was stirred under the ice bath for 10 minutes. Under ice bath conditions, compound 32.1 (150 mg, 1.29 mmol) was dissolved in acetic anhydride (3 mL), then an acetic anhydride-concentrated nitric acid mixture (3 mL) was slowly added dropwise to the reaction solution, and then the reaction solution was stirred at room temperature for 1 hour. The reaction was confirmed to be complete by TLC (bromocresol green color development). Water was added to the reaction solution, extraction was performed with ethyl acetate (15 mL), the organic phases were separated and combined, and then the organic phases were washed with saturated saline water. The organic phases were dried with anhydrous sodium sulfate, filtered, spin-dried, and purified by silica column to obtain white solid 32.2 (60 mg, yield: 43%).

### Step C: (S)-4-(3-(1,3-dioxyisoindol-2-yl)-1-oxy-1-(thieno[2,3-c]pyridin-2-ylamino)prop-2-yl)benzyl 1-((nitrooxy)methyl)cyclopropanecarboxylic acid (compound 32.3)

Compound 32.2 (55 mg, 0.342 mmol) was dissolved in N,N-dimethylformamide (7 mL), and then 1-(3-dimethylaminopropyl)-3-ethylcarbodiimide hydrochloride (98.34 mg, 0.513 mmol), 4-dimethylaminopyridine (62.58 mg, 0.513 mmol), and compound 1.11 (109.7 mg, 0.24 mmol) were added successively. The mixture was stirred overnight at room temperature. The reaction was confirmed to be complete by LCMS (about 15% of raw material 1.11 remained). Water was added to the reaction solution, extraction was performed with ethyl acetate (15 mL), and the organic phases were separated and combined. The organic phases were washed with saturated saline water, dried with anhydrous sodium sulfate, filtered, spin-dried, and purified by silica column to obtain white solid 32.3 (82 mg, yield: 40%). LCMS ESI(+) m/z:601.1(M+1).

### Step D: (S)-4-(3-amino-1-oxy-1-(thieno[2,3-c]pyridin-2-ylamino)prop-2-yl)benzyl 1-((nitrooxy)methyl)cyclopropane carboxylate (compound 32)

Compound 32.3 (82 mg, 0.137 mmol) was dissolved in 7 mL of ethanol, and then hydrazine hydrate (68.5 mg, 1.37 mmol) was added to the reaction solution. The reaction was heated to 60 °C and stirred for 3 hours under an inert gas atmosphere. The reaction was confirmed to be complete by LCMS. Water was added to the reaction solution, and extraction was performed with ethyl acetate (12 mL). The organic phase was washed with saturated saline water, dried with anhydrous sodium sulfate, filtered, concentrated, and spin-dried. The residue was dissolved in methanol/tetrahydrofuran (1.5 mL/1.5 mL). After reverse preparation and purification, white solid product Example 32 (29.5 mg, yield: 43%, purity: 94%) was obtained. LCMS ESI(+) m/z:471.1(M+1).¹H NMR (400 MHz, DMSO) δ 13.47 (s, 1H), 9.48 (s, 1H), 8.52 (d, J = 6.4 Hz, 1H), 8.25 - 8.13 (m, 4H), 7.49 (d, J = 8.4 Hz, 2H), 7.41 - 7.35 (m, 3H), 5.11 (s, 2H), 4.71 (s, 2H), 4.47 (dd, J = 8.4, 5.6 Hz, 1H), 3.64 (s, 1H), 3.17 (d, J = 6.4 Hz, 1H), 1.31 (dd, J = 7.2, 4.2 Hz, 2H), 1.19 (dd, J = 7.2, 4.4 Hz, 2H).

### Example 33

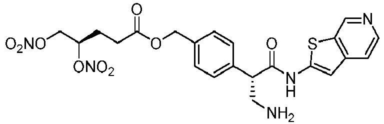

### 4-((S)-3-amino-1-oxy-1-(thieno[2,3-c]pyridin-2-ylamino)prop-2-yl)benzyl(R)-4,5-bis(nitrooxy)valeric acid

The specific reaction equation is as follows:

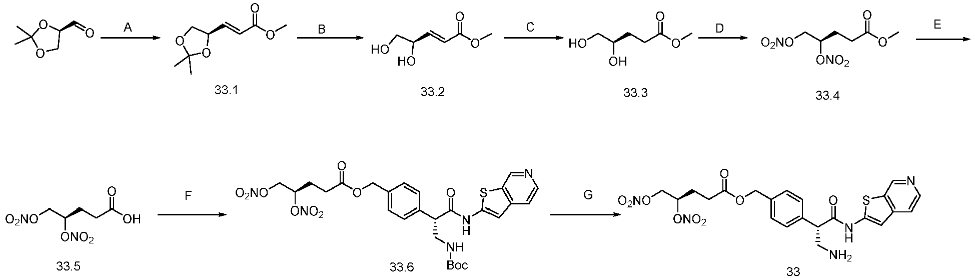

### Step A: (S,E)-3-(2,2-dimethyl-1,3-dioxolane-4-yl)methyl acrylate (compound 33.1)

Compound (2-methoxyethyl)dimethyl phosphate (2.28 mL, 15.36 mmol) was slowly added dropwise to 15 mL of glycol dimethyl ether containing sodium hydride (60%, 614 mg, 15.36 mmol) at 0 °C to stir for 0.5 hours. Then, (S)-2,2-dimethyl-1,3-dioxolane-4-carbaldehyde (2 g, 15.36 mmol) was added to stir at room temperature for 2 hours. The reaction was monitored to be complete by TLC. After the reaction was completed, the reaction was quenched by adding saturated ammonium chloride solution, and extraction was performed with ethyl acetate (100 mL). The organic phases were combined and washed with saturated saline water (50 mL). The organic phases were dried with anhydrous sodium sulfate, filtered, spin-dried, and purified by column chromatography to obtain compound 33.1 (1.645 g, yield: 58%, colorless oily liquid).

### Step B: methyl (S,E)-4,5-dihydroxypent-2-enoate (compound 33.2)

Compound 33.1 (1.16 g, 6.23 mmol) was dissolved in 7.5 mL of aqueous acetic acid (80%), and heated to 80 °C to stir for 3 hours. The reaction was monitored to be complete by TLC. After the reaction was completed, the reaction solution was directly spin-dried and purified by column chromatography to obtain compound 33.2 (706 mg, yield: 78%, colorless oily liquid).

### Step C: methyl (R)-4,5-dihydroxyvalerate (compound 33.3)

Compound 33.2 (706 mg, 4.84 mmol) was dissolved in 10 mL of ethanol, and under a hydrogen atmosphere, palladium on carbon (10%, 71 mg) was added to stir at room temperature overnight. The reaction was monitored to be complete by TLC. After the reaction was completed, the reaction solution was filtered with diatomite, the filtrate was spin-dried, and the residue was purified by column chromatography to obtain compound 33.3 (558 mg, yield: 78%, colorless oily liquid).

### Step D: methyl (R)-4,5-bis(nitrooxy)valerate (compound 33.4)

Compound 33.3 (100 mg, 0.68 mmol) was dissolved in 1 mL of acetic anhydride, a mixed acid solution of concentrated nitric acid and acetic anhydride (0.8 mL of nitric acid, 1.6 mL of acetic anhydride) was added under an ice-water bath, and stirring was performed at 0 °C for 4 hours. The reaction was monitored to be complete by TLC. After the reaction was completed, 30 mL of ethyl acetate was added to dilute, and the reaction solution was washed with water (10 mLX2), saturated sodium bicarbonate solution (10 mL), and saturated saline water (10 mLX2) successively. The organic phase was dried with anhydrous sodium sulfate, filtered, and spin-dried to obtain crude product 33.4 (138 mg, yield: 85%, light yellow liquid).

### Step E: (R)-4,5-bis(nitrooxy)valerate (compound 33.5)

Compound 33.4 (93 mg, 0.39 mmol) was dissolved in a mixed solution of 1.5 mL of tetrahydrofuran and 0.5 mL of water. Lithium hydroxide monohydrate (20 mg, 0.47 mmol) was added under an ice-water bath to stir at this temperature for 1 hour. The reaction was monitored to be complete by TLC. After the reaction was completed, the pH was adjusted to 4 with 2 M hydrochloric acid solution, and extraction was performed with ethyl acetate (20 mL). The organic phase was washed with saturated saline water, dried with anhydrous sodium sulfate, filtered, spin-dried, and purified by column chromatography to obtain compound 33.5 (45 mg, yield: 85%, colorless oily liquid). ¹H NMR (400 MHz, DMSO-*d*₆) δ 12.28 (s, 1H), 5.70 - 5.22 (m, 1H), 4.94 (dd, J = 12.8, 2.4 Hz, 1H), 4.72 (dd, J = 12.8, 6.0 Hz, 1H), 2.48 - 2.17 (m, 2H), 2.13 - 1.70 (m, 2H).

### Step F: 4-((S)-3-((tert-butoxycarbonyl)amino)-1-oxo-1-(thieno[2,3-c]pyridin-2-ylamino)prop-2-yl)benzyl(R)-4,5-bis(nitrooxy)valerate (compound 33.6)

Compound 15.2 (51 mg, 0.12 mmol) and compound 33.5 (40 mg, 0.18 mmol) were dissolved in 2 mL of N,N-dimethylformamide. Then, 1-ethyl-(3-dimethylaminopropyl)carbodiimide hydrochloride (46 mg, 0.24 mmol) and 4-dimethylaminopyridine (22 mg, 0.18 mmol) were added to react overnight at room temperature. The reaction was monitored to be complete by LCMS. Water was added to quench the reaction, extraction was performed with ethyl acetate (30 mLX2), and the organic phases were combined and washed with saturated saline water (10 mLX2). The organic phases were dried with anhydrous sodium sulfate, filtered, spin-dried, and purified by column chromatography to obtain product 33.6 (55 mg, yield: 72%, light yellow solid).

### Step G: 4-((S)-3-amino-1-oxy-1-(thieno[2,3-c]pyridin-2-ylamino)prop-2-yl)benzyl(R)-4,5-bis(nitrooxy)valeric acid (compound 33)

Compound 33.6 (55 mg, 0.087 mmol) was dissolved in 2 mL of ethyl acetate, and 2 mL of ethyl acetate hydrochloric acid solution (2 M) was added to stir at room temperature for 2 hours. The reaction was monitored to be complete by LCMS. After the reaction was completed, the solvent was spin-dried, and the residue was dissolved in ultrapure water, filtered, and purified by reverse phase preparation. After lyophilization, target product Example 33 (45 mg, yield: 97%, purity: 99%) was obtained. LCMS ESI(+) m/z: 534.1(M+1). ¹H NMR (400 MHz, DMSO-d6) δ 13.22 (s, 1H), 9.45 (s, 1H), 8.52 (d, J = 6.4 Hz, 1H), 8.11 (s, 4H), 7.46 (d, J = 7.6 Hz, 2H), 7.40 (d, J = 8.4 Hz, 2H), 7.34 (s, 1H), 5.49-5.40 (m, 1H), 5.08 (s, 2H), 4.92 (dd, J = 12.8, 2.8 Hz, 1H), 4.71 (dd, J = 12.8, 6.0 Hz, 1H), 4.39 (s, 1H), 3.63 (s, 1H), 3.17 (d, J = 5.6 Hz, 1H), 2.59-2.53 (m, 2H), 2.08-1.87 (m, 2H).

### Example 34

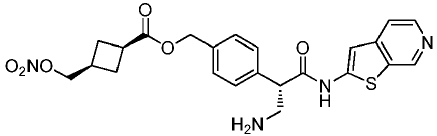

### (1S,3R)-4-((S)-3-amino-1-oxy-1-(thieno[2,3-c]pyridin-2-ylamino)propan-2-yl)benzyl 3-((nitrooxy)methyl)cyclobutane-1-carboxylate

The specific reaction equation is as follows:

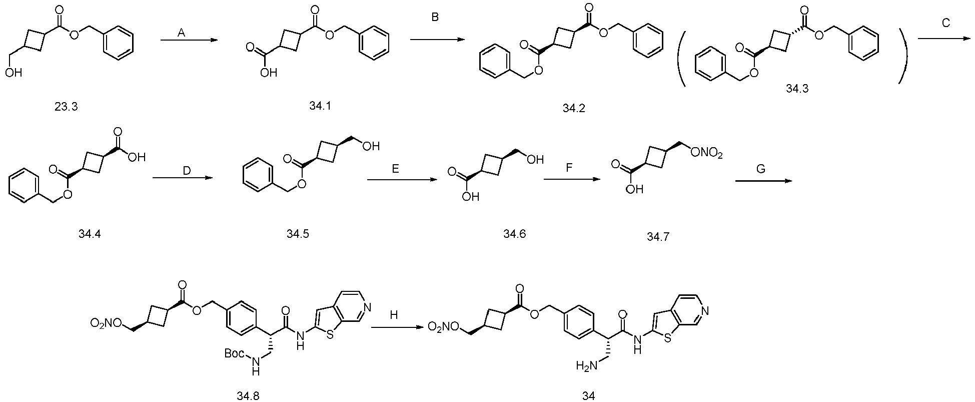

### Step A: 3-((benzyloxy)carbonyl)cyclobutanecarboxylic acid (compound 34.1)

Compound 23.3 (2.33 g, 10.58 mmol) was dissolved in 30 mL of dichloromethane, 30 mL of acetonitrile, and 30 mL of water, and the reaction solution was cooled to 0 °C. Sodium periodate (6.8 g, 31.73 mmol) and ruthenium trichloride monohydrate (233 mg, 1.06 mmol) were added, and the reaction solution was raised to room temperature and reacted for 6 hours. The reaction solution was diluted with ethyl acetate (100 mL), 1 M hydrochloric acid solution was added while stirring until all the solids were dissolved, and the aqueous phase was extracted one time with ethyl acetate (50 mL). The combined organic phases were washed two times with 10% sodium bisulfite solution (30 mL), washed with saturated saline water (30 mL) one time, dried with anhydrous sodium sulfate, filtered, spin-dried, and purified by column chromatography to obtain product 34.1 (2.3 g, yield: 93%, colorless oily substance). LCMS ESI(+)m/z:235.1(M+1).

### Step B: (1S,3S)-dibenzylcyclobutane-1,3-dicarboxylate (compound 34.2) and (1r,3r)-dibenzylcyclobutane-1,3-dicarboxylate (compound 34.3))

Compound 34.1 (2.3 g, 9.82 mmol) was dissolved in 30 mL of N,N-dimethylformamide, and potassium carbonate (4.07 g, 29.46 mmol) and benzyl bromide (5.04 g, 29.46 mmol) were added to react at room temperature overnight. After the reaction was monitored to be complete by LCMS, water (50 mL) was added to the reaction solution, and extraction was performed two times with ethyl acetate (70 mL). The combined organic phases were washed with saturated saline water (50 mL) four times, dried with anhydrous sodium sulfate, filtered, spin-dried, and purified by column chromatography to obtain relatively less polar product 34.2 (1.29 g, colorless oily substance) and relatively more polar product 34.3 (1.58 g, colorless oily liquid, yield: 90%).

### Step C: (1s,3s)-3-((benzyloxy)carbonyl)cyclobutanecarboxylic acid (compound 34.4)

Compound 34.2 (1.19 g, 3.67 mmol) was dissolved in 15 mL of tetrahydrofuran, 15 mL of methanol, and 15 mL of water, sodium hydroxide (146.7 mg, 3.67 mmol) was added, and the reaction solution was reacted at room temperature for 3 hours. After the reaction was monitored to be complete by TLC, 2 M hydrochloric acid solution was added to the reaction solution to pH 3, and then extraction was performed two times with ethyl acetate (60 mL). The combined organic phases were washed one time with saturated saline water (30 mL), dried with anhydrous sodium sulfate, filtered, spin-dried, and purified by column chromatography to obtain product 34.4 (380 mg, yield: 44%, colorless oily substance). LCMS ESI(+)m/z:235.1(M+1).

### Step D: (1s,3s)-benzyl-3-(hydroxymethyl)cyclobutanecarboxylate (compound 34.5)

Compound 34.4 (380 mg, 1.62 mmol) was dissolved in 15 mL of tetrahydrofuran, and 0.24 mL of borane dimethyl sulfide solution (10 M) was added dropwise in an ice bath under nitrogen protection. After the dropwise addition was completed, the mixture was reacted at room temperature for 3 hours. After the reaction was monitored to be complete by TLC, 5 mL of methanol and 4 mL of 2 M hydrochloric acid solution were added in the ice bath successively, and stirring was continued for 10 minutes at room temperature. Water (20 mL) was added to the reaction solution, and extraction was performed with ethyl acetate (50 mL×4). The combined organic phases were washed with saturated saline water (30 mL), dried with anhydrous sodium sulfate, filtered, spin-dried, and purified by column chromatography to obtain product 34.5 (245 mg, yield: 69%, colorless oily substance). LCMS ESI(+)m/z:221.1(M+1).

### Step E: (1s, 3s)-3-(hydroxymethyl)cyclobutane carboxylic acid (compound 34.6)

Compound 34.5 (245 mg, 1.11 mmol) was dissolved in 6 mL of methanol and 15 mL of tetrahydrofuran, and an aqueous solution (5 mL) of sodium hydroxide (89 mg, 2.22 mmol) was added to react by stirring at room temperature for 3 hours. After the reaction was monitored to be complete by TLC, the reaction solution was diluted with water (40 mL), washed with ethyl acetate (25 mL×3), then the aqueous phase was adjusted to pH 2 with 2 M hydrochloric acid solution, and extracted with ethyl acetate (40mL×6). The organic phases were combined, dried with anhydrous sodium sulfate, filtered, and spin-dried to obtain compound 34.6 (145 mg, yield: 100%, white solid).

### Step F: (1s,3s)-3-((nitrooxy)methyl)cyclobutanecarboxylic acid (compound 34.7)

In an ice-water bath, 1.5 mL of concentrated nitric acid was added dropwise to 3 mL of acetic anhydride and stirring was performed for 5 minutes. Compound 34.6 (145 mg, 1.11 mmol) was dissolved in 5 mL of acetic anhydride, and the nitric acid acetic anhydride solution was added dropwise under the ice-water bath to react at 0 °C for 3 hours. After the reaction was monitored to be complete by TLC, the reaction solution was diluted with water (20 mL) and extracted with ethyl acetate (40 mL×2). The organic phases were combined, washed with saturated saline water (20 mL), dried with anhydrous sodium sulfate, filtered, spin-dried, and purified by column chromatography to obtain product 34.7 (110 mg, yield: 56%, white solid).

### Step G: (1s,3R)-4-((S)-3-((tert-butoxycarbonyl)amino)-1-oxo-1-(thieno[2,3-c]pyridin-2-ylamino)prop-2-yl benzyl 3-((nitrooxy)methyl)cyclobutanecarboxylate (compound 34.8)

Compound 34.7 (50 mg, 0.28 mmol) was dissolved in 20 mL of N,N-dimethylformamide, and 1-(3-dimethylaminopropyl)-3-ethylcarbodiimide hydrochloride (82 mg, 0.42 mmol), 4-dimethylaminopyridine (53 mg, 0.42 mmol), and compound 15.2 (85 mg, 0.20 mmol) were added. The reaction solution was reacted overnight at room temperature. After the reaction was monitored to be complete by TLC, the reaction solution was diluted with water (30 mL) and extracted with ethyl acetate (40 mL×2). The organic phases were combined, washed with saturated saline water (20 mL×2), dried with anhydrous sodium sulfate, filtered, and spin-dried. The residue was purified by column chromatography to obtain product 34.8 (90 mg, yield: 78%, white solid). LCMS ESI(+)m/z:585.2(M+1).

### Step H: (1s,3R)-4-((S)-3-amino-1-oxo-1-(thieno[2,3-c]pyridin-2-ylamino)propane-2-yl)benzyl 3-((nitrooxy)methyl)cyclobutane-1-carboxylate (compound 34)

After compound 34.8 (90 mg, 0.15 mmol) was dissolved in 8 mL of dichloromethane, 1.5 mL of trifluoroacetic acid was added to react at room temperature for 1 hour. After the reaction was monitored to be complete by LCMS, saturated sodium bicarbonate solution was added to the reaction solution to pH 8, and extraction was performed with ethyl acetate (50mLX2). The combined organic phases were washed with saturated saline water (20 mL), dried with anhydrous sodium sulfate, filtered, spin-dried, and purified by reverse preparation to obtain product Example 34 (64 mg, yield: 85%, white solid). LCMS ESI(+)m/z:485.1 (M+1). ¹H NMR (400 MHz, DMSO) δ13.53 ( s, 1 H), 9.48 (s, 1 H), 8.52 (d, J = 6.4 Hz, 1 H), 8.24 ( s, 3 H), 8.15 (d, J = 6.4 Hz, 1 H), 7.50 (d, J = 8.0 Hz, 2 H), 7.43 - 7.34 (m, 3 H), 5.09 (s, 2 H), 4.58 (d, J = 7.2 Hz, 2 H), 4.53 - 4.44 (m, 1 H), 3.62-3.66 (m, 1 H), 3.22-3.29 (m, 1 H), 3.19 - 3.08 (m, 1 H), 2.59-2.68 (m, 1 H), 2.27-2.34 (m, 2 H), 2.03-2.10 (m, 2 H).

### Example 35

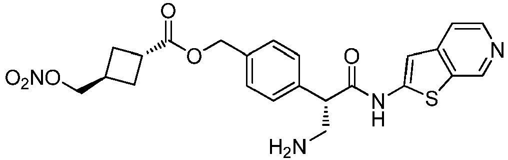

### (1R,3S)-4-((S)-3-amino-1-oxy-1-(thieno[2,3-c]pyridin-2-ylamino)propan-2-yl)benzyl 3-((nitrooxy)methyl)cyclobutane-1-carboxylate

The specific reaction equation is as follows:

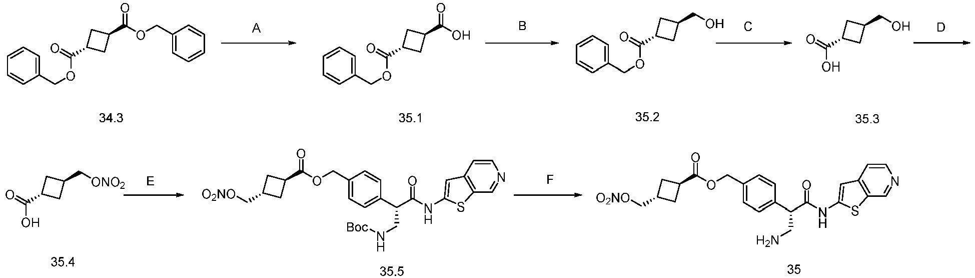

### Step A: (1R,3r)-3-((benzyloxy)carbonyl)cyclobutanecarboxylic acid (compound 35.1)

Compound 34.3 (1.48 g, 4.56 mmol) was dissolved in 15 mL of tetrahydrofuran, 15 mL of methanol, and 15 mL of water, sodium hydroxide (182 mg, 4.56 mmol) was added, and the reaction solution was reacted at room temperature for 3 hours. After the reaction was monitored to be complete by TLC, 2 M hydrochloric acid solution was added to the reaction solution to pH 3, and then extraction was performed two times with ethyl acetate (60 mL). The combined organic phases were washed one time with saturated saline water (30 mL), dried with anhydrous sodium sulfate, filtered, spin-dried, and purified by column chromatography to obtain product 35.1 (380 mg, yield: 36%, colorless oily substance). LCMS ESI(+)m/z:235.1(M+1).

### Step B: (1r,3r)-benzyl-3-(hydroxymethyl)cyclobutanecarboxylate (compound 35.2)

Compound 35.1 (380 mg, 1.62 mmol) was dissolved in 15 mL of tetrahydrofuran, and 0.24 mL of borane dimethyl sulfide solution (10 M) was added dropwise in an ice bath under nitrogen protection. After the dropwise addition was completed, the mixture was reacted at room temperature for 3 hours. After the reaction was monitored to be complete by TLC, 5 mL of methanol and 4 mL of 2 M hydrochloric acid solution were added in the ice bath successively, and stirring was continued for 10 minutes at room temperature. Water (20 mL) was added to the reaction solution, and extraction was performed with ethyl acetate (50 mL×4). The combined organic phases were washed with saturated saline water (30 mL), dried with anhydrous sodium sulfate, filtered, spin-dried, and purified by column chromatography to obtain product 35.2 (300 mg, yield: 84%, colorless oily substance). LCMS ESI(+)m/z:221.1(M+1).

### Step C: (1r,3r)-3-(hydroxymethyl)cyclobutane carboxylic acid (compound 35.3)

Compound 35.2 (300 mg, 1.36 mmol) was dissolved in 6 mL of methanol and 15 mL of tetrahydrofuran, and an aqueous solution (5 mL) of sodium hydroxide (109 mg, 2.72 mmol) was added to react by stirring at room temperature for 3 hours. After the reaction was monitored to be complete by TLC, the reaction solution was diluted with water (40 mL), washed with ethyl acetate (25 mL×3), then the aqueous phase was adjusted to pH 2 with 2 M hydrochloric acid solution, and extracted with ethyl acetate (40mL×6). The organic phases were combined, dried with anhydrous sodium sulfate, filtered, and spin-dried to obtain compound 35.3 (177 mg, yield: 100%, colorless oily substance).

### Step D: (1r,3r)-3-((nitrooxy)methyl)cyclobutanecarboxylic acid (compound 35.4)

In an ice-water bath, 1.5 mL of concentrated nitric acid was added dropwise to 3 mL of acetic anhydride and stirring was performed for 5 minutes. Compound 35.3 (177 mg, 1.36 mmol) was dissolved in 5 mL of acetic anhydride, and the nitric acid acetic anhydride solution was added dropwise under an ice-water bath to react at 0 °C for 3 hours. After the reaction was monitored to be complete by TLC, the reaction solution was diluted with water (20 mL) and extracted with ethyl acetate (40 mL×2). The organic phases were combined, washed with saturated saline water (20 mL), dried with anhydrous sodium sulfate, filtered, spin-dried, and purified by column chromatography to obtain product 35.4 (120 mg, yield: 50%, colorless oily substance).

### Step E: (1r,3S)-4-((S)-3-((tert-butoxycarbonyl)amino)-1-oxo-1-(thieno[2,3-c]pyridin-2-ylamino)prop-2-yl benzyl 3-((nitrooxy)methyl)cyclobutanecarboxylate (compound 35.5)

Compound 35.4 (50 mg, 0.28 mmol) was dissolved in 20 mL of N,N-dimethylformamide, and 1-(3-dimethylaminopropyl)-3-ethylcarbodiimide hydrochloride (82 mg, 0.42 mmol), 4-dimethylaminopyridine (53 mg, 0.42 mmol), and compound 15.2 (85 mg, 0.20 mmol) were added. The reaction solution was reacted overnight at room temperature. After the reaction was monitored to be complete by TLC, the reaction solution was diluted with water (30 mL) and extracted with ethyl acetate (40 mL×2). The organic phases were combined, washed with saturated saline water (20 mL×2), dried with anhydrous sodium sulfate, filtered, spin-dried, and purified by column chromatography to obtain product 35.5 (96 mg, yield: 82%, white solid). LCMS ESI(+)m/z:585.2(M+1).

### Step F: (1r,3S)-4-((S)-3-amino-1-oxo-1-(thieno[2,3-c]pyridin-2-ylamino)propane-2-yl)benzyl 3-((nitrooxy)methyl)cyclobutane-1-carboxylate (compound 35)

After compound 35.5 (96 mg, 0.164 mmol) was dissolved in 8 mL of dichloromethane, 1.5 mL of trifluoroacetic acid was added to react at room temperature for 1 hour. After the reaction was monitored to be complete by LCMS, saturated sodium bicarbonate solution was added to the reaction solution to pH 8, and extraction was performed with ethyl acetate (50 mLX2). The combined organic phases were washed with saturated saline water (20 mL), dried with anhydrous sodium sulfate, filtered, spin-dried, and purified by reverse preparation to obtain product Example 35 (66 mg, yield: 83%, white solid). LCMS ESI(+)m/z:485.1 (M+1). ¹H NMR (400 MHz, DMSO) δ13.53(s, 1 H), 9.47 (d, J = 5.2 Hz, 1 H), 8.52 (d, J = 6.4 Hz, 1 H), 8.37 - 8.08 (m, 4 H), 7.49 (t, J = 8.4 Hz, 2 H), 7.44 - 7.32 (m, 3 H), 5.07 (s, 2 H), 4.61 - 4.40 (m, 3 H), 3.63 ( s, 1 H), 3.09-3.18 (m, 2 H), 2.68 - 2.54 (m, 1 H), 2.34 - 2.19 (m, 2 H), 2.08 - 1.95 (m, 2 H).

### Example 36

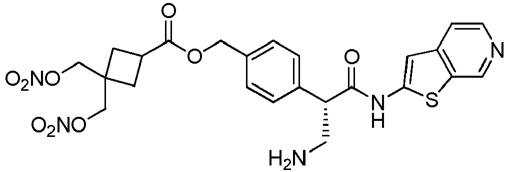

### (S)-4-(3-amino-1-oxy-1-(thieno[2,3-c]pyridin-2-ylamino)prop-2-yl)benzyl 3,3-bis((nitrooxy)methyl)cyclobutane-1-carboxylate

The specific reaction equation is as follows:

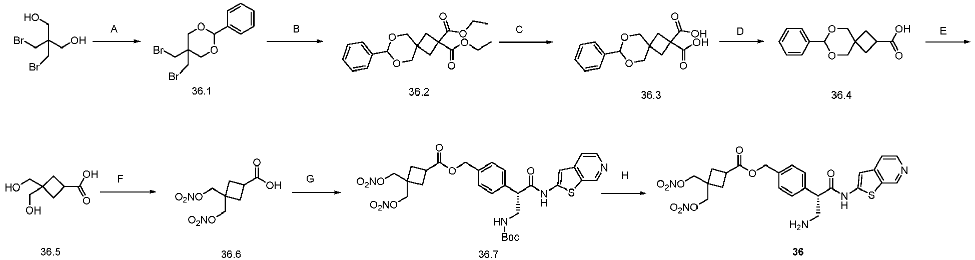

### Step A: 5,5-bis(bromomethyl)-2-phenyl-1,3-dioxane (compound 36.1)

2,2-bis(bromomethyl)propane-1,3-diol (10 g, 38.18 mmol) was dissolved in 140 mL of toluene, and benzaldehyde (4.25 g, 40.09 mmol) and p-toluenesulfonic acid (657 mg, 3.82 mmol) were added. The reaction solution was raised to 120 °C and reacted for 4 hours. The reaction solution was cooled to room temperature, washed with saturated sodium bicarbonate solution (40 mL) three times, and washed with saturated saline water (30 mL) one time. The reaction solution was dried with anhydrous sodium sulfate, filtered, and spin-dried. The residue was recrystallized with methanol at room temperature to obtain product 36.1 (12.7 g, yield: 95%, white solid).

### Step B: diethyl 7-phenyl-6,8-dioxaspiro[3.5]nonane-2,2-dicarboxylate (compound 36.2)

Diethyl malonate (2.0 g, 12.49 mmol) was added dropwise to 20 mL of N,N-dimethylformamide suspension containing sodium hydrogen (500 mg, 12.49 mmol), and stirring was performed until no more bubbles were released. 10 mL of a N,N-dimethylformamide solution of compound 36.1 (2.19 g, 6.24 mmol) was added, and the reaction solution was reacted at 140 °C for 3 hours. After the reaction was monitored to be complete by LCMS, the reaction solution was cooled to room temperature, ammonium chloride solution (30 mL) was added, and extraction was performed two times with ethyl acetate (70 mL). The combined organic phases were washed with saturated saline water (50 mL) four times, dried with anhydrous sodium sulfate, filtered, spin-dried, and purified by column chromatography to obtain product 36.2 (1.4 g, yield: 64%, white solid). LCMS ESI(+)m/z:349.2(M+1).

### Step C: 7-phenyl-6,8-dioxaspiro[3.5]nonane-2,2-dicarboxylic acid (compound 36.3)

Compound 36.2 (1.4 g, 4.02 mmol) was dissolved in 20 mL of ethanol and 15 mL of water, potassium hydroxide (676 mg, 12.06 mmol) was added, and the reaction solution was reacted at 80 °C for 0.5 hours. After the reaction was monitored to be complete by LCMS, the reaction solution was concentrated under reduced pressure to remove ethanol, and 2 M hydrochloric acid solution was added to the reaction solution to pH 4. A solid was precipitated, filtered, and dried to obtain product 36.3 (1.09 g, yield: 93%, white solid). LCMS ESI(+)m/z:293.1(M+1).

### Step D: 7-phenyl-6,8-dioxaspiro[3.5]nonane-2-carboxylic acid (compound 36.4)

Compound 36.3 (1.09 g, 3.73 mmol) was dissolved in 20 mL of pyridine, and the reaction solution was refluxed for 20 hours. After the reaction was monitored to be complete by LCMS, the reaction solution was concentrated under reduced pressure to remove pyridine, and the residue was adjusted to pH 5 with 2 M hydrochloric acid solution, and extracted with ethyl acetate (50 mL×2). The combined organic phases were washed with saturated saline water (30 mL), dried with anhydrous sodium sulfate, filtered, spin-dried, and purified by column chromatography to obtain product 36.4 (500 mg, yield: 54%, white solid). LCMS ESI(+)m/z:249.1(M+1).

### Step E: 3,3-bis(hydroxymethyl)cyclobutane carboxylic acid (compound 36.5)

Compound 36.4 (350 mg, 1.41 mmol) was dissolved in 25 mL of methanol, palladium on carbon (100 mg) was added, and the reaction solution was stirred and reacted for 36 hours at room temperature under a hydrogen atmosphere. After the reaction was monitored to be complete by TLC, the reaction solution was filtered with diatomite, and the filtrate was concentrated under reduced pressure to obtain compound 36.5 (220 mg, yield: 98%, white solid).

### Step F: 3,3-bis((nitrooxy)methyl)cyclobutane carboxylic acid (compound 36.6)

In an ice-water bath, 1.5 mL of concentrated nitric acid was added dropwise to 3 mL of acetic anhydride and stirring was performed for 10 minutes. Compound 36.5 (110 mg, 0.69 mmol) was dissolved in 15 mL of acetic anhydride, and the nitric acid acetic anhydride solution was added dropwise under the ice-water bath to react at room temperature for 2 hours. After the reaction was monitored to be complete by TLC, the reaction solution was diluted with water (20 mL) and extracted with ethyl acetate (40 mL×2). The organic phases were combined, washed with saturated saline water (20 mL), dried with anhydrous sodium sulfate, filtered, spin-dried, and purified by column chromatography to obtain product 36.6 (57mg, yield: 33%, white solid).

### Step G: (S)-4-(3-((tert-butoxycarbonyl)amino)-1-oxy-1-(thieno[2,3-c]pyridin-2-ylamino)prop-2-yl)benzyl 3,3-bis((nitrooxy)methyl)cyclobutane-1-carboxylate (compound 36.7)

Compound 36.6 (50 mg, 0.20 mmol) was dissolved in 20 mL of N,N-dimethylformamide, and 1-(3-dimethylaminopropyl)-3-ethylcarbodiimide hydrochloride (57 mg, 0.30 mmol), 4-dimethylaminopyridine (37 mg, 0.30 mmol), and compound 15.2 (77 mg, 0.18 mmol) were added. The reaction solution was reacted overnight at room temperature. After the reaction was monitored to be complete by LCMS, the reaction solution was diluted with water (30 mL) and extracted with ethyl acetate (40 mL×2). The organic phases were combined, washed with saturated saline water (20 mL×2), dried with anhydrous sodium sulfate, filtered, spin-dried, and purified by column chromatography to obtain product 36.7 (82 mg, yield: 69%, white solid). LCMS ESI(+)m/z:660.2(M+1).

### Step H: (S)-4-(3-amino-1-oxy-1-(thieno[2,3-c]pyridin-2-ylamino)prop-2-yl)benzyl 3,3-bis((nitrooxy)methyl)cyclobutane-1-carboxylate (compound 36)

After compound 36.7 (82 mg, 0.124 mmol) was dissolved in 10 mL of dichloromethane, 2 mL of trifluoroacetic acid was added to react at room temperature for 2 hours. After the reaction was monitored to be complete by LCMS, saturated sodium bicarbonate solution was added to the reaction solution to pH 8, and extraction was performed with ethyl acetate (50 mLX2). The combined organic phases were washed with saturated saline water (20 mL), dried with anhydrous sodium sulfate, filtered, spin-dried, and purified by reverse preparation to obtain product Example 36 (58 mg, yield: 84%, white solid). LCMS ESI(+)m/z:560.1 (M+1). ¹H NMR (400 MHz, DMSO) δ13.36 ( s, 1 H), 9.46 (s, 1 H), 8.52 (d, J = 6.4 Hz, 1 H), 8.25 - 8.08 (m, 4 H), 7.52 - 7.44 (m, 2 H), 7.42 - 7.34 (m, 3 H), 5.10 (s, 2 H), 4.66 (s, 2 H), 4.51 (s, 2 H), 4.47 - 4.40 (m, 1 H), 3.60-3.68 (m, 1 H), 3.33 - 3.29 (m, 1 H), 3.21 - 3.07 (m, 1 H), 2.32 - 2.14 (m, 4 H).

### Example 37

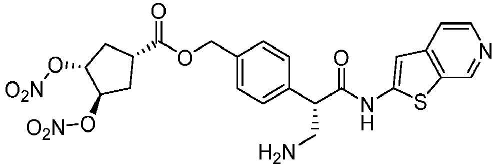

### (3R,4R)-4-((S)-3-amino-1-oxy-1-(thieno[2,3-c]pyridin-2-ylamino)propan-2-yl)benzyl 3,4-bis(nitrooxy)cyclopentane carboxylate

The specific reaction equation is as follows:

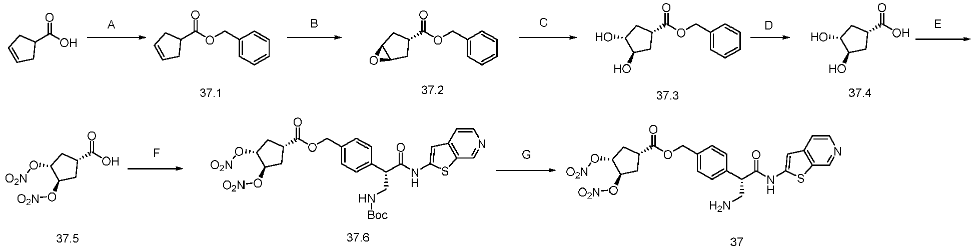

### Step A: benzyl cyclopent-3-enecarboxylate (compound 37.1)

Cyclopent-3-ene carboxylic acid (1 g, 8.93 mmol) was dissolved in 12 mL of N,N-dimethylformamide, and potassium carbonate (2.5 g, 17.86 mmol) and benzyl bromide (1.83 g, 10.72 mmol) were added. The reaction solution was stirred at room temperature overnight. Water (20 mL) was added to the reaction solution to dilute, and extraction was performed with ethyl acetate (40 mL) two times. The organic phases were combined, washed with saturated saline water (30 mL) two times, dried with anhydrous sodium sulfate, filtered, spin-dried, and purified by column chromatography to obtain product 37.1 (1.69 g, yield: 94%, colorless oily substance). LCMS ESI(+)m/z:203.1(M+1).

### Step B: benzyl (1R,3s,5S)-6-oxabicyclo[3.1.0]hexane-3-carboxylate (compound 37.2)

Compound 37.1 (650 mg, 3.22 mmol) was dissolved in 15 mL of dichloromethane, the reaction solution was cooled to 0 °C, m-chloroperoxybenzoic acid (724 mg, 4.20 mmol) was added in batches, and the reaction solution was stirred at room temperature for 3 hours. A white solid was precipitated, the reaction solution was filtered, and the filtrate was washed with saturated sodium bicarbonate solution (20 mL), 20% sodium sulfite solution (20 mL), and saturated saline water (20 mL) successively, dried with anhydrous sodium sulfate, filtered, spin-dried, and purified by column chromatography to obtain product 37.2 (460 mg, yield: 88%, colorless oily substance). LCMS ESI(+)m/z:219.1(M+1).

### Step C: (3R,4R)-3,4-dihydroxycyclopentanecarboxylate (compound 37.3)

Compound 37.2 (460 mg, 2.11 mmol) was dissolved in 4 mL of tetrahydrofuran and 4 mL of water, 0.17 mL of sulfuric acid solution was added, and the reaction solution was stirred at room temperature overnight. Saturated sodium bicarbonate was added to the reaction solution to pH 8, and extraction was performed with ethyl acetate (50 mL) two times. The organic phases were combined, dried with anhydrous sodium sulfate, filtered, concentrated under reduced pressure, and purified by column chromatography to obtain product 37.3 (290 mg, yield: 58%, colorless oily substance). LCMS ESI(+)m/z:237.1(M+1).

### Step D: (3R,4R)-3,4-dihydroxycyclopentanecarboxylic acid (compound 37.4)

Compound 37.3 (330 mg, 1.4 mmol) was dissolved in 6 mL of methanol, palladium on carbon (33 mg) was added, and the reaction solution was stirred for 3 hours at room temperature under a hydrogen atmosphere. The reaction solution was filtered with diatomite, and the filtrate was concentrated under reduced pressure to obtain product 37.4 (200 mg, yield: 98%, white solid). LCMS ESI(+)m/z:145.1 (M-1).

### Step E: (3R,4R)-3,4-bis(nitrooxy)cyclopentanecarboxylic acid (compound 37.5)

In an ice-water bath, 1 mL of concentrated nitric acid was added dropwise to 2 mL of acetic anhydride and stirring was performed for 10 minutes. Compound 37.4 (100 mg, 0.69 mmol) was dissolved in 2 mL of acetic anhydride, and the nitric acid acetic anhydride solution was added dropwise under the ice-water bath to react at 0 °C for 1 hour. After the reaction was monitored to be complete by TLC, the reaction solution was diluted with water (20 mL) and extracted with ethyl acetate (40 mL×2). The organic phases were combined, washed with saturated saline water (20 mL), dried with anhydrous sodium sulfate, filtered, spin-dried, and purified by column chromatography to obtain product 37.5 (95 mg, yield: 59%, colorless oily substance).

### Step F: (3R,4R)-4-((S)-3-((tert-butoxycarbonyl)amino)-1-oxo-1-(thieno[2,3-c]pyridin-2-ylamino)prop-2-yl benzyl 3,4-bis(nitrooxy)cyclopentanecarboxylate (compound 37.6)

Compound 37.5 (40 mg, 0.17 mmol) was dissolved in 5 mL of N,N-dimethylformamide, and 1-(3-dimethylaminopropyl)-3-ethylcarbodiimide hydrochloride (49 mg, 0.255 mmol), 4-dimethylaminopyridine (31 mg, 0.255 mmol), and compound 15.2 (44 mg, 0.102 mmol) were added. The reaction solution was reacted overnight at room temperature. After the reaction was monitored to be complete by LCMS, the reaction solution was diluted with water (30 mL) and extracted with ethyl acetate (40 mL×2). The organic phases were combined, washed with saturated saline water (20 mL×2), dried with anhydrous sodium sulfate, filtered, spin-dried, and purified by column chromatography to obtain product 37.6 (50 mg, yield: 46%, white solid). LCMS ESI(+)m/z:646.2(M+1).

### Step G: (3R,4R)-4-((S)-3-amino-1-oxy-1-(thieno[2,3-c]pyridin-2-ylamino)propan-2-yl)benzyl 3,4-bis(nitrooxy)cyclopentane carboxylate (compound 37)

After compound 37.6 (50 mg, 0.08 mmol) was dissolved in 4 mL of dichloromethane, 0.8 mL of trifluoroacetic acid was added to react at room temperature for 2 hours. After the reaction was monitored to be complete by LCMS, saturated sodium bicarbonate solution was added to the reaction solution to pH 8, and extraction was performed with ethyl acetate (50 mLX2). The combined organic phases were washed with saturated saline water (20 mL), dried with anhydrous sodium sulfate, filtered, spin-dried, and purified by reverse preparation to obtain product Example 37 (27 mg, yield: 60%, white solid). LCMS ESI(+)m/z:546.1 (M+1). ¹H NMR (400 MHz, DMSO) δ13.42(s, 1 H), 9.47 (s, 1 H), 8.52 (d, J = 6.4 Hz, 1 H), 8.20 ( s, 3 H), 8.14 (d, J = 6.4 Hz, 1 H), 7.54 - 7.45 (m, 2 H), 7.39-7.41 (m, 3 H), 5.70 - 5.51 (m, 2 H), 5.11 (s, 2 H), 4.55 - 4.37 (m, 1 H), 3.62-3.66 ( m, 1 H), 3.22 - 3.05 (m, 2 H), 2.60 - 2.53 (m, 1 H), 2.45 - 2.35 (m, 1 H), 2.14-2.21 (m, 1 H), 2.10 - 1.99 (m, 1 H).

### Example 38

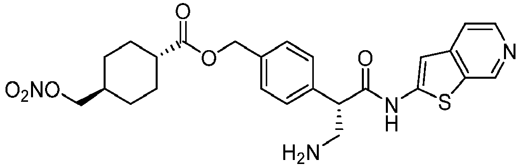

### (1r,4S)-4-((S)-3-amino-1-oxy-1-(thieno[2,3-c]pyridin-2-ylamino)propan-2-yl)benzyl 4-((nitrooxy)methyl)cyclohexanecarboxylic acid

The specific reaction equation is as follows:

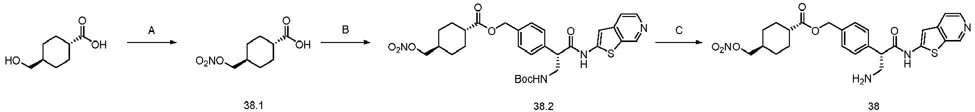

### Step A: (1R,4R)-4-((nitrooxy)methyl)cyclohexanecarboxylic acid (compound 38.1)

In an ice-water bath, 3 mL of concentrated nitric acid was added dropwise to 3 mL of concentrated sulfuric acid, and stirring was performed for 10 minutes. Next, a dichloromethane (2 mL) solution of (1R,4R)-4-(hydroxymethyl)cyclohexane carboxylic acid (100 mg, 0.63 mmol) was added under the ice-water bath, and the temperature was kept below 5 °C and the mixture was stirred for 30 minutes. After the reaction was monitored to be complete by TLC, the reaction solution was diluted with water (20 mL) in the ice-water bath and extracted with dichloromethane (20 mL×2). The organic phases were combined, dried with anhydrous sodium sulfate, filtered, spin-dried, and purified by column chromatography to obtain product 38.1 (102 mg, yield: 79%, white solid).

### Step B: (1R,4S)-4-((S)-3-((tert-butoxycarbonyl)amino)-1-oxo-1-(thieno[2,3-c]pyridin-2-ylamino)prop-2-yl benzyl 4-((nitrooxy)methyl)cyclohexanecarboxylate (compound 38.2)

Compound 38.1 (43.5 mg, 0.21 mmol), 1-(3-dimethylaminopropyl)-3-ethylcarbodiimide hydrochloride (54.8 mg, 0.28 mmol), and 4-dimethylaminopyridine (33.5 mg, 0.28 mmol) were dissolved in N,N-dimethylformamide (3 mL), and compound 15.2 (60.8 mg, 0.14 mmol) was added to react at room temperature overnight. After the reaction was monitored to be complete by LCMS, the reaction solution was diluted with water (15 mL) and extracted with ethyl acetate (15 mL×2). The organic phases were combined, washed with saturated saline water (20 mL×5), dried with anhydrous sodium sulfate, filtered, spin-dried, and purified by column chromatography to obtain product 38.2 (82.5 mg, yield: 94%, light yellow solid). LCMS ESI(+)m/z:613.2(M+1).

### Step C: (1R,4S)-4-((S)-3-amino-1-oxo-1-(thieno[2,3-c]pyridin-2-ylamino)propane-2-yl)benzyl 4-((nitrooxy)methyl)cyclohexanecarboxylic acid (compound 38)

Compound 38.2 (80 mg, 0.13 mmol) was dissolved in an ethyl acetate solution (4 M) of 5 mL of hydrogen chloride to react at room temperature for 1 hour. After the reaction was monitored to be complete by LCMS, the reaction solution was concentrated to dryness and purified by reverse preparation to obtain product Example 38 (40.4 mg, yield: 60%, white solid). LCMS ESI(+)m/z:513.2 (M+1). ¹H NMR (400 MHz, DMSO) δ 13.53 (s, 1H), 9.47 (s, 1H), 8.52 (d, J = 6.4 Hz, 1H), 8.25 (s, 3H), 8.14 (d, J = 6.4 Hz, 1H), 7.50 (d, J = 8.4 Hz, 2H), 7.41 (s, 1H), 7.38 (d, J = 8.2 Hz, 2H), 5.06 (s, 2H), 4.52 - 4.44 (m, 1H), 4.34 (d, J = 6.4 Hz, 2H), 3.70 - 3.58 (m, 1H), 3.21 - 3.08 (m, 1H), 2.30 (tt, J = 12.0, 3.2 Hz, 1H), 1.93 (d, J = 10.4 Hz, 2H), 1.79 - 1.62 (m, 3H), 1.34 (qd, J = 12.8, 3.2 Hz, 2H), 1.06 (qd, J = 12.8, 3.2 Hz, 2H).

### Example 39

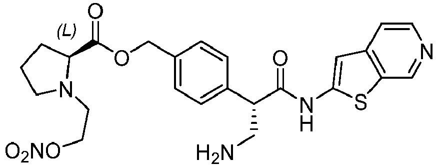

### (S)-4-((S)-3-amino-1-oxy-1-(thieno[2,3-c]pyridin-2-ylamino)propan-2-yl)benzyl 1-(2-(nitrooxy))ethyl)pyrrolidine-2-carboxylate

The specific reaction equation is as follows:

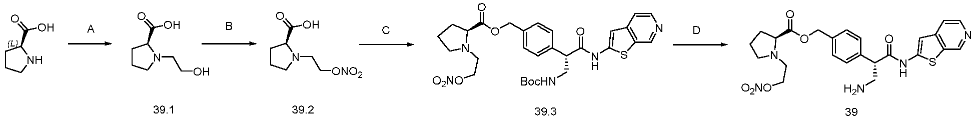

### Step A: (S)-1-(2-hydroxyethyl)pyrrolidine-2-carboxylic acid (compound 39.1)

L-proline (921 mg, 8 mmol) and potassium hydroxide (1.35 g, 24 mmol) were dissolved in 20 mL of isopropanol, and 2-bromoethanol (1.2 g, 9.6 mmol) was added dropwise to react overnight at 40 °C. After the reaction was monitored to be complete by LCMS, the pH was adjusted to less than 4 with hydrochloric acid. The organic solvent was spun off under reduced pressure, 20 mL of ethanol and 20 mL of dichloromethane were added to stir for 2 hours, and then the reaction solution was filtered. The filtrate was concentrated and recrystallized with 20 mL of acetone and 2 mL of methanol to obtain compound 39.1 (968 mg, yield 76%, white solid).

### Step B: (S)-1-(2-(nitrooxy)ethyl)pyrrolidine-2-carboxylic acid (compound 39.2)

In an ice-water bath, 3 mL of concentrated nitric acid was added dropwise to 3 mL of concentrated sulfuric acid, and stirring was performed for 10 minutes. Then, a dichloromethane (10 mL) solution of compound 39.1 (500 mg, 3.1 mmol) was added under the ice-water bath, and the temperature was kept below 5 °C to stir for 1 hour. After the reaction was monitored to be complete by TLC, the reaction solution was diluted with dichloromethane (40 mL) in the ice-water bath, the pH was adjusted to about 5 with sodium carbonate solid, and the organic solvent was spun off. 30 mL of a mixed solution of dichloromethane and ethanol (1:1) was added, and after stirring at room temperature for 1 hour, the reaction solution was filtered and the filtrate was concentrated to obtain product 39.2 (337 mg, yield: 53%, yellow solid).

### Step C: (S)-4-((S)-3-((tert-butoxycarbonyl)amino)-1-oxy-1-(thieno[2,3-c]pyridin-2-ylamino)prop-2-yl)benzyl 1-(2-(nitrooxy)ethyl)pyrrolidine-2-carboxylate (compound 39.3)

Compound 39.2 (73 mg, 0.36 mmol), 1-(3-dimethylaminopropyl)-3-ethylcarbodiimide hydrochloride (69 mg, 0.36 mmol), and 4-dimethylaminopyridine (44 mg, 0.36 mmol) were dissolved in N,N-dimethylformamide (3 mL), and compound 15.2 (51 mg, 0.12 mmol) was added to react at room temperature overnight. After the reaction was monitored to be complete by LCMS, the reaction solution was diluted with water (15 mL) and extracted with ethyl acetate (15 mL×2). The organic phases were combined, washed with saturated saline water (20 mL×5), dried with anhydrous sodium sulfate, filtered, spin-dried, and purified by column chromatography to obtain product 39.3 (47 mg, yield: 64%, light yellow solid). LCMS ESI(+)m/z:614.2(M+1).

### Step D: (S)-4-((S)-3-amino-1-oxy-1-(thieno[2,3-c]pyridin-2-ylamino)propan-2-yl)benzyl 1-(2-(nitrooxy))ethyl)pyrrolidine-2-carboxylate (compound 39)

Compound 39.3 (47 mg, 0.076 mmol) was dissolved in an ethyl acetate solution (4 M) of 5 mL of hydrogen chloride to react at room temperature for 1 hour. After the reaction was monitored to be complete by LCMS, the reaction solution was concentrated to dryness and purified by reverse preparation to obtain product Example 39 (19 mg, yield: 52%, white solid). LCMS ESI(+)m/z:514.2(M+1).

### Example 40

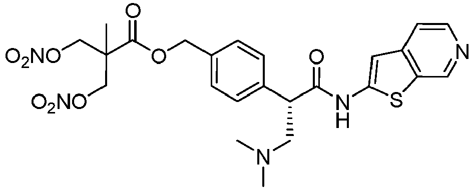

### (S)-4-(3-(dimethylamino)-1-oxo-1-(thieno[2,3-c]pyridin-2-ylamino)propan-2-yl)benzyl 2-methyl-3-(nitrooxy)-2-((nitrooxy)methyl)propionate

The specific reaction equation is as follows:

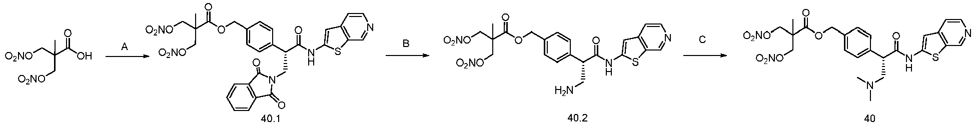

### Step A: (S)-4-(3-(1,3-dioxyisoindol-2-yl)-1-oxy-1-(thieno[2,3-c]pyridin-2-ylamino)prop-2-yl)benzyl 2-3-(nitrooxy)methyl-2-((nitrooxy)methyl)propionate (compound 40.1)

2-methyl-3-(nitrooxy)-2-((nitrooxy)methyl)propionic acid (146 mg, 0.32 mmol), 1-[bis(dimethylamino)methylene]-1H-1,2,3-triazolo[4,5-b]pyridinium 3-oxide hexafluorophosphate (182 mg, 0.48 mmol), and diisopropylethylamine (123.8 mg, 0.96 mmol) were dissolved in N,N-dimethylformamide (10 mL), and compound 15.2 (107.5 mg, 0.32 mmol) was added to react at room temperature for 3 hours. After the reaction was monitored to be complete by TLC, the reaction solution was diluted with water (30 mL) and extracted with ethyl acetate (30 mL×2). The organic phases were combined, washed with saturated saline water (30 mL×5),dried with anhydrous sodium sulfate, filtered, spin-dried, and purified by column chromatography to obtain product 40.1 (157 mg, yield: 74%, light yellow solid). LCMS ESI(+)m/z:664.1(M+1).

### Step B: (S)-4-(3-amino-1-oxo-1-(thieno[2,3-c]pyridin-2-ylamino)prop-2-yl)benzyl 2-methyl-3-(nitrooxy)-2-((nitrooxy)methyl)propionate (compound 40.2)

Compound 40.1 (157 mg, 0.237 mmol) was dissolved in 5 mL of ethanol, and hydrazine hydrate (118 mg, 2.37 mmol) was added to react at 55 °C for 2 hours. After the reaction was monitored to be complete by LCMS, the reaction solution was diluted with water (20 mL) and extracted with ethyl acetate (20 mL×2). The organic phases were combined, washed with saturated saline water (20 mL), dried with anhydrous sodium sulfate, filtered, spin-dried, and purified by column chromatography to obtain product 40.2 (75 mg, yield: 59%, light yellow solid). LCMS ESI(+)m/z:534.1(M+1).

### Step C: (S)-4-(3-(dimethylamino)-1-oxo-1-(thieno[2,3-c]pyridin-2-ylamino)propan-2-yl)benzyl 2-methyl-3-(nitrooxy)-2-((nitrooxy)methyl)propionate (compound 40)

Under nitrogen protection, compound 40.2 (75 mg, 0.14 mmol), formaldehyde (57 mg, 37% to 40% aqueous solution, 0.7 mmol), and a drop of acetic acid were dissolved in 5 mL of methanol, and sodium cyanoborohydride (26.4 mg, 0.42 mmol) was added under an ice-water bath to react at room temperature for 1 hour. After the reaction was monitored to be complete by LCMS, the reaction solution was quenched with saturated sodium bicarbonate solution (10 mL), diluted with water (10 mL), and extracted with ethyl acetate (20 mL×2). The organic phases were combined, washed with saturated saline water (20 mL), dried with anhydrous sodium sulfate, filtered, spin-dried, and purified by column chromatography to obtain the crude product. Reverse preparation and purification were further performed to obtain product Example 40 (48 mg, yield: 57%, white solid). LCMS ESI(+)m/z:562.2 (M+1). ¹H NMR (400 MHz, DMSO) δ 13.47 (s, 1H), 9.27 (s, 1H), 8.44 (s, 1H), 7.92 (s, 1H), 7.54 (d, J = 7.6 Hz, 2H), 7.40 (d, J = 7.6 Hz, 2H), 7.27 (s, 1H), 5.17 (s, 2H), 4.86 - 4.69 (m, 5H), 4.07 - 3.96 (m, 1H), 3.49 - 3.41 (m, 1H), 2.79 (s, 6H), 1.29 (s, 3H).

### Example 41

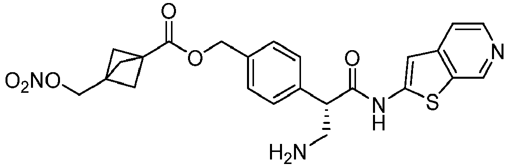

### (S)-4-(3-amino-1-oxy-1-(thieno[2,3-c]pyridin-2-ylamino)prop-2-yl)benzyl 3-((nitrooxy)methyl)bicyclo[1.1.1]pentane-1-carboxylate

The specific reaction equation is as follows:

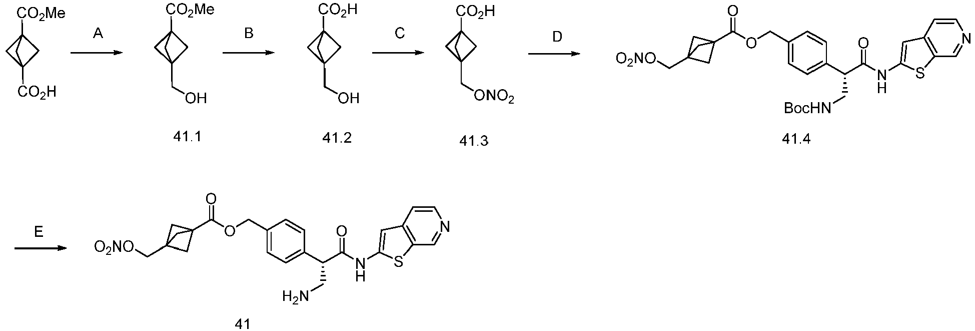

### Step A: methyl 3-(hydroxymethyl)bicyclo[1.1.1]pentane-1-carboxylate (compound 41.1)

Under nitrogen protection, 3-(methoxycarbonyl)bicyclo[1.1.1]pentane-1-carboxylic acid (493 mg, 2.9 mmol) was dissolved in 15 mL of tetrahydrofuran, and a dimethyl sulfide solution of borane (0.44 mL, 10 M, 4.35 mmol) was added dropwise under an ice-water bath to react at room temperature for 3 hours. After the reaction was monitored to be complete by TLC, the reaction was quenched with 5 mL of methanol and 4 mL of hydrochloric acid (2 M). After the organic solvent was span off under reduced pressure, dilution was performed with water (20 mL), and extraction was performed with ethyl acetate (20 mL×2). The organic phases were combined, washed with saturated saline water (20 mL), dried with anhydrous sodium sulfate, filtered, spin-dried, and purified by column chromatography to obtain product 41.1 (402 mg, yield: 89%, white solid).

### Step B: 3-(hydroxymethyl)bicyclo[1.1.1]pentane-1-carboxylic acid (compound 41.2)

Compound 41.1 (400 mg, 2.5 mmol) was dissolved in 10 mL of tetrahydrofuran and 10 mL of methanol, and an aqueous solution (5 mL) of sodium hydroxide (200 mg, 5 mmol) was added to react overnight at room temperature. After the reaction was monitored to be complete by TLC, the organic solvent was spun off under reduced pressure, then diluted with water (15 mL) and washed with ethyl acetate (15 mL×2). The pH of the aqueous layer was adjusted to less than 4 with hydrochloric acid, extracted with ethyl acetate (20 mL×2), and the organic phases were combined, dried with anhydrous sodium sulfate, filtered, spin-dried, and purified by column chromatography to obtain product 41.2 (287 mg, yield: 81%, white solid).

### Step C: 3-((nitrooxy)methyl)bicyclo[1.1.1]pentane-1-carboxylic acid (compound 41.3)

In an ice-water bath, 5 mL of concentrated nitric acid was added dropwise to 5 mL of concentrated sulfuric acid, and stirring was performed for 10 minutes. Then, a dichloromethane (5 mL) solution of compound 41.2 (285 mg, 2 mmol) was added under the ice-water bath, and the temperature was kept below 5 °C to stir for 2 hours. After the reaction was monitored to be complete by TLC, the reaction solution was poured into 20 mL of ice water to dilute, and extraction was performed with ethyl acetate (20 mL×2). The organic phases were combined, washed with saturated saline water (20 mL), dried with anhydrous sodium sulfate, filtered, spin-dried, and purified by column chromatography to obtain product 41.3 (303 mg, yield: 81%, white solid).

### Step D: (S)-4-(3-((tert-butoxycarbonyl)amino)-1-oxo-1-(thieno[2,3-c]pyridin-2-ylamino)propan-2-yl)benzyl 3-((nitrooxy)methyl)bicyclo[1.1.1]pentane-1-carboxylate (compound 41.4)

Compound 41.3 (33.7 mg, 0.18 mmol), 1-(3-dimethylaminopropyl)-3-ethylcarbodiimide hydrochloride (46 mg, 0.24 mmol), and 4-dimethylaminopyridine (29.3 mg, 0.24 mmol) were dissolved in N,N-dimethylformamide (2 mL), and compound 15.2 (51 mg, 0.12 mmol) was added to react at room temperature overnight. After the reaction was monitored to be complete by LCMS, the reaction solution was diluted with water (15 mL) and extracted with ethyl acetate (15 mL×2). The organic phases were combined, washed with saturated saline water (20 mL×3), dried with anhydrous sodium sulfate, filtered, and spin-dried. The residue was purified by column chromatography to obtain product 41.4 (54.9 mg, yield: 77%, light yellow solid). LCMS ESI(+)m/z:597.2(M+1).

### Step E: (S)-4-(3-amino-1-oxy-1-(thieno[2,3-c]pyridin-2-ylamino)prop-2-yl)benzyl 3-((nitrooxy)methyl)bicyclo [1.1.1]pentane-1-carboxylate (compound 41)

Compound 41.4 (54 mg, 0.09 mmol) was dissolved in an ethyl acetate solution (4 M) of 3 mL of ethyl acetate and 3 mL of hydrogen chloride to react at room temperature for 2.5 hours. After the reaction was monitored to be complete by LCMS, the reaction solution was concentrated to dryness and purified by reverse preparation to obtain product Example 41 (40 mg, yield: 83%, white solid). LCMS ESI(+)m/z:497.2 (M+1).¹H NMR (400 MHz, DMSO) δ 13.56 (s, 1H), 9.47 (s, 1H), 8.52 (d, J = 6.4 Hz, 1H), 8.21 (s, 3H), 8.15 (d, J = 6.4 Hz, 1H), 7.50 (d, J = 7.6 Hz, 2H), 7.43 ― 7.31 (m, 3H), 5.08 (s, 2H), 4.58 (s, 2H), 4.53 ― 4.40 (m, 1H), 3.70 ― 3.56 (m, 1H), 3.22 ― 3.08 (m, 1H), 2.02 (s, 6H).

### Example 42

### ROCK2 kinase inhibitor activity test

### <1> Experimental process

1) 4X ROCK2 reaction solution, 4X ATP reaction solution, 4X substrate solution, and 4X compound solution were prepared according to experimental requirements.
2) 2.5 µL of 4X kinase solution and 2.5 µL of diluted 4X test compound solutions with different concentration gradients were added to a 384-well plate. 2 replicate wells were provided for each concentration, and an enzyme solution blank control group and a negative control group (DMSO group) were provided at the same time. Centrifugation was performed at 1000 rpm for 1 minute, and the enzyme and the compounds were mixed. 2.5 µL of 4X substrate solution was added to the 384-well plate, centrifugation was performed at 1000 rpm for 1 minute, 2.5 µL of 4X ATP solution was added to the 384-well plate, centrifugation was performed at 1000 rpm for 1 minute to start the enzyme reaction, and incubation was performed at room temperature for 1.5 hours.
   The final concentration of each component of the ROCK2 reaction was: ROCK2: 1 nM, substrate: 500 nM, ATP: 6 uM, and the final concentration range of the test compounds was: 100 nM to 5 pM.
3) 4X Streptavidin-XL-665 detection solution was prepared according to experimental requirements.
4) After the enzyme reaction was completed, 5 ul of 4X Streptavidin-XL-665 detection solution was added to each well of the 384-well plate, and centrifugation was performed at 1000 rpm for 1 minute. 5 µL of 4X STK Antibody-cryptate detection antibody solution was added to each well of the 384-well plate, centrifugation was performed at 1000 rpm for 1 minute, and incubation was performed for 1 hour at room temperature.
5) After antibody incubation was completed, the signal value of each well was measured by the HTRF program on an Envision plate reader.

### <2> Data analysis

1) Taking the enzyme solution blank control group as 100% inhibition rate and the negative control group (DMSO group) as 0% inhibition rate, the percentage inhibition rate corresponding to each concentration of the test compound was calculated.
2) In the GraphPad Prism software, a nonlinear regression analysis was performed on the logarithm of the concentrations of the test compounds and the corresponding percentage inhibition rates to obtain the half-inhibitory concentrations (IC₅₀) of the test compounds.

The ROCK2 kinase inhibitor activities (IC₅₀) of the compounds detected by this method are as follows:

| Ex# | ICso (nM) | Ex# | IC₅₀ (nM) |
|---|---|---|---|
| 1 | 0.5 | 21 | 1.23 |
| 2 | 5.59 | 22 | 1.6 |
| 3 | 10.8 | 23 | 1.13 |
| 4 | 1.78 | 24 | 0.45 |
| 5 | 19.2 | 25 | 1.78 |
| 6 | 1.3 | 26 | 9.9 |
| 7 | 2.19 | 27 | 4.62 |
| 8 | 5.9 | 28 | 1.32 |
| 9 | 1.02 | 29 | 2.74 |
| 10 | 19.32 | 30 | 16.5 |
| 11 | 6.01 | 31 | 1.58 |
| 12 | 2.95 | 32 | 3.11 |
| 13 | 1.51 | 33 | 1.18 |
| 14 | 77.34 | 34 | 1.04 |
| 15 | 1.63 | 35 | 6.5 |
| 16 | 1.86 | 36 | 32.55 |
| 17 | 5.44 | 37 | 1.43 |
| 18 | 4.62 | 38 | 19.69 |
| 19 | 1.33 | 39 | 29.22 |
| 20 | 1.43 | 40 | 2.05 |

### Example 43: cell activity test

### < 1 > Experimental process

1) The A7R5 cells in the logarithmic growth phase were digested, 2E5 cells per well were inoculated in a 6-well plate and incubated overnight at 37 °C with 5% CO₂, and the cell culture medium was replaced with a starvation medium containing 0.05% FBS after 24 hours. The cells were starved for 20 hours.
2) The starvation medium was removed, the cells were treated with the prepared compound concentration gradient for 60 minutes, VIP-5021 (10 uM) was set as the positive control, and the DMSO group was set as the negative control.
3) After the compound treatment, the compound solutions were aspirated, washed with PBS two times, 100 ul of cell lysate was added to each well, the cells were gently scraped with a cell scraper, and the cells were transferred to a centrifuge tube and lysed on ice for 60 minutes.
4) After the lysis, centrifugation was performed at 4 °C and 14000 rpm for 15 minutes, the lysate supernatant was collected, protein quantification was performed using the BCA method, the protein concentration was adjusted, the loading buffer was added according to proportion, and treatment was performed at 100 °C for 10 minutes to denature the protein.
5) An electrophoresis equipment was assembled, 20 ug of total protein was loaded per sample, the voltage was fixed at 125 V, and electrophoresis was performed for 90 minutes. After the electrophoresis was completed, the membrane transfer system was assembled with a fixed current of 300 mA and the membrane was transferred for 120 minutes. After the membrane transfer was completed, the PVDF membrane was removed and washed with PBS two times. A blocking solution was added to block at room temperature for 60 minutes. The blocking solution was removed, a solution containing the primary antibody (Phospho-Myosin Light Chain2 (Ser19)) (antibody dilution ratio 1:1000) was added, GAPDH was selected as internal control, and incubation was performed overnight at 4 °C.
6) Washing was performed with PBST three times, HRP-antibody containing the corresponding species (secondary antibody dilution ratio 1:2000) was added, incubation was performed at room temperature for 60 minutes, and washing was performed with PBST three times. HRP chemiluminescence substrate was prepared, and dropwisely added on the PVDF membrane based on 0.1 mL per square centimeter to react for 30 seconds, and the chemiluminescence signal was collected with a gel imager (ChemiDoc XRS+(BIO-RAD)).
7) Each band was quantitatively analyzed using image J software, and the phosphorylated protein level of Phospho-Myosin Light Chain2 (Ser19) in each sample was corrected using GAPDH expression as internal reference.

### <2> Data analysis

1) Taking the MLC2 (Ser19) protein phosphorylation level of the highest concentration compound sample as 100% inhibition rate, and taking the MLC2 (Ser19) protein phosphorylation level of the negative control group (DMSO group) as 0% inhibition rate, the percentage inhibition rate corresponding to each concentration of the test compounds was calculated.
2) In the GraphPad Prism software, a nonlinear regression analysis was performed on the logarithm of the concentrations of the test compounds and the corresponding percentage inhibition rates to obtain the half-inhibitory concentrations (IC₅₀) of the cell levels of the test compounds.

The cell activities (IC₅₀) of the compounds detected by this method are as follows:

| Ex# | ICso (nM) | Ex# | IC₅₀ (nM) |
|---|---|---|---|
| 1 | 53 | 9 | 30.3 |
| 7 | 8.8 | 11 | 21.4 |
| 8 | 35.8 | 13 | 39.5 |

## Claims

1. A small molecule compound of a NO donor, **characterized in that** the small molecule compound of the NO donor is a compound shown in the following structural formula I or a stereoisomer, a geometric isomer, a tautomer, a racemate, a deuterated isotopic derivative, a hydrate, a solvate, a metabolite, or a pharmaceutically acceptable salt or prodrug thereof ; 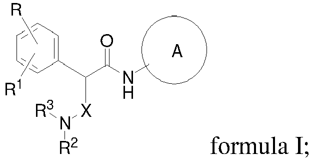
wherein a ring A is a substituted or unsubstituted heteroaromatic ring;
X is selected from (CH₂)ₙ, wherein n is selected from 0, 1, 2, or 3;
R is a substituent of terminal -O-NO₂;
R¹ is selected from hydrogen, a hydroxyl group, a halogen, an amino group, a cyano group, a substituted or unsubstituted alkyl group, a substituted or unsubstituted alkoxy group, a substituted or unsubstituted alkenyl group, a substituted or unsubstituted alkynyl group, or a substituted or unsubstituted heteroalkyl group;
R² and R³ are each independently selected from hydrogen, a substituted or unsubstituted alkyl group, a substituted or unsubstituted cycloalkyl group, or an amino protecting group;
alternatively, R² and R³ are connected to each other to form a substituted or unsubstituted cycloheteroalkyl group.

2. The small molecule compound of the NO donor of claim 1, **characterized in that** the small molecule compound of the NO donor is a compound shown in the following structural formula or a stereoisomer, a geometric isomer, a tautomer, a racemate, a deuterated isotopic derivative, a hydrate, a solvate, a metabolite, or a pharmaceutically acceptable salt or prodrug thereof: 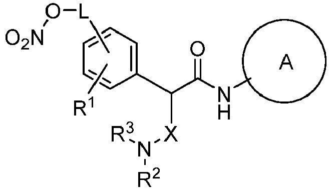 L is a linking group selected from a substituted or unsubstituted alkylene group, a substituted or unsubstituted heteroalkylene group, a substituted or unsubstituted alkyleneoxy group, a substituted or unsubstituted cycloalkyl group, a substituted or unsubstituted heterocyclic group, a substituted or unsubstituted aryl group, a substituted or unsubstituted heteroaryl group, or a substituted or unsubstituted ester group.

3. The small molecule compound of the NO donor of claim 1, **characterized in that**:
the ring A is selected from formula A or formula B; 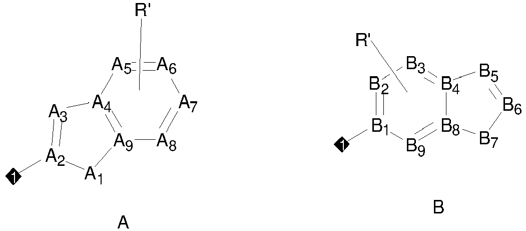
R' is one or a plurality of substituting substituent groups on the heteroaromatic ring;
the substituent group is selected from hydrogen, a hydroxyl group, a halogen, an amino group, a cyano group, a substituted or unsubstituted alkyl group, a substituted or unsubstituted alkoxy group, or a substituted or unsubstituted heteroalkyl group;
at least one of A₁, A₂, A₃, A₄, A₅, A₆, A₇, A₈, and A₉ is selected from nitrogen, sulfur, or oxygen;
at least one of B₁, B₂, B₃, B₄, B₅, B₆, B₇, B₈, and B₉ is selected from nitrogen, sulfur, or oxygen.

4. The small molecule compound of the NO donor of claim 1, **characterized in that**:
the ring A is selected from formula la, formula Ib, or formula Ic; 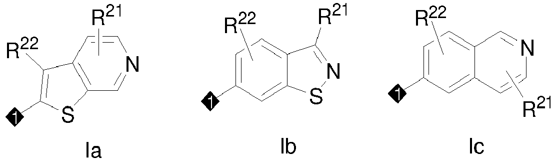
R²¹ is selected from hydrogen, a hydroxyl group, a halogen, an amino group, a cyano group, a substituted or unsubstituted alkyl group, a substituted or unsubstituted alkoxy group, or a substituted or unsubstituted heteroalkyl group;
R²² is selected from hydrogen, a halogen, a cyano group, a substituted or unsubstituted alkyl group, or a substituted or unsubstituted alkoxy group.

5. The small molecule compound of the NO donor of claim 1, **characterized in that** the small molecule compound of the NO donor is a compound shown in the following structural formula or a stereoisomer, a geometric isomer, a tautomer, a racemate, a deuterated isotopic derivative, a hydrate, a solvate, a metabolite, or a pharmaceutically acceptable salt or prodrug thereof: 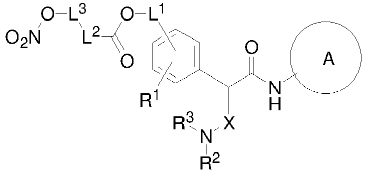
wherein L¹ is a chemical bond or a substituted or unsubstituted alkylene group;
L² is selected from a substituted or unsubstituted alkylene group, a substituted or unsubstituted alkyleneoxy group, a substituted or unsubstituted cycloalkyl group, a substituted or unsubstituted aryl group, or a substituted or unsubstituted heteroaryl group;
L³ is a chemical bond or a substituted or unsubstituted alkyleneoxy group.

6. The small molecule compound of the NO donor of claim 1, **characterized in that** the small molecule compound of the NO donor is a compound shown in the following structural formula or a stereoisomer, a geometric isomer, a tautomer, a racemate, a deuterated isotopic derivative, a hydrate, a solvate, a metabolite, or a pharmaceutically acceptable salt or prodrug thereof: 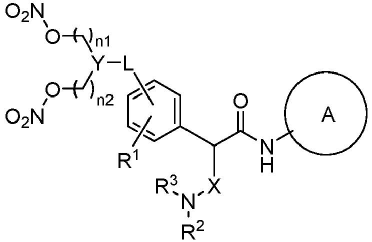
n1 is 0 or a natural number;
n2 is 0 or a natural number;
Y is selected from a substituted or unsubstituted alkyl group, a substituted or unsubstituted cycloalkyl group, a substituted or unsubstituted heterocyclic group, a substituted or unsubstituted aryl group, or a substituted or unsubstituted heteroaryl group.

7. A preparation method of the small molecule compound of the NO donor of claim 1, **characterized in that**:
a hydroxyl group in a compound shown in the following structure is obtained by a nitration reaction;

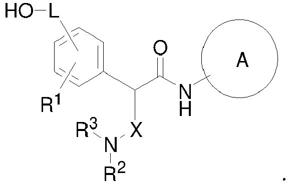

8. A preparation method of the small molecule compound of the NO donor of claim 1, **characterized in that**:
a halogen in a compound shown in the following structure is obtained by a nitration reaction; 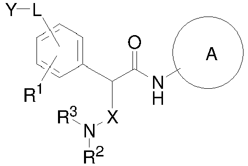 Y is a halogen.

9. A preparation method of the small molecule compound of the NO donor of claim 3, **characterized in that**:
the small molecule compound of the NO donor is obtained by a coupling reaction of compounds represented by the following formula IIIa and formula IIIb; 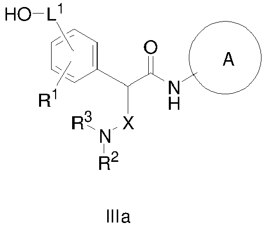 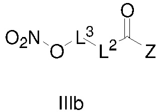 Z is a halogen.

10. The small molecule compound of the NO donor of any of claims 1 to 9, **characterized in that** the small molecule compound of the NO donor is used as an inhibitor of a ROCK kinase.
